# EUROPEAN PATENT SPECIFICATION

(11) **EP 1 005 356 B1**
(45) Date of publication and mention of the grant of the patent: **12.04.2006**
(21) Application number: 99928455.7
(22) Date of filing: 07.06.1999
(51) Int. Cl.: A61K 39/44, A61K 39/395, A61K 51/00, C07K 2/00, C07K 4/00, C07B 61/00, C07D 501/00, C07D 499/00, C07K 9/00, A61P 31/04, A61K 38/14

(54) **NOVEL ANTIBACTERIAL AGENTS**
NEUARTIGE ANTIBIOTIKA
AGENTS ANTIBACTERIENS Nouveaux

(30) Priority: 08.06.1998 US 88448 P; 16.07.1998 US 93072 P
(43) Date of publication of application: 07.06.2000
(73) Proprietor: Theravance, Inc., South San Francisco, CA 94080 (US)
(72) Inventor: GRIFFIN, John, H., Atherton, CA 94027 (US); MORAN, Edmund, J., San Francisco, CA 94127 (US); CHRISTENSEN, Burton, G., Alamo, CA 94507 (US); JUDICE, J. Kevin, El Granada, CA 94018 (US); MU, Yongqi, Los Altos, CA 94022 (US); PACE, John, San Anselmo, CA 94960 (US)
(74) Representative: Scott, Susan Margaret
(86) International application number: PCT/US1999/012776
(87) International publication number: WO 1999/064049

(56) References cited:
- WO-A-95/19974
- WO-A1-92/05802
- BE-A- 886 467
- OHYA, SATOSHI ET AL: "Potent cephalosporinase inhibitors: 7.beta.-[2-(1,3-dithiolan-2- ylidene)acetamido]cephalosporins and related compounds" ANTIMICROB. AGENTS CHEMOTHER. (1982), 21(4), 613-17, XP000923478
- DATABASE CHEMABS [Online] CHEMICAL ABSTRACTS SERVICE, COLUMBUS, OHIO, US; JIN, JUNG IL ET AL: "Synthesis and antibacterial activities of new.beta.-lactam compounds" retrieved from STN Database accession no. 107:236287 XP002142373 & YAKHAK HOECHI (1986), 30(6), 294-300,
- FITOUSSI, FREDERIC ET AL: "Comparative in vitro killing activities of meropenem, imipenem, ceftriaxone, and ceftriaxone plus vancomycin at clinically achievable cerebrospinal fluid concentrations against penicillin-resistant Streptococcus pneumoniae isolates from children with meningitis" ANTIMICROB. AGENTS CHEMOTHER. (1998), 42(4), 942-944 , XP002169323
- BAJAKSOUZIAN, S. ET AL: "Antipneumococcal activities of cefpirome and cefotaxime, alone and in combination with vancomycin and teicoplanin, determined by checkerboard and time-kill methods" ANTIMICROB. AGENTS CHEMOTHER. (1996), 40(9), 1973-1976 , XP002169324
- DATABASE CHEMABS [Online] CHEMICAL ABSTRACTS SERVICE, COLUMBUS, OHIO, US; FRIEDLAND, IAN R. ET AL: "Time-kill studies of antibiotic combinations against penicillin-resistant and -susceptible Streptococcus pneumoniae" retrieved from STN Database accession no. 121:251056 XP002169326 & J. ANTIMICROB. CHEMOTHER. (1994), 34(2), 231-7 ,
- DATABASE CHEMABS [Online] CHEMICAL ABSTRACTS SERVICE, COLUMBUS, OHIO, US; DONABEDIAN, HAIG ET AL: "Synergy of vancomycin with penicillins and cephalosporins against Pseudomonas, Klebsiella, and Serratia" retrieved from STN Database accession no. 88:944 XP002169327 & YALE J. BIOL. MED. (1977), 50(2), 165-76 ,
- SVENSSON, ERIK ET AL: "Pharmacodynamic effects of antibiotics and antibiotic combinations on growing and nongrowing Staphylococcus epidermidis cells" ANTIMICROB. AGENTS CHEMOTHER. (1997), 41(1), 107-111 , XP002169325
- DATABASE WPI Section Ch, Week 199211 Derwent Publications Ltd., London, GB; Class B05, AN 1992-085865 XP002169328 & JP 04 029930 A (FUJISAWA PHARM CO LTD), 31 January 1992 (1992-01-31)
- RUHLAND et al., "Solid-Supported Combinatorial Synthesis of Structurally Diverse beta-Lactams", J. AM. CHEM. SOC., 10 January 1996, Vol. 118, pages 253-254, XP002919650
- PITLIK et al., "Solution-Phase Synthesis of a Combinatorial Monocyclic beta-Lactam Library: Potential Protease Inhibitors", BIOORG. MED. CHEM. LETT., 16 December 1997, Vol. 7, No. 24, pages 3129-3134, XP002919651
- MURATA et al., "Effect of Dimerization of the D-Glucose Analoque of Muramyl Dipeptide on Stimulation of Macrophage-like Cells", CARBOHYDRATE RES., 02 January 1997, Vol. 297, pages 127-133, XP002919652
- SHUKER et al., "Discovering High-Affinity Ligands for Proteins: SAR by NMR", SCIENCE, 29 November 1996, Vol. 274, pages 1531-1534, XP002074440

## Description

### BACKGROUND OF THE INVENTION

### Field of the Invention

This invention relates to novel multibinding compounds (agents) that are antibacterial agents. The multibinding compounds of the invention comprise 2 ligands covalently connected by a linker, wherein each of said ligands in their monovalent (i.e., unlinked) state have the ability to bind to an enzyme involved in cell wall biosynthesis and metabolism, a precursor used in the synthesis of the bacterial cell wall and/or the cell surface and thereby interfere with the synthesis and or metabolism of the cell wall. The ligands are selected from the beta lactam and glycopeptide classes of antibacterial agents.

The invention also relates to pharmaceutical compositions comprising a pharmaceutically acceptable excipient and a therapeutically effective amount of one or more compound(s) of the invention, uses of such compounds and methods of preparing such compounds.

### Background

Bacteria possess a rigid outer layer, the cell wall. The cell wall maintains the shape of the microorganism which has a high internal osmotic pressure. Injury to the cell wall (e.g. by lysozyme) or inhibition of the cell wall's formation leads to lysis of the cell.

The cell wall contains a chemically distinct complex polymer "mucopeptide" ("murein", "peptidogylcan") consisting of polysaccharides and a highly cross-linked polypeptide. The polysaccharides comprise an alternating copolymer of the amino sugars N-acetylglucosamine and N-acetylmuramic acid, the latter being found only in bacteria. To the N-acetylmuramic residues are attached pentapeptides. The polysaccharide backbone of the cell wall is formed by oligomerization of disaccharide pentapeptide precursors (lipid intermediate II) and is catalyzed an enzyme known as transglycosylase. The final rigidity of the cell wall is imparted by cross-linking of the peptide chains as a result of transpeptidation reactions by several bacterial enzymes one of which is known as peptidoglycan transpeptidase.

One method by which antibacterial agents exert their antibacterial activity is by inhibiting the transglycosylase enzyme, thus interfering with the penultimate step in the synthesis of the bacterial cell wall. Although not wishing to be bound by theory, it is believed that a glycopeptide, for example vancomycin, binds with high affinity and specificity to N-terminal sequences (L-lysyl-D-alanyl-D-alanine in vancomycin sensitive organisms) of peptidoglycan precursors known as lipid intermediate II. By binding to and sequestering these precursors, vancomycin prevents their utilization by the cell wall biosynthesis machinery. In a formal sense, therefore, vancomycin inhibits the bacterial transglycosylase that is responsible for adding lipid intermediate II subunits to growing peptidoglycan chains. This step preceeds the cross-linking transpeptidation step which is inhibited by beta lactam antibiotics. It is believed that the β-lactam antibiotics bind to certain cell receptors (the penicillin binding proteins, "PBPs") which catalyze the transpeptidation reaction and other cell wall metabolic processes. The incomplete cell wall likely serves as a substrate for autolytic enzymes in the cell wall and results in lysis if the environment is isotonic.

Antibacterial agents have proved to be important weapons in the fight against pathogenic bacteria. However, an increasing problem with respect to the effectiveness of antibacterial agents relates to the emergence of strains of bacteria that are highly resistant to such agents. It would therefore be highly desirable to find antibacterial agents that are active against a broad spectrum of bacteria, in particular resistant strains. It would also be advantageous to discover antibacterial agents that demonstrate high activity and selectivity toward their targets, and are of low toxicity.

The multibinding compounds of the present invention fulfil this need.

### SUMMARY OF THE INVENTION

Accordingly, in one aspect, this invention provides a compound of Formula (I):

(L)ₚ(X)_{q} (I)

wherein:
p is 2;
q is 1;
   one ligand, L, is a beta lactam antibiotic, and the other ligand, L, is an optionally substituted glycopeptide antibiotic, or an aglycone derivative of an optionally substituted glycopeptide antibiotic; and
X is a linker,
provided that when one of the ligands is vancomycin attached via the carboxy terminus, then the other ligand cannot be cefalexin attached to the linker via acylation of its alpha amino group.

The invention also provides a pharmaceutical composition comprising a pharmaceutically acceptable carrier and an effective amount of a compound according to the invention.

The invention further provides a compound or a pharmaceutical composition according to the invention for use as a medicament, especially for treating a bacterial disease in a mammal.

The invention further provides the use of a compound or a pharmaceutical composition according to the invention for the manufacture of a medicament for treating a bacterial disease in a mammal.

Preferably,
the ligand that is a beta lactam antibiotic is selected from penems, penams, cephems, carbapenems, oxacephems, carbacephems, and monobactam ring systems; and
the ligand that is a glycopeptide antibiotic is selected from Actaplanin, Actinodidin, Ardacin, Avoparcin, Azureomycin, A477, A35512, A40926, A41030, A42867, A47934, A80407, A82846, A83850, A84575, A84428, AB-65, Balhimycin, Chloroeremomycin, Chloroorienticin, Chloropolysporin, Decaplanin, N-demethylvancomycin, Eremomycin, Galacardin, Helvecardin. Izupeptin, Kibdelin, LL-AM374, Mannopeptin, MM45289, MM47756, MM47761, MM47921, MM47766, MM55260, MM55266, MM55270, MM56579, MM56598, OA-7653, Oreenticin, Parvodicin. Ristocetin, Ristomycin, Synmonicin, Teicoplanin, UK-68597, UK-69542, UK-72051, Vancomycin, and aglycone derivatives thereof.

More preferably, the ligand that is a beta lactam antibiotic is selected from the group consisting of:
(i) a compound of formula (a): 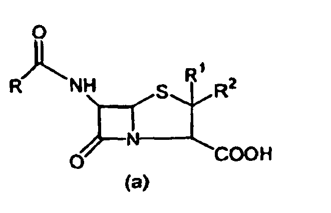 wherein:
   R is substituted alkyl, aryl, aralkyl, or heteroaryl wherein each of said substituent optionally links (a) to the linker via a covalent bond or R is a covalent bond that links (a) to the linker; and
   R¹ and R² are, independently of each other, alkyl or at least one of R¹ and R² is a covalent bond linking (a) to the linker;
(ii) a compound of formula (b): 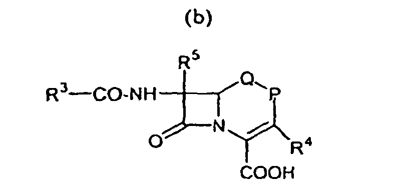 wherein:
   one of P and Q is O, S, or -CH₂- and the other is -CH₂-;
   R³ is substituted alkyl, heteroarylalkyl, aralkyl, heterocyclylalkyl, or -C(R⁶)=NOR⁷ (where R⁶ is aryl, heteroaryl, or substituted alkyl; and R⁷ is alkyl or substituted alkyl) wherein each of said substituent optionally links (b) to the linker or R³ is a covalent bond that links (b) to the linker; and
   R⁴ is hydrogen, alkyl, alkenyl, substituted alkenylene, substituted alkyl, halo, heteroarylalkyl, heterocyclylalkyl, -SR^{a} (where R^{a} is aryl, heteroaryl, heterocyclyl, or cycloalkyl) or -CH₂SR^{a} (where R^{a} is aryl, heteroaryl, heterocyclyl, or cycloalkyl) wherein each of said substituent optionally links (b) to the linker or R⁴ is a covalent bond that links (b) to the linker;
   R⁵ is hydrogen, hydroxy, or alkoxy;
(iii) a compound of formula (c): 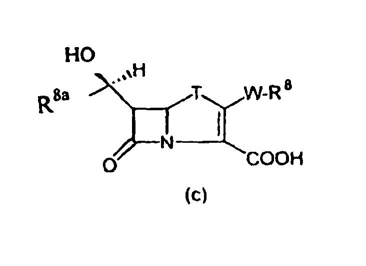 wherein:
   T is S or CH₂;
   R^{5a} is alkyl;
   W is O, S, -OCH₂-, or CH₂, and R³ is -(alkylene)-NHC(R^{b})=NH where R⁵ is a covalent bond linking (c) to the linker; or -W-R⁸ is a covalent bond that links (c) to the linker;
(iv) a compound of formula (d): 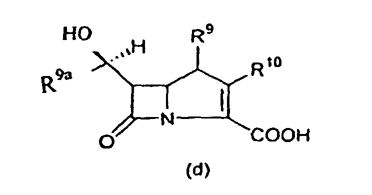 wherein:
   R⁹ and R^{9a} are alkyl;
   R¹⁰ is selected from the group consisting of hydrogen, alkyl, substituted alkyl, halo, aryl, heteroaryl, heterocyclyl, aralkyl, heteroaralkyl, heterocyclylalkyl or -CH₂SR^{a} (where R^{a} is aryl, heteroaryl, heterocyclyl, or cycloalkyl) wherein each of said substituent optionally links (d) to the linker or at least one of R⁹ and R¹⁰ is a covalent bond that links (d) to the linker; or
   R⁹ and R¹⁰ together with the carbon atoms to which they are attached form an aryl, heteroaryl, cycloalkyl, substituted cycloalkyl, or heterocyclyl ring of 4 to 7 ring atoms wherein one of the ring atoms optionally links (d) to the linker; or
(v) a compound of formula (e): 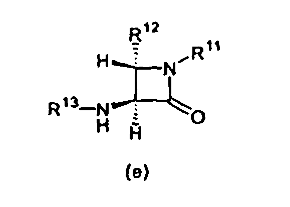 wherein:
   R¹¹ is -SO₃H or -(alkylene)-COOH;
   R¹² is alkyl, substituted alkyl, haloalkyl, alkoxy, aryl, aralkyl, heteroaryl, heteroaralkyl, cycloalkyl, substituted cycloalkyl, or heterocyclyl wherein each of said substituent optionally binds (e) to the linker or R¹² is a covalent bond that links (e) to the linker; and
   R¹³ is alkyl, acyl, or -COC(R¹⁴)=N-OR¹⁵ wherein R¹⁴ is aryl, heteroaryl which optionally links (e) to the linker, and R¹⁵ is -(alkylene)-COOR¹⁶ wherein R¹⁶ is hydrogen or optionally links (e) to the linker or R¹³ is a covalent bond that links (e) to the linker; and
   the ligand that is a glycopeptide antibiotic is an optionally substituted vancomycin which is linked to the linker via any hydroxyl group, carboxyl group or amino group; and pharmaceutically acceptable salts thereof.

Even more preferably, the ligand that is a beta lactam antibiotic is selected from:
(i) a compound of formula (a): 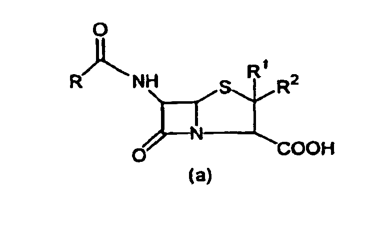 wherein:
   R is: 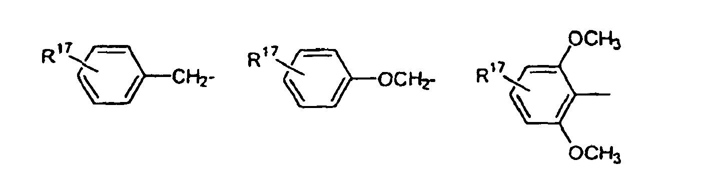 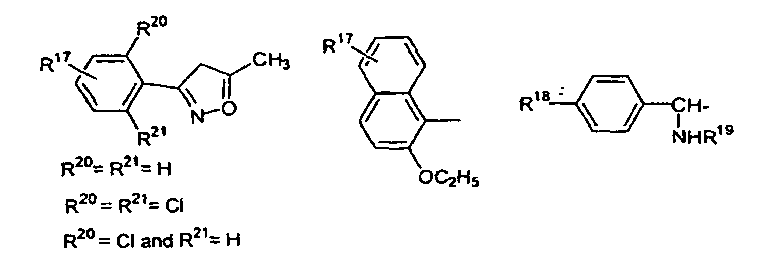 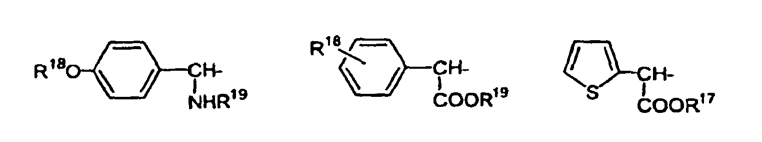 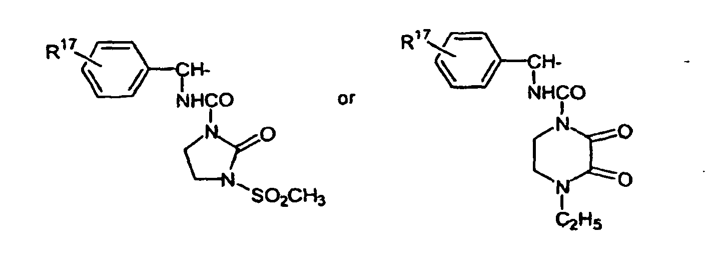
   where:
   R¹⁷ is a covalent bond that links the (a) group to the linker; one of R¹⁸ and R¹⁹ is hydrogen and the other is a covalent bond that links the (a) group to the linker; and
   R¹ and R² are methyl;
(ii) a compound of formula (b): 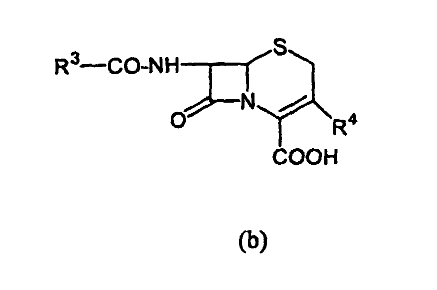 where:
   R³ and R⁴ are: 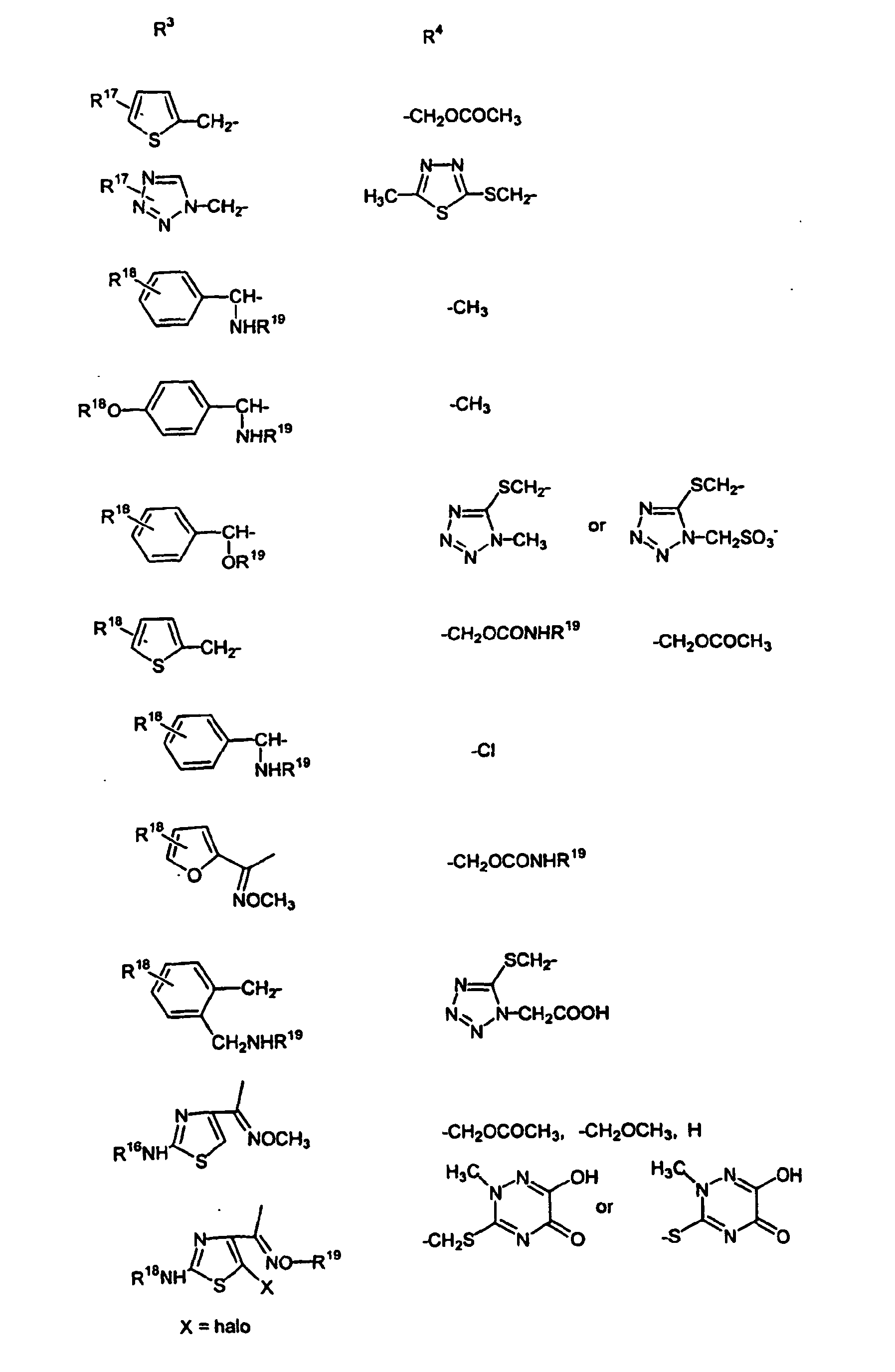 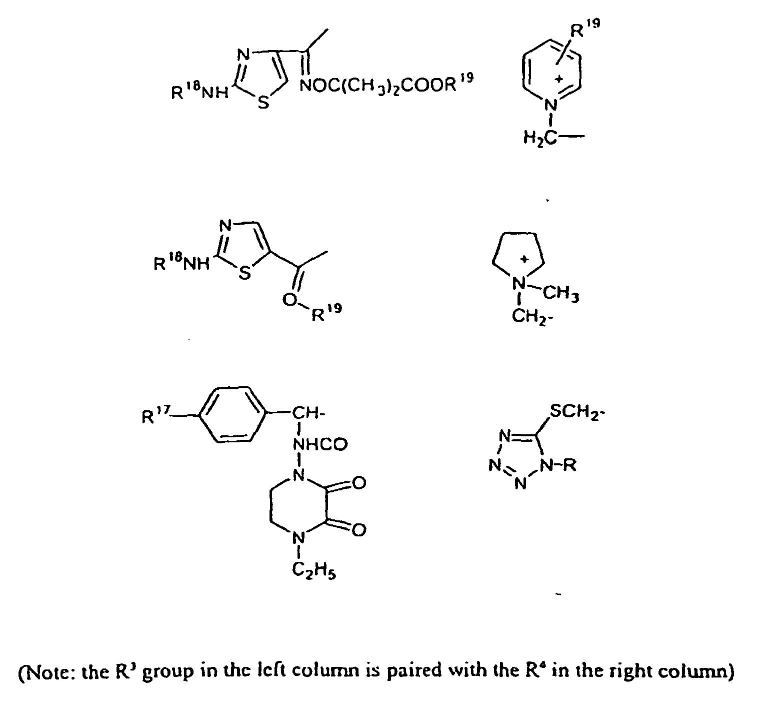
   wherein:
   R¹⁶ is a covalent bond that links the (b) group to the linker;
   one of R¹⁸ and R¹⁹ is hydrogen or alkyl and the other is a covalent bond that links the (b) group to the linker;
(iii) a compound of formula (c): 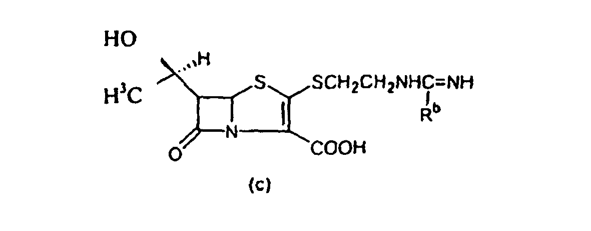 wherein R^{b} is a covalent bond linking (c) to the linker;
(iv) a compound of formula (d): 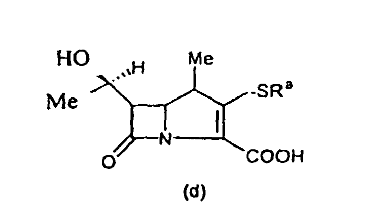 where R^{a} is: 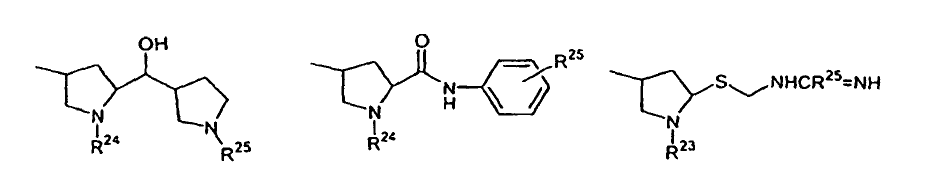 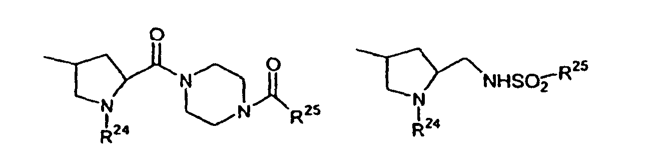 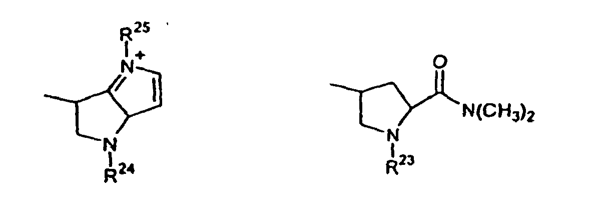 where:
   R²³ is a covalent bond that links (d) to the linker;
   one of R²⁴ and R²⁵ is alkyl, substituted alkyl, or aralkyl, and other is a covalent bond that links (d) to the linker; or
(v) a compound of formula (e): 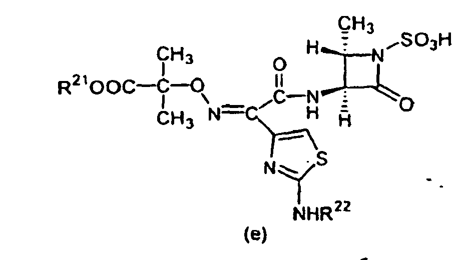
wherein one of R²¹ and R²² is hydrogen and the other links (d) to the linker; and pharmaceutically acceptable salts thereof.

Within the above preferred, more preferred and even more preferred compounds, a particularly preferred group of compounds is that wherein the linker is selected from a compound of formula:

-X^{a}-Z-(Y^{a}-Z)ₘ-X^{a}-

wherein
*m* is an integer of from 0 to 20;
X^{a} at each separate occurrence is selected from the group consisting of -O-, -S-, -NR-, -C(O)-, -C(O)O-, -OC(O)-, -C(O)NR-, -NRC(O)-, C(S), -C(S)O-, -C(S)NR-, -NRC(S)-, or a covalent bond where R is as defined below;
Z at each separate occurrence is selected from alkylene, substituted alkylene, cycloalkylene, substituted cylcoalkylene, alkenylene, substituted alkenylene, alkynylene, substituted alkynylene, cycloalkenylene, substituted cycloalkenylene, arylene, heteroarylene, heterocyclene, or a covalent bond;
each Y^{a} at each separate occurrence is selected from -O-, -C(O)-, -OC(O)-, -C(O)O-, -NR-, -S(O)n-, -C(O)NR'-, -NR'C(O)-, -NR'C(O)NR'-, -NR'C(S)NR'-, -C(=NR')-NR'-, -NR'-C(=NR')-, -OC(O)-NR'-, -NR'-C(O)-O-, -N=C(X^{a})-NR'-, -NR'-C(X^{a})=N-,-P(O)(OR')-O-, -O-P(O)(OR')-, -S(O)ₙCR'R"-, -S(O)ₙNR'-, -NR'-S(O)ₙ -, -S-S-, and a covalent bond; where *n* is 0, 1 or 2; and R, R' and R" at each separate occurrence are selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, aryl, heteroaryl and heterocyclic.

Compounds of the invention may be used for treating mammals having a disease state that is treatable by antibacterial agents, comprising administering a therapeutically effective amount of a compound of Formula I, or a mixture of compounds of Formula I, thereto.

The invention also relates to a pharmaceutical composition comprising a therapeutically effective amount of one or more compounds of Formula I or a pharmaceutically acceptable salt thereof, admixed with at least one pharmaceutically acceptable excipient.

### BRIEF DESCRIPTION OF THE DRAWINGS

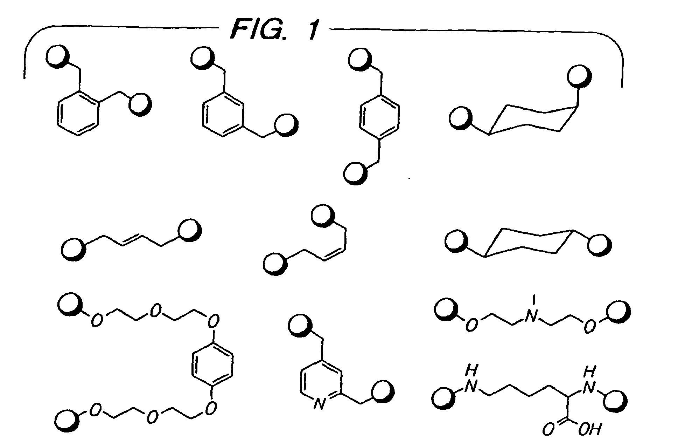

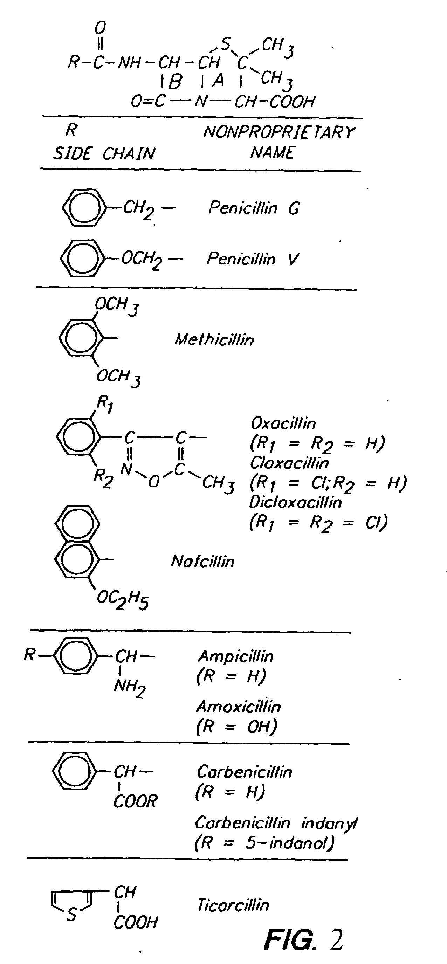

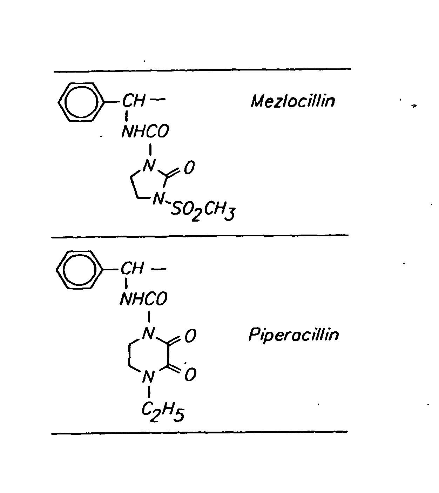

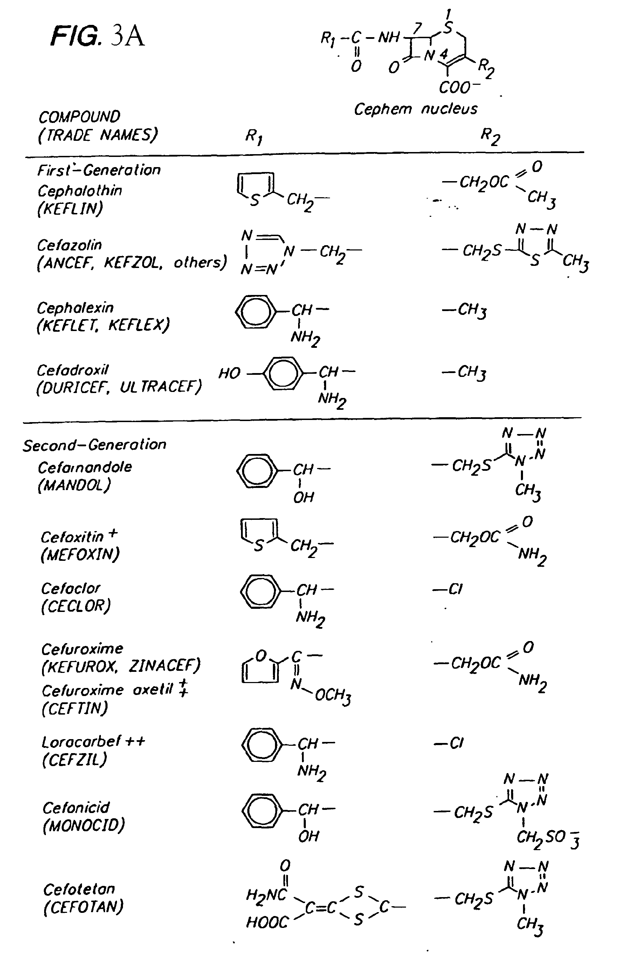

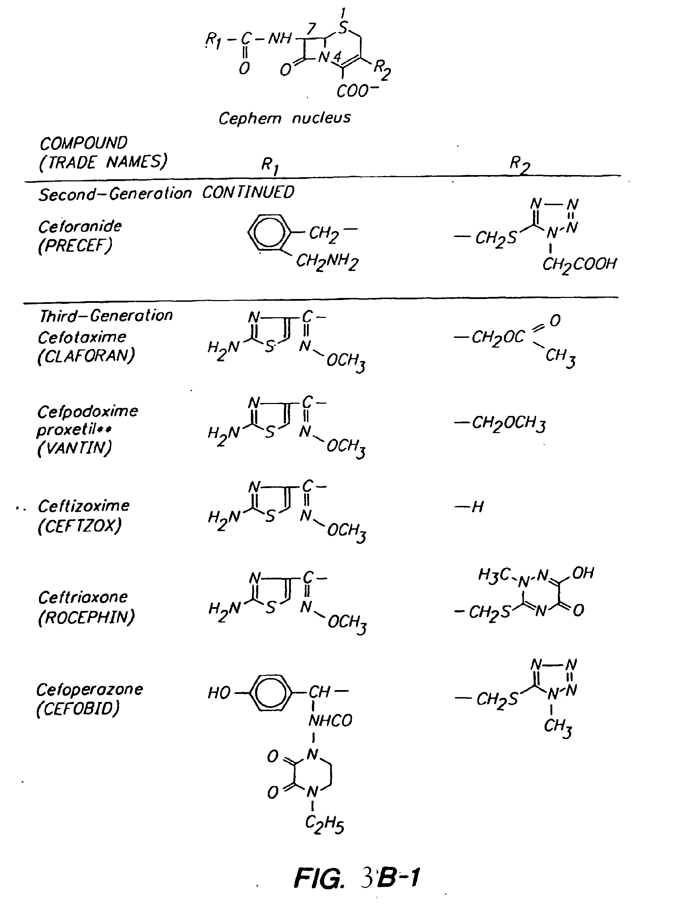

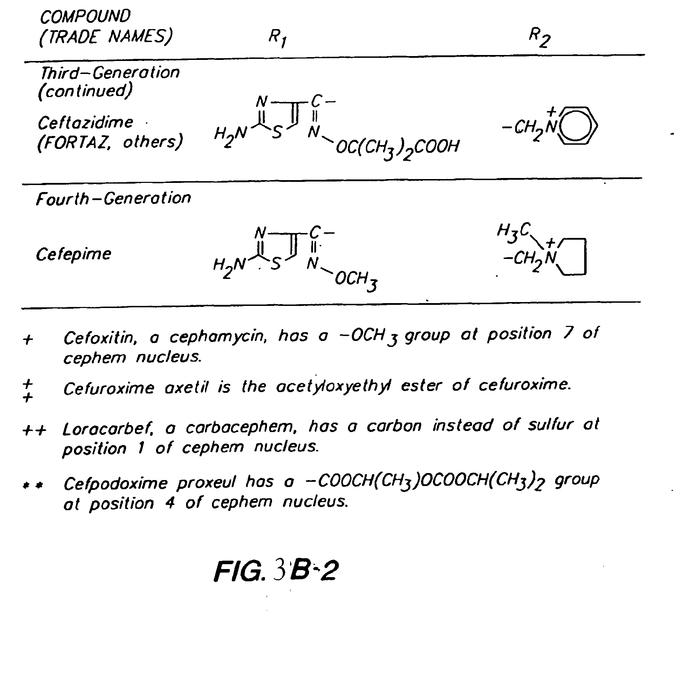

FIG. 1 illustrates examples of multibinding compounds comprising 2 ligands attached in different formats to a linker.
FIG.s 2, 3A, and 3B disclose some representative compounds of formula (a) and (b).
FIG.s 4 and 5 disclose examples of multibinding compounds comprising 2 ligands attached in different formats.
FIG.s 6-8 illustrate synthesis of compounds of Formula (I).

### DETAILED DESCRIPTION OF THE INVENTION

### Definitions

This invention is directed to multibinding compounds that are antibacterial agents and pharmaceutical compositions containing such compounds. When discussing such compounds, and compositions the following terms have the following meanings unless otherwise indicated. Any undefined terms have their art recognized meanings.

The term "alkyl" refers to a monoradical branched or unbranched saturated hydrocarbon chain preferably having from 1 to 40 carbon atoms, more preferably 1 to 10 carbon atoms, and even more preferably 1 to 6 carbon atoms. This term is exemplified by groups such as methyl, ethyl, *n*-propyl, *iso*-propyl, *n*-butyl, *iso*-butyl, *n*-hexyl, *n*-decyl, tetradecyl, and the like.

The term "substituted alkyl" refers to an alkyl group as defined above, having from 1 to 5 substituents, and preferably 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, guanidine, -C(=NR^{a})NHR^{b} (where R^{a} and R^{b} are independently selected from hydrogen, alkyl, aryl, aralkyl, heteroaryl, or heteroaralkyl), -NHSO₂NHR^{c} (where R^{c} is hydrogen, alkyl, aryl, aralkyl, heteroaryl, or heteroaralkyl) -SO-alkyl, -SO-substituted alkyl, - SO-aryl, -SO-heteroaryl, -SO₂-alkyl, -SO₂-substituted alkyl, -SO₂-aryl and -SO₂-heteroaryl. This term is exemplified by groups such as hydroxymethyl, hydroxyethyl, hydroxypropyl, 2-aminoethyl, 3-aminopropyl, 2-methylaminoethyl, 3-dimethylaminopropyl, 2-sulfonamidoethyl, 2-carboxyethyl, and the like.

The term "alkylene" refers to a diradical of a branched or unbranched saturated hydrocarbon chain, preferably having from 1 to 40 carbon atoms, more preferably 1 to 10 carbon atoms and even more preferably 1 to 6 carbon atoms. This term is exemplified by groups such as methylene (-CH₂-), ethylene (-CH₂CH₂-), the propylene isomers (e.g., -CH₂CH₂CH₂- and -CH(CH₃)CH₂-) and the like.

The term "substituted alkylene" refers to:
(a) an alkylene group, as defined above, having from 1 to 5 substituents, and preferably 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl and -SO₂-heteroaryl. Additionally, such substituted alkylene groups include those where 2 substituents on the alkylene group are fused to form one or more cycloalkyl, substituted cycloalkyl, cycloalkenyl, substituted cycloalkenyl, aryl, heterocyclic or heteroaryl groups fused to the alkylene group. Preferably such fused groups contain from 1 to 3 fused ring structures;
(b) an alkylene group as defined above wherein one or more carbons atoms, is replaced by oxygen, sulfur, and -NR- where R is hydrogen, substituted alkyl, cycloalkyl, alkenyl cycloalkenyl, alkynyl, aryl, heteroaryl and heterocyclic.

The term "alkaryl" or "aralkyl" refers to the groups -alkylene-aryl and -substituted alkylene-aryl where alkylene, substituted alkylene and aryl are defined herein. Such alkaryl groups are exemplified by benzyl, phenethyl and the like.

The term "alkoxy" refers to the groups alkyl-O-, alkenyl-O-, cycloalkyl-O-, cycloalkenyl-O-, and alkynyl-O-, where alkyl, alkenyl, cycloalkyl, cycloalkenyl, and alkynyl are as defined herein. Preferred alkoxy groups are alkyl-O- and include, by way of example, methoxy, ethoxy, *n*-propoxy, *iso*-propoxy, *n*-butoxy, *tert*-butoxy, *sec*-butoxy, *n*-pentoxy, *n-*hexoxy, 1,2-dimethylbutoxy, and the like.

The term "substituted alkoxy" refers to the groups substituted alkyl-O-, substituted alkenyl-O-, substituted cycloalkyl-O-, substituted cycloalkenyl-O-, and substituted alkynyl-O- where substituted alkyl, substituted alkenyl, substituted cycloalkyl, substituted cycloalkenyl and substituted alkynyl are as defined herein.

The term "alkenyl" refers to a monoradical of a branched or unbranched unsaturated hydrocarbon group preferably having from 2 to 40 carbon atoms, more preferably 2 to 10 carbon atoms and even more preferably 2 to 6 carbon atoms and having at least 1 and preferably from 1-6 sites of vinyl unsaturation. Preferred alkenyl groups include ethenyl (-CH=CH₂), *n*-propenyl (-CH₂CH=CH₂), *iso*-propenyl (-C(CH₃)=CH₂), and the like.

The term "substituted alkenyl" refers to an alkenyl group as defined above having from 1 to 5 substituents, and preferably 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl and -SO₂-heteroaryl.

The term "alkenylene" refers to a diradical of a branched or unbranched unsaturated hydrocarbon group preferably having from 2 to 40 carbon atoms, more preferably 2 to 10 carbon atoms and even more preferably 2 to 6 carbon atoms and having at least 1 and preferably from 1-6 sites of vinyl unsaturation. This term is exemplified by groups such as ethenylene (-CH=CH-), the propenylene isomers (e.g., -CH₂CH=CH-, -C(CH₃)=CH-, and the like.

The term "substituted alkenylene" refers to an alkenylene group as defined above having from 1 to 5 substituents, and preferably from 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl and -SO₂-heteroaryl. Additionally, such substituted alkenylene groups include those where 2 substituents on the alkenylene group are fused to form one or more cycloalkyl, substituted cycloalkyl, cycloalkenyl, substituted cycloalkenyl, aryl, heterocyclic or heteroaryl groups fused to the alkenylene group.

The term "alkynyl" refers to a monoradical of an unsaturated hydrocarbon preferably having from 2 to 40 carbon atoms, more preferably 2 to 20 carbon atoms and even more preferably 2 to 6 carbon atoms and having at least 1 and preferably from 1-6 sites of acetylene (triple bond) unsaturation. Preferred alkynyl groups include ethynyl (-C≡CH), propargyl (-CH₂C≡CH) and the like.

The term "substituted alkynyl" refers to an alkynyl group as defined above having from 1 to 5 substituents, and preferably 1 to 3 substituents, selected from alkoxy, substituted alkoxy, cycloalkyl, substituted cycloalkyl, cycloallcenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl, and -SO₂-heteroaryl.

The term "alkynylene" refers to a diradical of an unsaturated hydrocarbon preferably having from 2 to 40 carbon atoms, more preferably 2 to 10 carbon atoms and even more preferably 2 to 6 carbon atoms and having at least 1 and preferably from 1-6 sites of acetylene (triple bond) unsaturation. Preferred alkynylene groups include ethynylene (-C≡C-), propargylene (-CH₂C≡C-) and the like.

The term "substituted alkynylene" refers to an alkynylene group as defined above having from 1 to 5 substituents, and preferably 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl and -SO₂-heteroaryl.

The term "acyl" refers to the groups HC(O)-, alkyl-C(O)-, substituted alkyl-C(O)-, alkenyl-C(O)-, substituted alkenyl-C(O)-, cycloalkyl-C(O)-, substituted cycloalkyl-C(O)-, cycloalkenyl-C(O)-, substituted cycloalkenyl-C(O)-, aryl-C(O)-, heteroaryl-C(O)- and heterocyclic-C(O)- where alkyl, substituted alkyl, alkenyl, substituted alkenyl, cycloalkyl, substituted cycloalkyl, cycloalkenyl, substituted cycloalkenyl, aryl, heteroaryl and heterocyclic are as defined herein.

The term "acylamino" or "aminocarbonyl" refers to the group -C(O)NRR where each R is independently hydrogen, alkyl, substituted alkyl, aryl, heteroaryl, heterocyclic or where both R groups are joined to form a heterocyclic group (e.g., morpholino) wherein alkyl, substituted alkyl, aryl, heteroaryl and heterocyclic are as defined herein.

The term "sulfonylamino" refers to the group -NRSO₂R^{a} where R is hydrogen, alkyl, substituted alkyl, aralkyl, or heteroaralkyl, and R^{a} is alkyl, substituted alkyl, amino, or substituted amino wherein alkyl, substituted alkyl, aralkyl, heteroaralkyl and substituted amino are as defined herein.

The term "aminoacyl" refers to the group -NRC(O)R where each R is independently hydrogen, alkyl, substituted alkyl, alkenyl, substituted alkenyl, amino, substituted amino, aryl, heteroaryl, or heterocyclic wherein alkyl, substituted alkyl, alkenyl, substituted alkenyl, aryl, heteroaryl and heterocyclic are as defined herein.

The term "aminoacyloxy" or "alkoxycarbonylamino" refers to the group -NRC(O)OR where each R is independently hydrogen, alkyl, substituted alkyl, aryl, heteroaryl, or heterocyclic wherein alkyl, substituted alkyl, aryl, heteroaryl and heterocyclic are as defined herein.

The term "acyloxy" refers to the groups alkyl-C(O)O-, substituted alkyl-C(O)O-, cycloalkyl-C(O)O-, substituted cycloalkyl-C(O)O-, aryl-C(O)O-, heteroaryl-C(O)O-, and heterocyclic-C(O)O- wherein alkyl, substituted alkyl, cycloalkyl, substituted cycloalkyl, aryl, heteroaryl, and heterocyclic are as defined herein.

The term "aryl" refers to an unsaturated aromatic carbocyclic group of from 6 to 20 carbon atoms having a single ring (e.g., phenyl) or multiple condensed (fused) rings (e.g., naphthyl or anthryl). The aryl group may optionally be fused to a heterocyclic or cycloalkyl group. Preferred aryls include phenyl, naphthyl and the like. Unless otherwise constrained by the definition for the aryl substituent, such aryl groups can optionally be substituted with from 1 to 5 substituents, preferably 1 to 3 substituents, selected from the group consisting of acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, sulfonylamino, alkaryl, aryl, aryloxy, azido, carboxyl, carboxylalkyl, cyano, halo, nitro, heteroaryl, heteroaryloxy, heterocyclic, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO₂-alkyl, -SO₂-substituted alkyl, -SO₂-aryl, -SO₂₋heteroaryl and trihalomethyl. Preferred aryl substituents include alkyl, alkoxy, halo, cyano, nitro, trihalomethyl, and thioalkoxy.

The term "aryloxy" refers to the group aryl-O- wherein the aryl group is as defined above including optionally substituted aryl groups as also defined above.

The term "amino" refers to the group -NH₂.

The term "substituted amino" refers to the group -NRR where each R is independently selected from the group consisting of hydrogen, alkyl, substituted alkyl, acyl, cycloalkyl, substituted cycloalkyl, alkenyl, substituted alkenyl, cycloalkenyl, substituted cycloalkenyl, alkynyl, substituted alkynyl, aryl, heteroaryl and heterocyclic provided that both R's are not hydrogen.

The term "carboxyalkyl" or "alkoxycarbonyl" refers to the groups "-C(O)O-alkyl", "-C(O)O-substituted alkyl", "-C(O)O-cycloalkyl", "-C(O)O-substituted cycloalkyl", "-C(O)O-alkenyl", "-C(O)O-substituted alkenyl", "-C(O)O-alkynyl" and "-C(O)O-substituted alkynyl" where alkyl, substituted alkyl, cycloalkyl, substituted cycloalkyl, alkenyl, substituted alkenyl, alkynyl and substituted alkynyl are as defined herein.

The term "cycloalkyl" refers to cyclic alkyl groups of from 3 to 20 carbon atoms having a single cyclic ring or multiple condensed rings, said cycloalkyl group may optionally be fused to an aryl or heteroaryl group. Such cycloalkyl groups include, by way of example, single ring structures such as cyclopropyl, cyclobutyl, cyclopentyl, cyclooctyl, and the like, or multiple ring structures such as adamantanyl, and the like.

The term "substituted cycloalkyl" refers to cycloalkyl groups having from 1 to 5 substituents, and preferably 1 to 3 substituents, selected from alkyl, substituted alkyl, alkenyl, substituted alkenyl, alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂₋aryl and -SO₂-heteroaryl.

The term "cycloalkenyl" refers to cyclic alkenyl groups of from 4 to 20 carbon atoms having a single cyclic ring and at least one point of internal unsaturation. Examples of suitable cycloalkenyl groups include, for instance, cyclobut-2-enyl, cyclopent-3-enyl, cyclooct-3-enyl and the like.

The term "substituted cycloalkenyl" refers to cycloalkenyl groups having from 1 to 5 substituents, and preferably 1 to 3 substituents, selected from alkyl, substituted alkyl, alkenyl, substituted alkenyl, alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂₋aryl and -SO₂-heteroaryl.

The term "halo" or "halogen" refers to fluoro, chloro, bromo and iodo.

The term "heteroaryl" refers to an aromatic group of from 1 to 15 carbon atoms and 1 to 4 heteroatoms selected from oxygen, nitrogen and sulfur within at least one ring (if there is more than one ring). The heteroaryl ring may optionally be fused to a cycloalkyl or heterocyclyl ring. Unless otherwise constrained by the definition for the heteroaryl substituent, such heteroaryl groups can be optionally substituted with 1 to 5 substituents, preferably 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halo, nitro, heteroaryl, heteroaryloxy, heterocyclic, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO₂-alkyl, -SO₂-substituted alkyl, -SO₂-aryl, -SO₂-heteroaryl and trihalomethyl. Preferred heteroaryl substituents include alkyl, alkoxy, halo, cyano, nitro, trihalomethyl, and thioalkoxy. Such heteroaryl groups can have a single ring (e.g., pyridyl or furyl) or multiple condensed rings (e.g., indolizinyl or benzothienyl). Preferred heteroaryls include pyridyl, pyrrolyl and furyl.

The term "heteroaryloxy" refers to the group heteroaryl-O-.

The term "heterocycle" or "heterocyclyl" refers to a monoradical saturated unsaturated group having a single ring or multiple condensed rings, from 1 to 40 carbon atoms and from 1 to 10 hetero atoms, preferably 1 to 4 heteroatoms, selected from nitrogen, sulfur, phosphorus, and/or oxygen within the ring and further wherein one, two, or three of the ring carbon atoms may optionally be replaced with a carbonyl group (i.e., a keto group). The heterocycle group may optionally fused to an aryl or heteroaryl ring. Unless otherwise constrained by the definition for the heterocyclic substituent, such heterocyclic groups can be optionally substituted with 1 to 5, and preferably 1 to 3 substituents, selected from the group consisting of alkyl, acyloxy, alkyl, substituted alkyl, alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂₋aryl and -SO₂-heteroaryl. Such heterocyclic groups can have a single ring or multiple condensed rings. Preferred heterocyclics include morpholino, piperidinyl, and the like.

Examples of heteroaryls and heterocycles include, but are not limited to, pyrrole, thiophene, furan, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, pyrrolidine, piperidine, piperazine, indoline, morpholine, tetrahydrofuranyl, tetrahydrothiophene, and the like as well as N-alkoxy-nitrogen containing heterocycles.

The term "heterocyclooxy" refers to the group heterocyclic-O-.

The term "thioheterocyclooxy" refers to the group heterocyclic-S-.

The term "oxyacylamino" or "aminocarbonyloxy" refers to the group -OC(O)NRR where each R is independently hydrogen, alkyl, substituted alkyl, aryl, heteroaryl, or heterocyclic wherein alkyl, substituted alkyl, aryl, heteroaryl and heterocyclic are as defined herein.

The term "spiro-attached cycloalkyl group" refers to a cycloalkyl group joined to another ring via one carbon atom common to both rings.

The term "thiol" refers to the group -SH.

The term "thioalkoxy" or "alkylthio" refers to the group -S-alkyl.

The term "substituted thioalkoxy" refers to the group -S-substituted alkyl.

The term "thioaryloxy" refers to the group aryl-S- wherein the aryl group is as defined above including optionally substituted aryl groups also defined above.

The term "thioheteroaryloxy" refers to the group heteroaryl-S- wherein the heteroaryl group is as defined above including optionally substituted aryl groups as also defined above.

As to any of the above groups which contain one or more substituents, it is understood, of course, that such groups do not contain any substitution or substitution patterns which are sterically impractical and/or synthetically non-feasible. In addition, the compounds of this invention include all stereochemical isomers arising from the substitution of these compounds.

The term "pharmaceutically-acceptable salt" refers to salts which retain the biological effectiveness and properties of the multibinding compounds of this invention and which are not biologically or otherwise undesirable. In many cases, the multibinding compounds of this invention are capable of forming acid and/or base salts by virtue of the presence of amino and/or carboxyl groups or groups similar thereto.

Pharmaceutically-acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group. Examples of suitable amines include, by way of example only, isopropylamine, trimethyl amine, diethyl amine, tri(*iso-*propyl) amine, tri(n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like. It should also be understood that other carboxylic acid derivatives would be useful in the practice of this invention, for example, carboxylic acid amides, including carboxamides, lower alkyl carboxamides, dialkyl carboxamides, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like.

The term "pharmaceutically-acceptable cation" refers to the cation of a pharmaceutically-acceptable salt.

The term "pseudohalide" refers to functional groups which react in displacement reactions in a manner similar to a halogen. Such functional groups include, by way of example, mesyl, tosyl, azido and cyano groups.

The term "protecting group" or "blocking group" refers to any group which when bound to one or more hydroxyl, thiol, amino or carboxyl groups of the compounds (including intermediates thereof) prevents reactions from occurring at these groups and which protecting group can be removed by conventional chemical or enzymatic steps to reestablish the hydroxyl, thiol, amino or carboxyl group (*See.,* T.W. Greene and P.G.H. Wuts, *"Protective Groups in Organic Synthesis",* 2^{nd} Ed.). The particular removable blocking group employed is not critical and preferred removable hydroxyl blocking groups include conventional substituents such as allyl, benzyl, acetyl, chloroacetyl, thiobenzyl, benzylidine, phenacyl, t-butyl-diphenylsilyl and any other group that can be introduced chemically onto a hydroxyl functionality and later selectively removed either by chemical or enzymatic methods in mild conditions compatible with the nature of the product. Preferred removable thiol blocking groups include disulfide groups, acyl groups, benzyl groups, and the like.

Preferred removable amino blocking groups include conventional substituents such as t-butyoxycarbonyl (t-BOC), benzyloxycarbonyl (CBZ), fluorenylmethoxy-carbonyl (FMOC), allyloxycarbonyl (ALOC), and the like which can be removed by conventional conditions compatible with the nature of the product.

Preferred carboxyl protecting groups include esters such as methyl, ethyl, propyl, *t-*butyl etc. which can be removed by mild conditions compatible with the nature of the product.

The term "optional" or "optionally" means that the subsequently described event, circumstance or substituent may or may not occur, and that the description includes instances where said event or circumstance occurs and instances where it does not.

The term "ligand" or " ligands" as used herein denotes a compound that is a binding partner for penicillin binding proteins, a pencillinase enzyme, a cephalosporinase enzyme, a transpeptidase enzyme, a substrate of a transpeptidase enzyme, a beta-lactamase enzyme, a transglycoslase enzyme, or a transglycosylase enzyme substrate and is bound thereto by complementarity. The specific region or regions of the ligand that is (are) recognized by the penicillin binding proteins, a pencillinase enzyme, a cephalosporinase enzyme, a transpeptidase enzyme, a substrate of a transpeptidase enzyme, a beta-lactamase enzyme, a transglycoslase enzyme, or a transglycosylase enzyme substrate is designated as the "ligand domain". A ligand may be either capable of binding to its target by itself, or may require the presence of one or more non-ligand components for binding (e.g., Ca⁺², Mg⁺² or a water molecule is required for the binding of a ligand to various ligand binding sites). Examples of ligands useful in this invention are described herein. Those skilled in the art will appreciate that portions of the ligand structure that are not essential for specific molecular recognition and binding activity may be varied substantially, replaced or substituted with unrelated structures (for example, with ancillary groups as defined below) and, in some cases, omitted entirely without affecting the binding interaction. The primary requirement for a ligand is that it has a ligand domain as defined above. It is understood that the term ligand is not intended to be limited to compounds known to be useful in binding to penicillin binding proteins, a pencillinase enzyme, a cephalosporinase enzyme, a transpeptidase enzyme, a substrate of a transpeptidase enzyme, a beta-lactamase enzyme, a transglycoslase enzyme, or a transglycosylase enzyme substrate (e.g., known drugs). Those skilled in the art will understand that the term ligand can equally apply to a molecule that is not normally associated with penicillin binding proteins, a transglycoslase enzyme, or a transglycosylase enzyme substrate binding properties. The term "ligand" or " ligands" as used herein is intended to include the racemic forms of the ligands as well as individual enantiomers and diasteromers and non-racemic mixtures thereof.

The term "β-lactam antibiotic" refers to antibiotics, having a β-lactam ring core which can be depicted as follows:

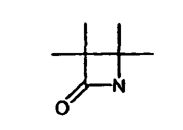

The β-lactam antibiotics are classified into the penicillins, cephalosporins, carbapenems, oxacephems, carbacephems, and monobactams and include drugs such as Penicillin G, Penicillin V, Methicillin, Oxacillin, Cloxacillin, Dicioxacillin, Nafcillin, Ampicillin, Amoxicillin, Carbenicillin, Carbenicillin indanyl, Ticarcillin, Mezlocillin, Piperacillin Cephalothin, Cefazolin, Cephalexin, Cefadroxil, Cefamandole, Cefoxitin, Cefaclor, Cefuroxime, Cefuroxime axetil, Loracarbef, Cefonicid, Cefotetan, Ceforanide, Cefotaxime, Cefpodoxime proxetil, Ceftizoxime, Ceftriaxone, Cefoperazone, Ceftazidime, Cefepime Imipenem, Meropenem, Aztreonam, Ritipenem, L-695256, GV-143253, Sanifitrinem, Fropenem, Lactivicin, BO-2727, MEN-10700, Ro-48-8724, Cefosilis, SB-216477, S-4661, GG-326, BLA-857, PGE-8335534, PGE-542860, LB-10522, GV-129606, BO-2052A, CS-834, MK-826, YH-1226, YM-40220, MDL-63908, FCE-25199, Panipenem, TOC-50, TOC-39, TOC-29, E-1101, Sulopenem, DU-6681, MC-02479, Temocillin, Carumonam, Ro-25-0534, SUN-A-0026, WS-1358A, Ro-25-1132, CGP-57701, CGP-37697A, TMA-230, Syn-2190, Biapenem, CS-834, DWP-204, DX-8739, CS-976, CKD-529, ER-35786, DZ-2640, 4-AAz, KR-21012, R0-25-0993, DA-1211, BMS-181139, J-11225, L-786392, DK-35C, Ro-25-6833, S-1090, E-1101, FK-518, DP-736, Cefditoren, LY-215891, R0-09-1428, Cefdaloxime, Cefoselis, KST-150185, Ro-09-1227, Cefclidin, Cefluprenam, Cefotiam, LB-10522, Cefcanel, BRL-57342, Cefprirome, YH-1226, Cefprozil, CKD-604, KST-150288, Cefcapene, Ro-24-8138, FK-312, Cefozopran, RU-59863, Ceftibuten, FR-193879, FK-041, Cefdinir, CP-6679, R0-63-9141, CFC-240, Cefpimizole, Cefminox, Cefetamet, CP-0467, PGE-7119699, R0-48-8391, AM-1817, AM-1732, MC-02002, BO-1341, BK-218, Ro-25-4835, R0-25-2016, YM-40220, Ro-23-9424, LY-206763, CR-240, YH-1266, MC-02331, Ro-44-3949, MC-02306, Ro-25-7103, BMS-180680. Preferred β-lactam antibiotics are Amoxicillin, Nafcillin, Cefadroxil, Ceftriaxone, Cefaclor, Aztreonam, Ceftazidime, Imipenem, Meropenem, Ritipenem, Ceftazidine, Pipericillin, Clauvlinic acid, Cefepime, Cefoxitin, Cefotaxime, Cefixime, Lefluzidine and derivatives thereof.

The glycopeptide antibiotics are characterized by a multi-ring peptide core and at least one sugar attached at various sites, of which vancomcin is an important example. Examples of the glycopeptide class of ligands included in this definition my be found in "Glycopeptides Classification, Occurrence, and and Discovery" by Rao, R.C. and Crandall, L.W., (Drugs and the Pharmaceutical Sciences" Vol. 63, edited by Ramakrishnan Nagarajan, published by Marcal Dekker, Inc.) which is hereby incorporated by reference. Disclosed are glycopeptides identified as Actaplanin, Actinodidin, Ardacin, Avoparcin, Azureomycin, A477, A35512, A40926, A41030, A42867, A47934, A80407, A82846, A83850, A84575, A84428, AB-65, Balhimycin, Chloroeremomycin, Chloroorientiein, Chlompolysporin, Decaplanin, N-demethylvancomycin, Eremomycin, Galacardin, Helvecardin. Izupeptin, Kibdelin, LL-AM374, Mannopeptin, MM45289, MM47756, MM47761, MM47921, MM47766, MM55260, MM55266, MM55270, MM56579, MM56598, OA-7653, Oreenticin, Parvodicin. Ristocetin, Ristomycin, Synmonicin, Teicoplanin, UK-68597, UK-69542, UK-72051, Vancomycin, and the like. Another preferred class of ligands is the general class of glycopeptides disclosed above on which the sugar moiety is absent. For example removal of the disaccharide moiety appended to the phenol on vancomycin (as shown below as Formula II) by mild hydrolysis gives vancomycin aglycone. A further preferred class are glycopeptides that have been further appended with additional saccharide residues, especially aminoglycosides, in a manner similar to vancosamine.

"Vancomycin" refers to the antibacterial compound whose structure is reproduced below as Formula II.

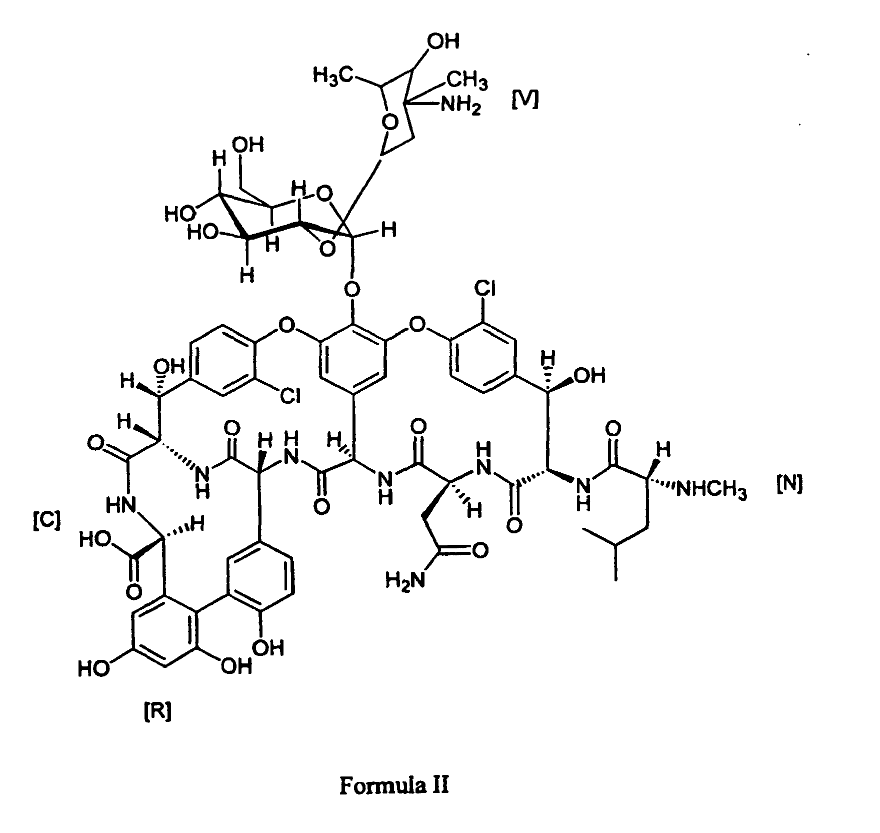

"Optional" or "optionally" means that the subsequently described event or circumstance may or may not occur, and that the description includes instances where said event or circumstance occurs and instances in which it does not. For example. "Optionally substituted glycopeptide" with respect to a compound of Formula I refers to a ligand as defined above in which those positions that are not linked to X may or may not be substituted by various groups as defined below. The term also includes those instances in which one amino acid of the basic core structure is replaced by another amino acid, for example as described in "Preparation and conformational analysis of vancomycin hexapeptide and aglucovancomycin hexapeptide", by Booth, P. M., Williams, D.H., Univ. Chem. Lab., Cambridge, UK., J. Chem. Soc., Perkin Trans. I (1989), (12), 2335-9, and "The Edman degradation of vancomycin:preparation of vancomycin hexapeptide", Booth, P.M., Stone, D.J.M, Williams, D.H., Univ. Chem. Lab., Cambridge, UK., J. Chem. Soc., Chem. Commun. (1987), (22), 1694-5. "Optionally substituted vancomycin" with respect to the multibinding agents of the invention refers to vancomycin in which the hydroxy group at any position, the [R] position, the carboxyl groups at the [C] position, or the amine groups at the [V] or [N] position that are not attached to the linker X may or may not be substituted by various groups. Such groups include:R^{a} where R^{a} at each occurrence is chosen from alkyl, alkyl optionally interrupted by 1-5 atoms chosen from O, S, or -NR^{b}- where R^{b} is alkyl, aryl, or heteroaryl, all of which are optionally substituted, haloalkyl, alkenyl, alkynyl, alkylamino, alkylaminoalkyl, cycloalkyl, alkanoyl, aryl, heteroaryl, heterocyclic, additional saccharide residues, especially aminoglycosides, all of which are optionally substituted as defined above; and: NR^{c}R^{d} in which R^{c} and R^{d} are independently hydrogen, alkyl, alkenyl, alkynyl, cycloalkyl, alkanoyl, aryl, heteroaryl, arylalkyl, or heteroarylalkyl, or R^{c} and R^{d} when taken together with the nitrogen atom to which they are attached represent a heterocyclic group, quarternary alkyl and aryl ammonium compounds, pyidinium ions, sulfonium ions, and the like, all of which are optionally substituted as defined above. An example of a preferred [C] substitution is dimethylaminopropylamino and glucosamino; and example of a preferred [V] substitution is alkyl, for example n-decyl, or alkylaminoalkyl, for example n-decylaminoethyl. "Optionally substituted vancomycin aglycone" with respect to the multibinding agents of the invention refers to vancomycin aglycone in which the hydroxy group at any position, particularly the hydroxy group at the [O] position, the [R] position, the carboxy groups at the [C] position, or the amine group at the [N] position, that are not attached to the linker X may or may not be substituted by various groups -R^{a} as defined above.

"Transglycosylase enzyme substrate" as used herein denotes the molecular target of the transglycosylase enzyme. The substrate binds to the enzyme and eventually results in the synthesis of the bacterial cell wall. The action of this enzyme is inhibited by a ligand domain that binds to the enzyme itself and/or the enzyme substrate. A ligand such as vancomycin binds to this substrate and in effect "sequesters" the substrate to prevent its recognition by the enzyme and subsequent use in the construction of the bacterial cell wall. There is also a growing feeling that some glycopeptides or derivatives thereof may directly bind to and inhibit the transglycolase.

The term "potency" refers to the minimum concentration at which a ligand is able to achieve a desirable biological or therapeutic effect. The potency of a ligand is typically proportional to its affinity for its ligand binding site. In some cases, the potency may be non-linearly correlated with its affinity. In comparing the potency of two drugs, e.g., a multibinding agent and the aggregate of its unlinked ligand, the dose-response curve of each is determined under identical test conditions (e.g., in an *in vitro* or *in vivo* assay, in an appropriate animal model). The finding that the multibinding agent produces an equivalent biological or therapeutic effect at a lower concentration than the aggregate unlinked ligand is indicative of enhanced potency.

The term "selectivity" or "specificity" is a measure of the binding preferences of a ligand for different ligand binding sites (receptors). The selectivity of a ligand with respect to its target ligand binding site relative to another ligand binding site is given by the ratio of the respective values of K_{d} (i.e., the dissociation constants for each ligand-receptor complex) or, in cases where a biological effect is observed below the K_{d}, the ratio of the respective EC₅₀'s (i.e., the concentrations that produce 50% of the maximum response for the ligand interacting with the two distinct ligand binding sites (receptors)).

The term "ligand binding site" denotes the site on a penicillin binding proteins, a transpeptidase enzyme, penicillinase enzyme, cephalosporinase enzyme, beta lactamase enzyme, a transpeptidase enzyme substrate, a transglycosylase enzyme and/or transglycosylase enzyme substrate that recognizes a ligand domain and provides a binding partner for the ligand. The ligand binding site may be defined by monomeric or multimeric structures. This interaction may be capable of producing a unique biological effect, for example, agonism, antagonism, and modulatory effects, or it may maintain an ongoing biological event, and the like.

It should be recognized that the ligand binding sites of the enzyme or the receptor that participate in biological multivalent binding interactions are constrained to varying degrees by their intra- and inter-molecular associations. For example, ligand binding sites may be covalently joined to a single structure, noncovalently associated in a multimeric structure, embedded in a membrane or polymeric matrix, and so on and therefore have less translational and rotational freedom than if the same structures were present as monomers in solution.

The term "inert organic solvent" or "inert solvent" means a solvent which is inert under the conditions of the reaction being described in conjunction therewith including, by way of example only, benzene, toluene, acetonitrile, tetrahydrofuran, dimethylformamide, chloroform, methylene chloride, diethyl ether, ethyl acetate, acetone, methylethyl ketone, methanol, ethanol, propanol, isopropanol, *t*-butanol, dioxane, pyridine, and the like. Unless specified to the contrary, the solvents used in the reactions described herein are inert solvents.

The term "treatment" refers to any treatment of a pathologic condition in a mammal, particularly a human, and includes:
(i) preventing the pathologic condition from occurring in a subject which may be predisposed to the condition but has not yet been diagnosed with the condition and, accordingly, the treatment constitutes prophylactic treatment for the disease condition;
(ii) inhibiting the pathologic condition, i.e., arresting its development;
(iii) relieving the pathologic condition, i.e., causing regression of the pathologic condition; or
(iv) relieving the conditions mediated by the pathologic condition.

The term "pathologic condition which is modulated by treatment with a ligand" covers all disease states (i.e., pathologic conditions) which are generally acknowledged in the art to be usefully treated with a ligand that is an antibacterial agent, and those disease states which have been found to be usefully treated by a specific multibinding compound of our invention.

The term "therapeutically effective amount" refers to that amount of multibinding compound which is sufficient to effect treatment, as defined above, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

The term "linker", identified where appropriate by the symbol 'X' refers to a group or groups that covalently attaches the 2 ligands (as identified above) in a manner that provides for a compound capable of multivalency. In some cases, the linker may itself be biologically active. The term "linker" does not, however, extend to cover solid inert supports such as beads, glass particles, fibers, and the like. But it is understood that the multibinding compounds of this invention can be attached to a solid support if desired. For example, such attachment to solid supports can be made for use in separation and purification processes and similar applications.

The extent to which multivalent binding is realized depends upon the efficiency with which the linker that joins the ligands presents these ligands to the array of available ligand binding sites. Beyond presenting these ligands for multivalent interactions with ligand binding sites, the linker spatially constrains these interactions to occur within dimensions defined by the linker. Thus, the structural features of the linker (valency, geometry, orientation, size, flexibility, chemical composition, etc.) are features of multibinding agents that play an important role in determining their activities.

The linkers used in this invention are selected to allow multivalent binding of ligands to the ligand binding sites of an enzyme involved in cell wall biosynthesis and metabolism, a precursor used in the synthesis of the bacterial cell wall and/or the cell surface, whether such sites are located interiorly, both interiorly and on the periphery of the enzyme structure, or at any intermediate position thereof.

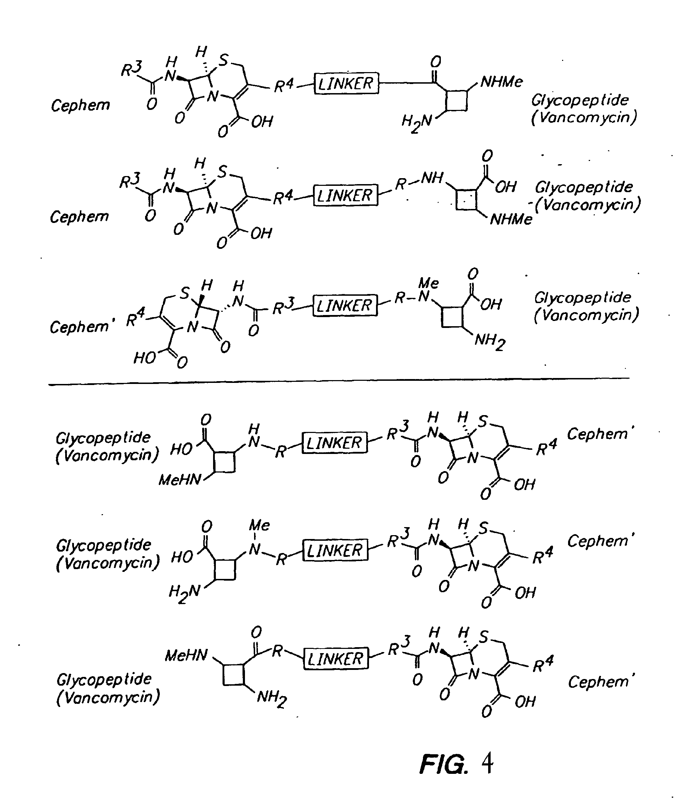

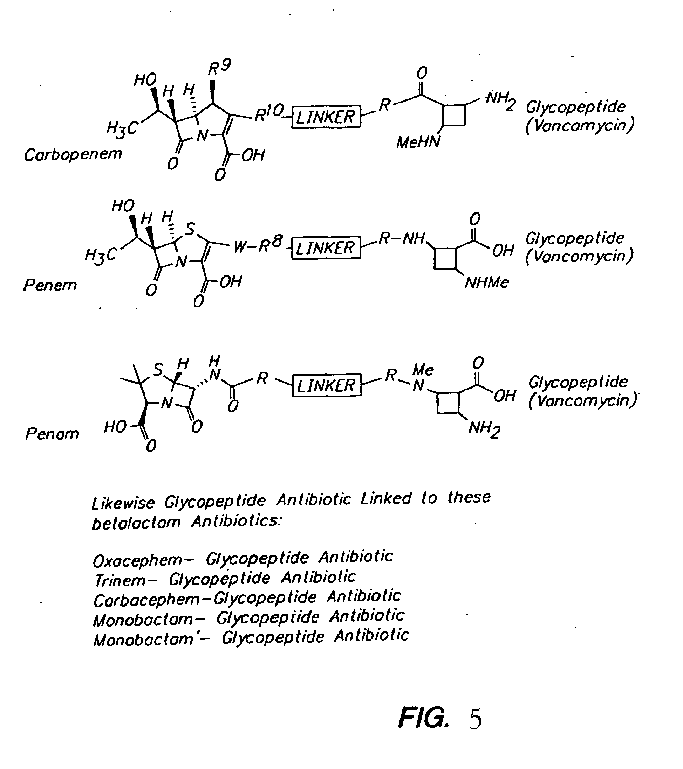

In the figures 4-8, glycopeptides are depicted in a simplified form as a shaded box that shows only the carboxy terminus, labeled [C], the sugar amine terminus (e.g., vancosamine), labeled [V], and the "non-sugar" amino terminus, labeled [N] as follows: 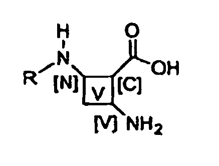 where R is hydrogen (as N-desmethylvancomycin) or methyl (as in vancomycin).

It can be seen by way of exemplification that one class of multivalent compounds that fall within the scope of the definition of Formula I include compounds wherein the glycopeptide ligand is connected by the linker at the [C], [V], or [N] terminus.

Another class of multivalent compounds that fall within the scope of the definition of Formula I include compounds which are the aglycone derivatives of glycopeptides. These are depicted as a triangle that shows only the carboxyl terminus, labeled [C], the aglycone hydroxy terminus. labeled [O], and the "non-sugar" amino terminus, labeled [N] as follows: 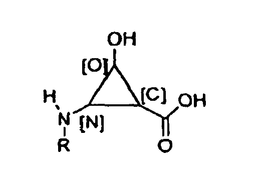 where R is hydrogen (as in N-desmethylvancomycin aglycone) or methyl (as in vancomycin aglycone) wherein the aglycone derivatives ligand is connected by one or more linkers at the [C], [V], or [N] terminus..

A third class of compounds falling within the scope of the invention are those in which the glycopeptides, or aglycone derivatives thereof, are linked via the [R] position. Reaction schemes that exemplify this linking strategy depict the ligands in a simplified form as above, i.e., as a shaded box in which the carboxyl terminus is labeled [C], the vancosamine amino terminus is labeled [V], and the "non-sugar" amino terminus is labeled [N], with the addition of the [R] position as a resorcinol derivative as shown below: 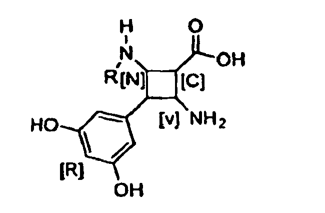 where R is hydrogen or methyl.

### GENERAL SYNTHETIC SCHEME

Compounds of this invention can be made by the methods depicted in the reaction schemes shown below.

The starting materials and reagents used in preparing these compounds are either available from commercial suppliers such as Aldrich Chemical Co., (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as Fieser and Fieser's Reagents for Organic Synthesis, Volumes 1-15 (John Wiley and Sons, 1991); Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989).

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

Furthermore, it will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and G. M. Wuts, *Protecting Groups in Organic Synthesis,* Second Edition, Wiley, New York, 1991, and references cited therein.

These schemes are merely illustrative of some methods by which the compounds of this invention can be synthesized, and various modifications to these schemes can be made and will be suggested to one skilled in the art having referred to this disclosure.

### Preparation of a multibinding compound of Formula (I)

In general, a bivalent multibinding compound of Formula (I) can be prepared as illustrated and described in Scheme A below.

A bivalent multibinding compound of Formula (I) can be prepared by covalently attaching the ligands, L, to a linker, X, as shown in Scheme A below.

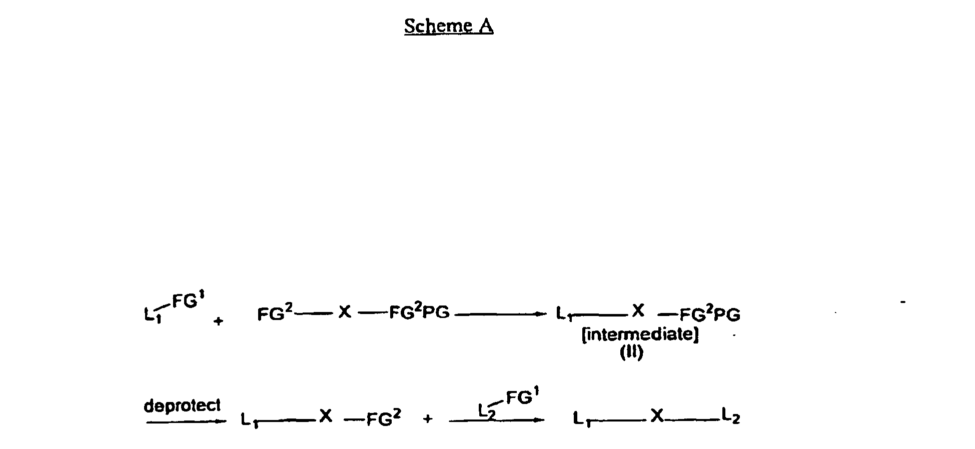

The compounds of Formula (I) are prepared in a stepwise manner by covalently attaching one equivalent of a ligand, L₁, with a ligand X where FG¹ and FG² represent a functional group as defined above, and FG²PG is a protected functional group to give an intermediate of formula (II). Deprotection of the second functional group on the ligand, followed by reaction with a ligand L₂, then provides a compound of Formula (I).

The ligands are covalently attached to the linker using conventional chemical techniques providing for covalent linkage of the ligand to the linker. Reaction chemistries resulting in such linkages are well known in the art and involve the use of complementary functional groups on the linker and ligand as shown in Table I below.

**Table I Representative Complementary Binding Chemistries**

| First Reactive Group | Second Reactive Group | Linkage |
|---|---|---|
| carboxyl | amine | amide |
| sulfonyl halide | amine | sulfonamide |
| hydroxyl | alkyl/aryl halide | ether |
| hydroxyl | isocyanate | urethane |
| amine | epoxide | β-hydroxyamine |
| amine | alkyl/aryl halide | alkylamine |
| hydroxyl | carboxyl | ester |
| amine | aldehyde/NaCNBH₃ | amine |
| hydroxylamine | sulfonyl halide | sulfonamide |
| aldehyde | amine/NaCHBH₃ | amine |
| aldehyde | amine/NaCHBH₃ | amine |
| amine | isocynate | urea |

By way of example, reaction between a carboxylic acid of either the linker or the β-lactam and a primary or secondary amine of the β-lactam or the linker in the presence of suitable, well-known activating agents such as dicyclohexylcarbodiimide, results in formation of an amide bond covalently linking the β-lactam to the linker; reaction between an amine group of either the linker or the β-lactam and a sulfonyl halide of the β-lactam or the linker, in the presence of a base such as triethylamine, pyridine, an the like results in formation of a sulfonamide bond covalently linking the β-lactam to the linker; and reaction between an alcohol or phenol group of either the linker or the β-lactam and an alkyl or aryl halide of the β-lactam or the β-lactam in the presence of a base such as triethylamine, pyridine, and the like, results in formation of an ether bond covalently linking the β-lactam to the linker.

Typically, a compound selected for use as a ligand will have at least one functional group, such as an amino, hydroxyl, thiol or carboxyl group and the like, which allows the compound to be readily coupled to the linker. Compounds having such functionality are either known in the art or can be prepared by routine modification of known compounds using conventional reagents and procedures.

Linkers can be attached to different positions on the ligand molecule to achieve different orientations of the ligand domains, and thereby facilitate multivalency. While a number of positions on the ligands are synthetically practical for linking, it is preferred to preserve those ligand substructures which are most important for ligand-receptor binding.

The linker is attached to the ligand at a position that retains ligand domain-ligand binding site interaction and specifically which permits the ligand domain of the ligand to orient itself to bind to the ligand binding site. Such positions and synthetic protocols for linkage are well known in the art. The term linker embraces everything that is not considered to be part of the ligand.

The relative orientation in which the ligand domains are displayed derives from the particular point or points of attachment of the ligands to the linker, and on the framework geometry. The determination of where acceptable substitutions can be made on a ligand is typically based on prior knowledge of structure-activity relationships (SAR) of the ligand and/or congeners and/or structural information about ligand-receptor complexes (e.g., X-ray crystallography, NMR, and the like). Such positions and the synthetic methods for covalent attachment are well known in the art. Following attachment to the selected linker (or attachment to a significant portion of the linker, for example 2-10 atoms of the linker), the univalent linker-ligand conjugate may be tested for retention of activity in the relevant assay.

The linker, when covalently attached to the ligands, provides a biocompatible, substantially non-immunogenic multibinding compound. The biological activity of the multibinding compound is highly sensitive to the valency, geometry, composition, size, flexibility or rigidity, etc. of the linker and, in turn, on the overall structure of the multibinding compound, as well as the presence or absence of anionic or cationic charge, the relative hydrophobicity/hydrophilicity of the linker, and the like on the linker. Accordingly, the linker is preferably chosen to maximize the biological activity of the multibinding compound. The linker may be chosen to enhance the biological activity of the molecule. In general, the linker may be chosen from any organic molecule construct that orients two ligands to their ligand binding sites to permit multivalency. In this regard, the linker can be considered as a "framework" on which the ligands are arranged in order to bring about the desired ligand-orienting result, and thus produce a multibinding compound.

For example, different orientations can be achieved by including in the framework groups containing mono- or polycyclic groups, including aryl and/or heteroaryl groups, or structures incorporating one or more carbon-carbon multiple bonds (alkenyl, alkenylene, alkynyl or alkynylene groups). Other groups can also include oligomers and polymers which are branched- or straight-chain species. In preferred embodiments, rigidity is imparted by the presence of cyclic groups (e.g., aryl, heteroaryl, cycloalkyl, heterocyclic, etc.). In other preferred embodiments, the ring is a six or ten member ring. In still further preferred embodiments, the ring is an aromatic ring such as, for example, phenyl or naphthyl.

Different hydrophobic/hydrophilic characteristics of the linker as well as the presence or absence of charged moieties can readily be controlled by the skilled artisan. For example, the hydrophobic nature of a linker derived from hexamethylene diamine (H₂N(CH₂)₆NH₂) or related polyamines can be modified to be substantially more hydrophilic by replacing the alkylene group with a poly(oxyalkylene) group such as found in the commercially available "Jeffamines".

Different frameworks can be designed to provide preferred orientations of the ligands. Such frameworks may be represented by using an array of dots (as shown below) wherein each dot may potentially be an atom, such as C, O, N, S, P, H, F, Cl, Br, and F or the dot may alternatively indicate the absence of an atom at that position. To facilitate the understanding of the framework structure, the framework is illustrated as a two dimensional array in the following diagram, although clearly the framework is a three dimensional array in practice:

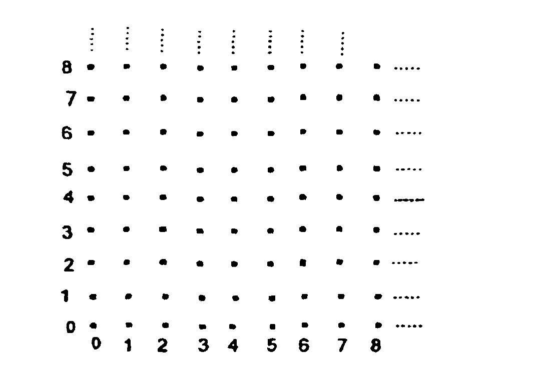

Each dot is either an atom, chosen from carbon, hydrogen, oxygen, nitrogen, sulfur, phosphorus, or halogen, or the dot represents a point in space (i.e., an absence of an atom). As is apparent to the skilled artisan, only certain atoms on the grid have the ability to act as an attachment point for the ligands, namely, C, O, N, S and P.

Atoms can be connected to each other via bonds (single, double or triple bonds with acceptable resonance and tautomeric forms), with regard to the usual constraints of chemical bonding. Ligands may be attached to the framework via single, double or triple bonds (with chemically acceptable tautomeric and resonance forms). Preferably, the linker connections to the ligand is selected such that the maximum spatial distance between two adjacent ligands is no more than 100Å.

An example of a linker as presented by the grid is shown below for a biphenyl construct.

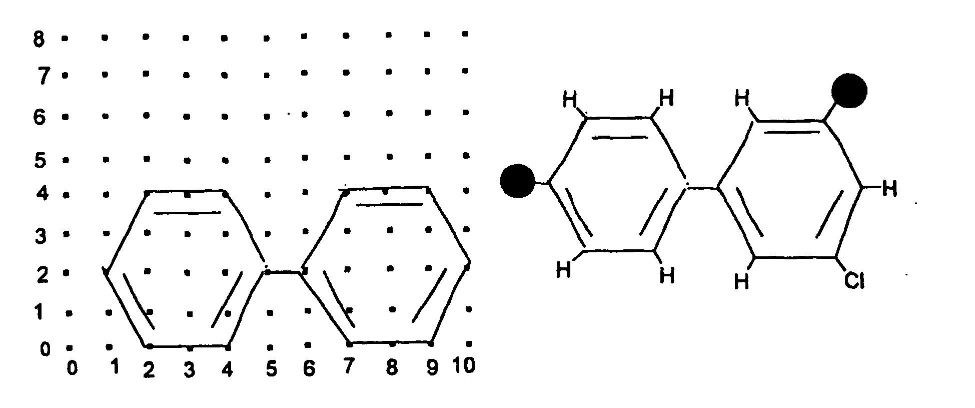

Nodes (1,2), (2,0), (4,4), (5,2), (4,0), (6,2), (7,4), (9,4), (10,2), (9,0), (7,0) all represent carbon atoms. Node (10,0) represents a chlorine atom. All other nodes (or dots) are points in space (i.e., represent an absence of atoms).

Nodes (1,2) and (9,4) are attachment points. Hydrogen atoms are affixed to nodes (2,4), (4,4), (4,0), (2,0), (7,4), (10,2) and (7,0). Nodes (5,2) and (6,2) are connected by a single bond.

The carbon atoms present are connected by either a single or double bonds, taking into consideration the principle of resonance and/or tautomerism.

The intersection of the framework (linker) and the ligand group, and indeed, the framework (linker) itself can have many different bonding patterns. Examples of acceptable patterns of three contiguous atom arrangements are shown in the following diagram:

| | | | | |
|---|---|---|---|---|
| CCC | NCC | OCC | SCC | PCC |
| CCN | NCN | OCN | SCN | PCN |
| CCO | NCO | OCO | SCO | PCO |
| CCS | NCS | OCS | SCS | PCS |
| CCP | NCP | OCP | SCP | PCP |
| | | | | |
| CNC | NNC | ONC | SNC | PNC |
| CNN | NNN | ONN | SNN | PNN |
| CNO | NNO | ONO | SNO | PNO |
| CNS | NNS | ONS | SNS | PNS |
| CNP | NNP | ONP | SNP | PNP |
| | | | | |
| COC | NOC | OOC | SOC | POC |
| COO | NON | OON | SON | PON |
| COC | NOO | OOO | SOO | POO |
| COP | NOP | OOS | SOS | POS |
| | | OOP | SOP | POP |
| CSC | NSC | | | |
| CSN | NSN | OSC | SSC | PSC |
| CSO | NSO | OSN | SSN | PSN |
| CSS | NSS | OSO | SSO | PSO |
| CSP | NSP | OSS | SSS | PSS |
| | | OSP | SSP | PSP |
| CPC | NPC | | | |
| CPN | NPN | OPC | SPC | PPC |
| CPO | NPO | OPN | SPN | PPN |
| CPS | NPS | OPO | SPO | PPO |
| CPP | NPP | OPS | SPS | PPS |
| | | OPP | SPP | PPP |

One skilled in the art would be able to identify bonding patterns that would produce multivalent compounds. Methods for producing these bonding arrangements are described in March, "Advanced Organic Chemistry", 4th Edition, Wiley-Interscience, New York, New York (1992). These arrangements are described in the grid of dots shown in the scheme above. All of the possible arrangements for the five most preferred atoms are shown. Each atom has a variety of acceptable oxidation states. The bonding arrangements underlined are less acceptable and are not preferred.

Examples of molecular structures in which the above bonding patterns could be employed as components of the linker are shown below.

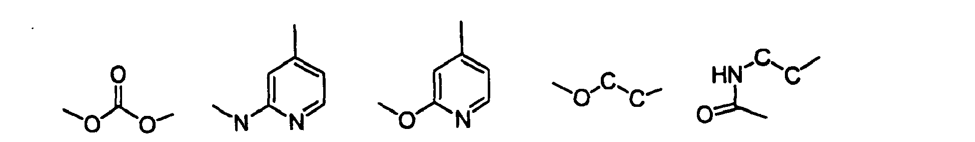

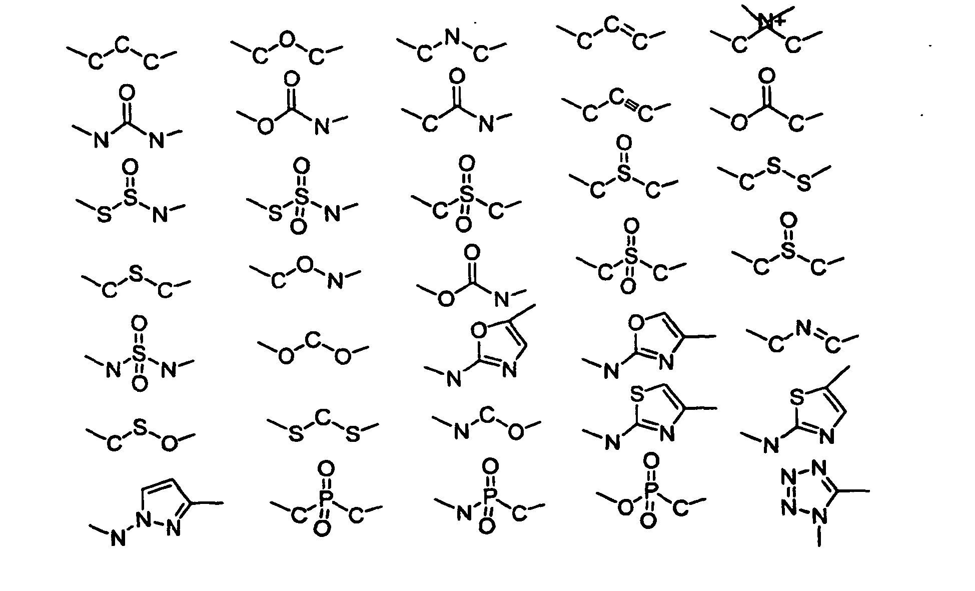

The identification of an appropriate framework geometry and size for ligand domain presentation are important steps in the construction of a multibinding compound with enhanced activity. Systematic spatial searching strategies can be used to aid in the identification of preferred frameworks through an iterative process. Figure 1 illustrates a useful strategy for determining an optimal framework display orientation for ligand domains. Various other strategies are known to those skilled in the art of molecular design and can be used for preparing compounds of this invention.

As shown in Figure 1, display vectors around similar central core structures such as a phenyl structure (Panel A) and a cyclohexane structure (Panel B) can be varied, as can the spacing of the ligand domain from the core structure (i.e., the length of the attaching moiety). It is to be noted that core structures other than those shown here can be used for determining the optimal framework display orientation of the ligands. The process may require the use of multiple copies of the same central core structure or combinations of different types of display cores.

Assays of each of the individual compounds of a collection generated as described above will lead to a subset of compounds with the desired enhanced activities (e.g., potency, selectivity, etc.). The analysis of this subset using a technique such as Ensemble Molecular Dynamics will provide a framework orientation that favors the properties desired. A wide diversity of linkers is commercially available (see, e.g., Available Chemical Directory (ACD)). Many of the linkers that are suitable for use in this invention fall into this category. Other can be readily synthesized by methods well known in the art and/or are described below.

Having selected a preferred framework geometry, the physical properties of the linker can be optimized by varying the chemical composition thereof. The composition of the linker can be varied in numerous ways to achieve the desired physical properties for the multibinding compound.

It can therefore be seen that there is a plethora of possibilities for the composition of a linker. Examples of linkers include aliphatic moieties, aromatic moieties, steroidal moieties, peptides, and the like. Specific examples are peptides or polyamides, hydrocarbons, aromatic groups, ethers, lipids, cationic or anionic groups, or a combination thereof.

Examples are given below, but it should be understood that various changes may be made and equivalents may be substituted without departing from the true spirit and scope of the invention. For example, properties of the linker can be modified by the addition or insertion of ancillary groups into or onto the linker, for example, to change the solubility of the multibinding compound (in water, fats, lipids, biological fluids, etc.), hydrophobicity, hydrophilicity, linker flexibility, antigenicity, stability, and the like. For example, the introduction of one or more poly(ethylene glycol) (PEG) groups onto or into the linker enhances the hydrophilicity and water solubility of the multibinding compound, increases both molecular weight and molecular size and, depending on the nature of the unPEGylated linker, may increase the *in vivo* retention time. Further PEG may decrease antigenicity and potentially enhances the overall rigidity of the linker.

Ancillary groups which enhance the water solubility/hydrophilicity of the linker and, accordingly, the resulting multibinding compounds are useful in practicing this invention. Thus, it is within the scope of the present invention to use ancillary groups such as, for example, small repeating units of ethylene glycols, alcohols, polyols (e.g., glycerin, glycerol propoxylate, saccharides, including mono-, oligosaccharides, etc.), carboxylates (e.g., small repeating units of glutamic acid, acrylic acid, etc.), amines (e.g., tetraethylenepentamine), and the like) to enhance the water solubility and/or hydrophilicity of the multibinding compounds of this invention. In preferred embodiments, the ancillary group used to improve water solubility/hydrophilicity will be a polyether.

The incorporation of lipophilic ancillary groups within the structure of the linker to enhance the lipophilicity and/or hydrophobicity of the multibinding compounds described herein is also within the scope of this invention. Lipophilic groups useful with the linkers of this invention include, by way of example only, aryl and heteroaryl groups which, as above, may be either unsubstituted or substituted with other groups, but are at least substituted with a group which allows their covalent attachment to the linker. Other lipophilic groups useful with the linkers of this invention include fatty acid derivatives which do not form bilayers in aqueous medium until higher concentrations are reached.

Also within the scope of this invention is the use of ancillary groups which result in the multibinding compound being incorporated or anchored into a vesicle or other membranous structure such as a liposome or a micelle. The term "lipid" refers to any fatty acid derivative that is capable of forming a bilayer or a micelle such that a hydrophobic portion of the lipid material orients toward the bilayer while a hydrophilic portion orients toward the aqueous phase. Hydrophilic characteristics derive from the presence of phosphato, carboxylic, sulfato, amino, sulfhydryl, nitro and other like groups well known in the art. Hydrophobicity could be conferred by the inclusion of groups that include, but are not limited to, long chain saturated and unsaturated aliphatic hydrocarbon groups of up to 20 carbon atoms and such groups substituted by one or more aryl, heteroaryl, cycloalkyl, and/or heterocyclic group(s). Preferred lipids are phosphglycerides and sphingolipids, representative examples of which include phosphatidylcholine, phosphatidylethanolamine, phosphatidylserine, phosphatidylinositol, phosphatidic acid, palmitoyleoyl phosphatidylcholine, lysophosphatidylcholine, lysophosphatidyl-ethanolamine, dipalmitoylphosphatidylcholine, dioleoylphosphatidylcholine, distearoyl-phosphatidylcholine or dilinoleoylphosphatidylcholine could be used. Other compounds lacking phosphorus, such as sphingolipid and glycosphingolipid families are also within the group designated as lipid. Additionally, the amphipathic lipids described above may be mixed with other lipids including triglycerides and sterols.

The flexibility of the linker can be manipulated by the inclusion of ancillary groups which are bulky and/or rigid. The presence of bulky or rigid groups can hinder free rotation about bonds in the linker or bonds between the linker and the ancillary group(s) or bonds between the linker and the functional groups. Rigid groups can include, for example, those groups whose conformational lability is restrained by the presence of rings and/or multiple bonds within the group, for example, aryl, heteroaryl, cycloalkyl, cycloalkenyl, and heterocyclic groups. Other groups which can impart rigidity include polypeptide groups such as oligo- or polyproline chains.

Rigidity can also be imparted electrostatically. Thus, if the ancillary groups are either positively or negatively charged, the similarly charged ancillary groups will force the presenter linker into a configuration affording the maximum distance between each of the like charges. The energetic cost of bringing the like-charged groups closer to each other will tend to hold the linker in a configuration that maintains the separation between the like-charged ancillary groups. Further ancillary groups bearing opposite charges will tend to be attracted to their oppositely charged counterparts and potentially may enter into both inter- and intramolecular ionic bonds. This non-covalent mechanism will tend to hold the linker into a conformation which allows bonding between the oppositely charged groups. The addition of ancillary groups which are charged, or alternatively, bear a latent charge when deprotected, following addition to the linker, include deprotectation of a carboxyl, hydroxyl, thiol or amino group by a change in pH, oxidation, reduction or other mechanisms known to those skilled in the art which result in removal of the protecting group, is within the scope of this invention.

Rigidity may also be imparted by internal hydrogen bonding or by hydrophobic collapse.

Bulky groups can include, for example, large atoms, ions (e.g., iodine, sulfur, metal ions, etc.) or groups containing large atoms, polycyclic groups, including aromatic groups, non-aromatic groups and structures incorporating one or more carbon-carbon multiple bonds (i.e., alkenes and alkynes). Bulky groups can also include oligomers and polymers which are branched- or straight-chain species. Species that are branched are expected to increase the rigidity of the structure more per unit molecular weight gain than are straight-chain species.

In preferred embodiments, rigidity is imparted by the presence of cyclic groups (e.g., aryl, heteroaryl, cycloalkyl, heterocyclic, etc.). In other preferred embodiments, the linker comprises one or more six-membered rings. In still further preferred embodiments, the ring is an aryl group such as, for example, phenyl or naphthyl.

In view of the above, it is apparent that the appropriate selection of a linker group providing suitable orientation, restricted/unrestricted rotation, the desired degree of hydrophobicity/hydrophilicity, etc. is well within the skill of the art. Eliminating or reducing antigenicity of the multibinding compounds described herein is also within the scope of this invention. In certain cases, the antigenicity of a multibinding compound may be eliminated or reduced by use of groups such as, for example, poly(ethylene glycol).

As explained above, the multibinding compounds described herein comprise 2 ligands attached to a linker that attaches the ligands in such a manner that they are presented to the enzyme for multivalent interactions with ligand binding sites thereon/therein. The linker spatially constrains these interactions to occur within dimensions defined by the linker. This and other factors increases the biological activity of the multibinding compound as compared to the same number of ligands made available in monobinding form.

As noted previously, the linker may be considered as a framework to which ligands are attached. Thus, it should be recognized that the ligands can be attached at any suitable position on this framework, for example, at the termini of a linear chain or at any intermediate position.

Suitable divalent linkers include, by way of example only, those derived from dicarboxylic acids, disulfonylhalides, dialdehydes, diketones, dihalides, diisocyanates,diamines, diols, mixtures of carboxylic acids, sulfonylhalides, aldehydes, ketones, halides, isocyanates, amines and diols. In each case, the carboxylic acid, sulfonylhalide, aldehyde, ketone, halide, isocyanate, amine and diol functional group is reacted with a complementary functionality on the ligand to form a covalent linkage. Such complementary functionality is well known in the art as illustrated in the following table:

### COMPLEMENTARY BINDING CHEMISTRIES

| First Reactive Group | Second Reactive Group | Linkage |
|---|---|---|
| carboxyl | amine | amide |
| sulfonyl halide | amine | sulfonamide |
| hydroxyl | alkyl/aryl halide | ether |
| hydroxyl | isocyanate | urethane |
| amine | epoxide | β-hydroxyamine |
| amine | alkyl/aryl halide | alkylamine |
| hydroxyl | carboxyl | ester |
| amine | aldehyde/NaCNBH₃ | amine |
| hydroxylamine | sulfonyl halide | sulfonamide |
| aldehyde | amine/NaCHBH₃ | amine |
| aldehyde | amine/NaCHBH₃ | amine |
| amine | isocynate | urea |

Exemplary linkers include the following linkers identified as X-1 through X-418 as set forth below:

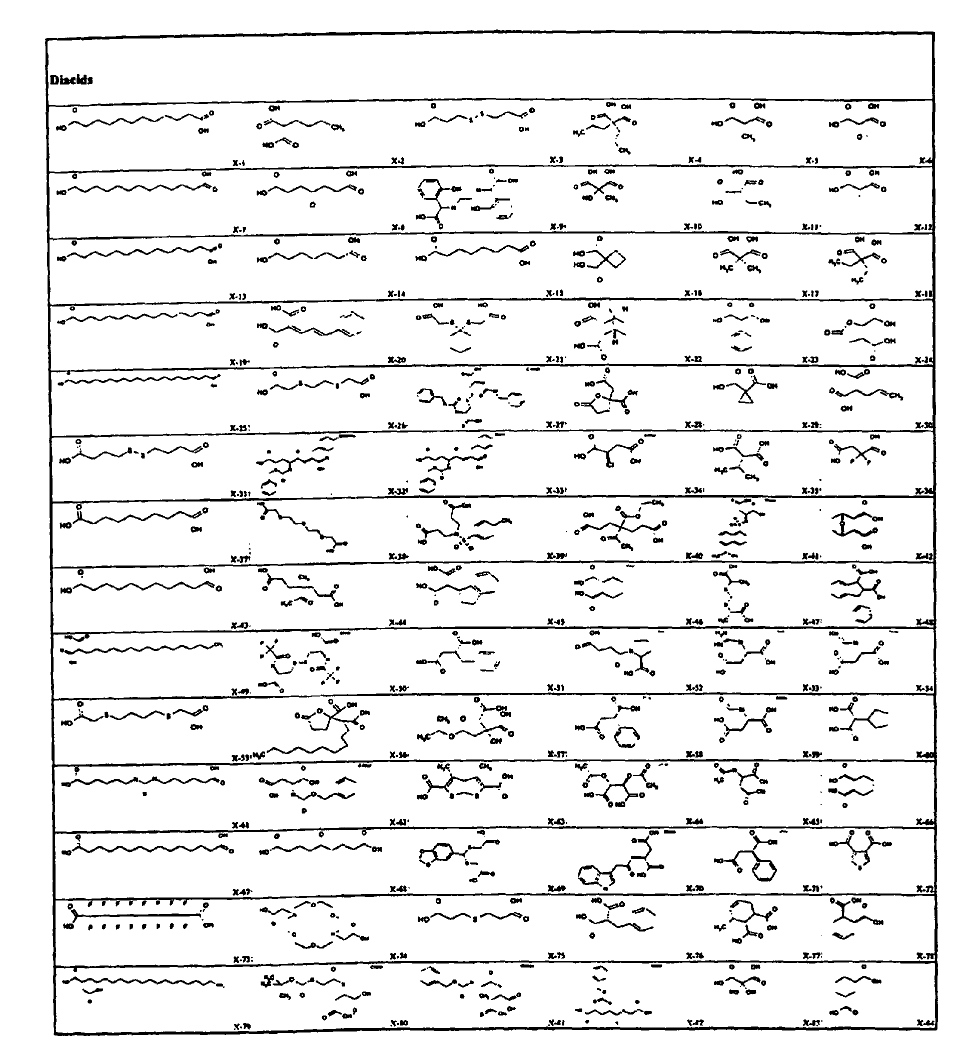

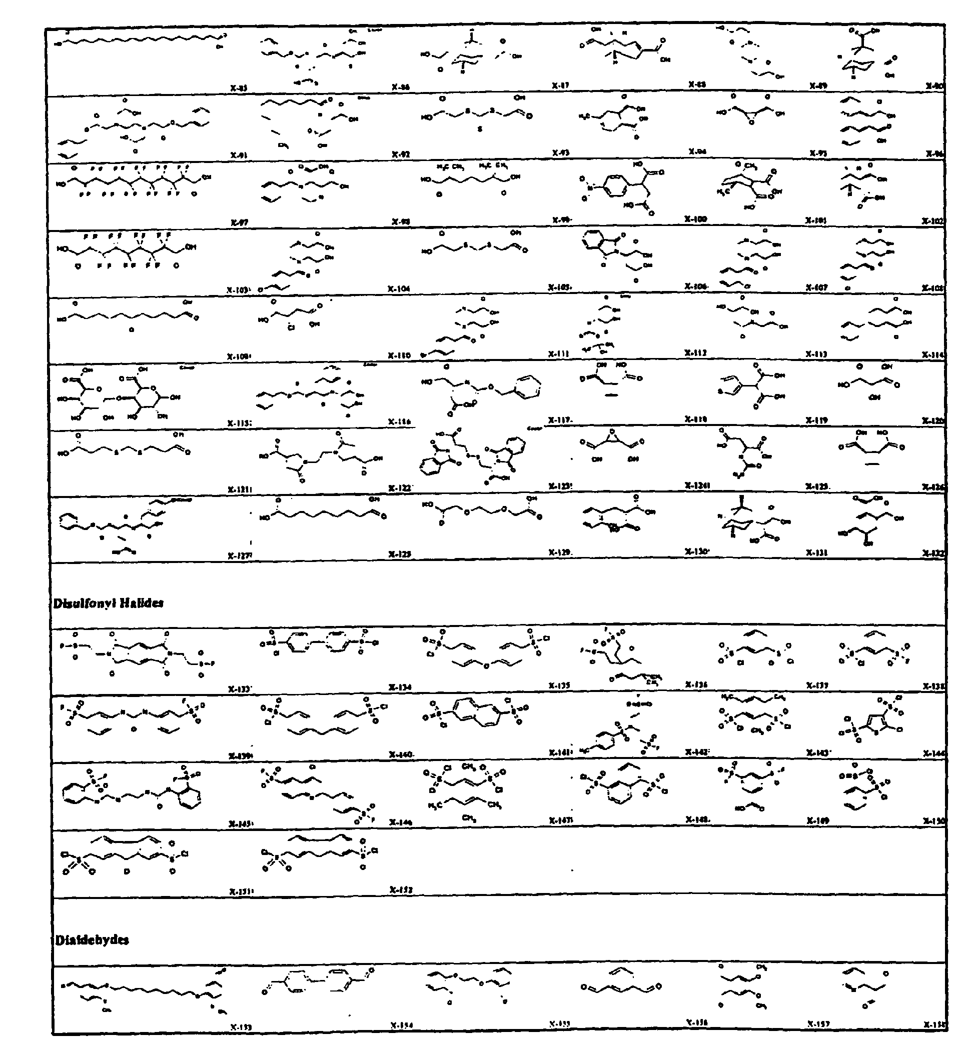

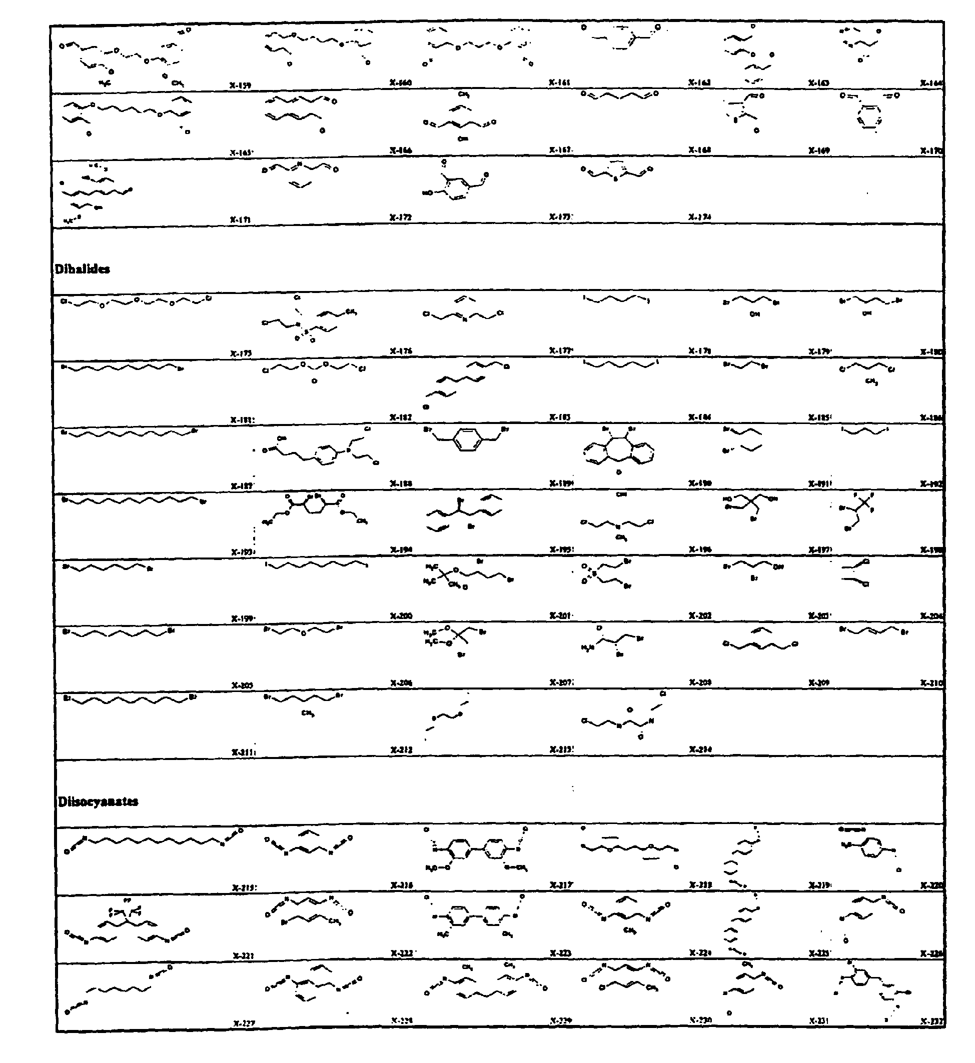

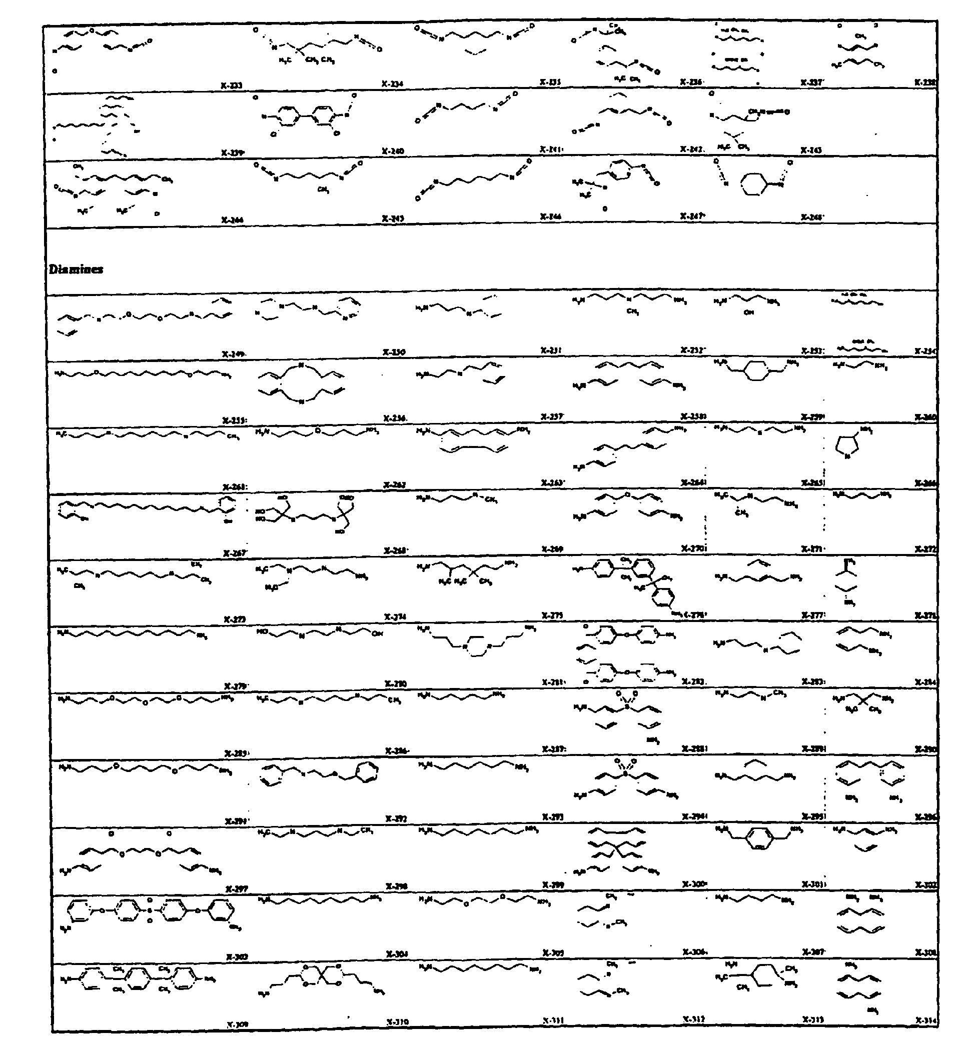

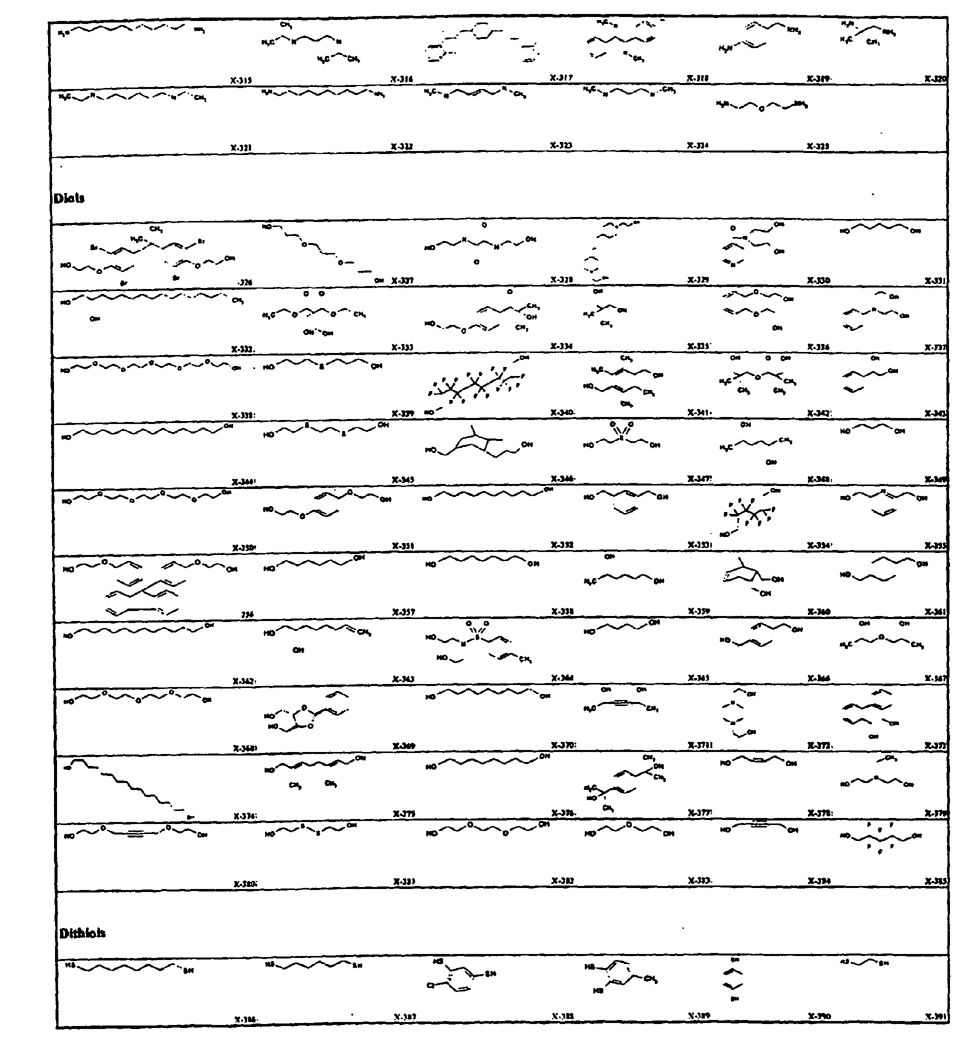

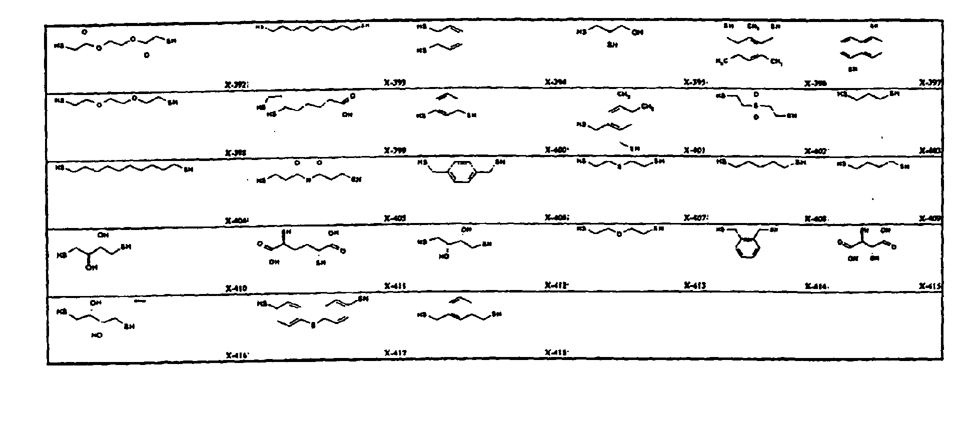

For a bivalent multibinding compounds of the Invention, beta lactam antibiotic ligands represented as L₁ for use in this invention include, by way of example, L₁-1 through L₁-5, the ligands L₁-1 through L₁-5 having been selected from the compounds of formula (a)-(e) disclosed in the Summary of the invention: compound (a) (L₁-1), compound (b) (L₁-2), compound (c) (L₁-3), compound (d) (L₁-4) and compound (e) (L₁-5).

The glycopeptide ligands represented as L₂ for use in this invention include, by way of example, L₂-1 through L₂-2: L₂-1 being an optionally substituted vancomycin and L₂-2 being an aglycone derivative of an optionally substituted vancomycin.

Combinations of ligands (L₁ and L₂) and linkers (X) per this invention include, by way example only, heterodimers wherein a first ligand, L₁, selected from L₁-1 through L₁-5 above, and a second ligand, L₂, and a linker, X, are selected from the following:

| | | | | | |
|---|---|---|---|---|---|
| L₂-1/X-1- | L₂-1/X-2- | L₂-1/X-3- | L₂-1/X-4- | L₂-1/X-5- | L₂-1/X-6- |
| L₂-1/X-7- | L₂-1/X-8- | L₂-1/X-9- | L₂-1/X-10- | L₂-1/X-11- | L₂-1/X-12- |
| L₂-1/X-13- | L₂-1/X-14- | L₂-1/X-15- | L₂-1/X-16- | L₂-1/X-17- | L₂-1/X-18- |
| L₂-1/X-19- | L₂-1/X-20- | L₂-1/X-21- | L₂-1/X-22- | L₂-1/X-23- | L₂-1/X-24- |
| L₂-1/X-25- | L₂-1/X-26- | L₂-1/X-27- | L₂-1/X-28- | L₂-1/X-29- | L₂-1/X-30- |
| L₂-1/X-31- | L₂-1/X-32- | L₂-1/X-33- | L₂-1/X-34- | L₂-1/X-35- | L₂-1/X-36- |
| L₂-1/X-37- | L₂-1/X-38- | L₂-1/X-39- | L₂-1/X-40- | L₂-1/X-41- | L₂-1/X-42- |
| L₂-1/X-43- | L₂-1/X-44- | L₂-1/X-45- | L₂-1/X-46- | L₂-1/X-47- | L₂-1/X-48- |
| L₂-1/X-49- | L₂-1/X-50- | L₂-1/X-51- | L₂-1/X-52- | L₂-1/X-53- | L₂-1/X-54- |
| L₂-1/X-55- | L₂-1/X-56- | L₂-1/X-57- | L₂-1/X-58- | L₂-1/X-59- | L₂-1/X-60- |
| L₂-1/X-61- | L₂-1/X-62- | L₂-1/X-63- | L₂-1/X-64- | L₂-1/X-65- | L₂-1/X-66- |
| L₂-1/X-67- | L₂-1/X-68- | L₂-1/X-69- | L₂-1/X-70- | L₂-1/X-71- | L₂-1/X-72- |
| L₂-1/X-73- | L₂-1/X-74- | L₂-1/X-75- | L₂-1/X-76- | L₂-1/X-77- | L₂-1/X-78- |
| L₂-1/X-79- | L₂-1/X-80- | L₂-1/X-81- | L₂-1/X-82- | L₂-1/X-83- | L₂-1/X-84- |
| L₂-1/X-85- | L₂-1/X-86- | L₂-1/X-87- | L₂-1/X-88- | L₂-1/X-89- | L₂-1/X-90- |
| L₂-1/X-91- | L₂-1/X-92- | L₂-1/X-93- | L₂-1/X-94- | L₂-1/X-95- | L₂-1/X-96- |
| L₂-1/X-97- | L₂-1/X-98- | L₂-1/X-99- | L₂-1/X-100- | L₂-1/X-101- | L₂-1/X-102- |
| L₂-1/X-103- | L₂-1/X-104- | L₂-1/X-105- | L₂-1/X-106- | L₂-1/X-107- | L₂-1/X-108- |
| L₂-1/X-109- | L₂-1/X-110- | L₂-1/X-111- | L₂-1/X-112- | L₂-1/X-113- | L₂-1/X-114- |
| L₂-1/X-115- | L₂-1/X-116- | L₂-1/X-117- | L₂-1/X-118- | L₂-1/X-119- | L₂-1/X-120- |
| L₂-1/X-121- | L₂-1/X-122- | L₂-1/X-123- | L₂-1/X-124- | L₂-1/X-125- | L₂-1/X-126- |
| L₂-1/X-127- | L₂-1/X-128- | L₂-1/X-129- | L₂-1/X-130- | L₂-1/X-131- | L₂-1/X-132- |
| L₂-1/X-133- | L₂-1/X-134- | L₂-1/X-135- | L₂-1/X-136- | L₂-1/X-137- | L₂-1/X-138- |
| L₂-1/X-139- | L₂-1/X-140- | L₂-1/X-141- | L₂-1/X-142- | L₂-1/X-143- | L₂-1/X-144- |
| L₂-1/X-145- | L₂-1/X-146- | L₂-1/X-147- | L₂-1/X-148- | L₂-1/X-149- | L₂-1/X-150- |
| L₂-1/X-151- | L₂-1/X-152- | L₂-1/X-153- | L₂-1/X-154- | L₂-1/X-155- | L₂-1/X-156- |
| L₂-1/X-157- | L₂-1/X-158- | L₂-1/X-159- | L₂-1/X-160- | L₂-1/X-161- | L₂-1/X-162- |
| L₂-1/X-163- | L₂-1/X-164- | L₂-1/X-165- | L₂-1/X-166- | L₂-1/X-167- | L₂-1/X-168- |
| L₂-1/X-169- | L₂-1/X-170- | L₂-1/X-171- | L₂-1/X-172- | | |
| L₂-1/X-173- | L₂-1/X-174- | L₂-1/X-175- | L₂-1/X-176- | L₂-1/X-177- | L₂-1/X-178- |
| L₂-1/X-179- | L₂-1/X-180- | L₂-1/X-181- | L₂-1/X-182- | L₂-1/X-183- | L₂-1/X-184- |
| L₂-1/X-185- | L₂-1/X-186- | L₂-1/X-187- | L₂-1/X-188- | L₂-1/X-189- | L₂-1/X-190- |
| L₂-1/X-191- | L₂-1/X-192- | L₂-1/X-193- | L₂-1/X-194- | L₂-1/X-195- | L₂-1/X-196- |
| L₂-1/X-197- | L₂-1/X-198- | L₂-1/X-199- | L₂-1/X-200- | L₂-1/X-201- | L₂-1/X-202- |
| L₂-1/X-203- | L₂-1/X-204- | L₂-1/X-205- | L₂-1/X-206- | L₂-1/X-207- | L₂-1/X-208- |
| L₂-1/X-209- | L₂-1/X-210- | L₂-1/X-211- | L₂-1/X-212- | L₂-1/X-213- | L₂-1/X-214- |
| L₂-1/X-215- | L₂-1/X-216- | L₂-1/X-217- | L₂-1/X-218- | L₂-1/X-219- | L₂-1/X-220- |
| L₂-1/X-221- | L₂-1/X-222- | L₂-1/X-223- | L₂-1/X-224- | L₂-1/X-225- | L₂-1/X-226- |
| L₂-1/X-227- | L₂-1/X-228- | L₂-1/X-229- | L₂-1/X-230- | L₂-1/X-231- | L₂-1/X-232- |
| L₂-1/X-233- | L₂-1/X-234- | L₂-1/X-235- | L₂-1/X-236- | L₂-1/X-237- | L₂-1/X-238- |
| L₂-1/X-239- | L₂-1/X-240- | L₂-1/X-241- | L₂-1/X-242- | L₂-1/X-243- | L₂-1/X-244- |
| L₂-1/X-245- | L₂-1/X-246- | L₂-1/X-247- | L₂-1/X-248- | L₂-1/X-249- | L₂-1/X-250- |
| L₂-1/X-251- | L₂-1/X-252- | L₂-1/X-253- | L₂-1/X-254- | L₂-1/X-255- | L₂-1/X-256- |
| L₂-1/X-257- | L₂-1/X-258- | L₂-1/X-259- | L₂-1/X-260- | L₂-1/X-261- | L₂-1/X-262- |
| L₂-1/X-263- | L₂-1/X-264- | L₂-1/X-265- | L₂-1/X-266- | L₂-1/X-267- | L₂-1/X-268- |
| L₂-1/X-269- | L₂-1/X-270- | L₂-1/X-271- | L₂-1/X-272- | L₂-1/X-273- | L₂-1/X-274- |
| L₂-1/X-275- | L₂-1/X-276- | L₂-1/X-277- | L₂-1/X-278- | L₂-1/X-279- | L₂-1/X-280- |
| L₂-1/X-281- | L₂-1/X-282- | L₂-1/X-283- | L₂-1/X-284- | L₂-1/X-285- | L₂-1/X-286- |
| L₂-1/X-287- | L₂-1/X-288- | L₂-1/X-289- | L₂-1/X-290- | L₂-1/X-291- | L₂-1/X-292- |
| L₂-1/X-293- | L₂-1/X-294- | L₂-1/X-295- | L₂-1/X-296- | L₂-1/X-297- | L₂-1/X-298- |
| L₂-1/X-299- | L₂-1/X-300- | L₂-1/X-301- | L₂-1/X-302- | L₂-1/X-303- | L₂-1/X-304- |
| L₂-1/X-305- | L₂-1/X-306- | L₂-1/X-307- | L₂-1/X-308- | L₂-1/X-309- | L₂-1/X-310- |
| L₂-1/X-311- | L₂-1/X-312- | L₂-1/X-313- | L₂-1/X-314- | L₂-1/X-315- | L₂-1/X-316- |
| L₂-1/X-317- | L₂-1/X-318- | L₂-1/X-319- | L₂-1/X-320- | L₂-1/X-321- | L₂-1/X-322- |
| L₂-1/X-323- | L₂-1/X-324- | L₂-1/X-325- | L₂-1/X-326- | L₂-1/X-327- | L₂-1/X-328- |
| L₂-1/X-329- | L₂-1/X-330- | L₂-1/X-331- | L₂-1/X-332- | L₂-1/X-333- | L₂-1/X-334- |
| L₂-1/X-335- | L₂-1/X-336- | L₂-1/X-337- | L₂-1/X-338- | L₂-1/X-339- | L₂-1/X-340- |
| L₂-1/X-341- | L₂-1/X-342- | L₂-1/X-343- | L₂-1/X-344- | L₂-1/X-345- | L₂-1/X-346- |
| L₂-1/X-347- | L₂-1/X-348- | L₂-1/X-349- | L₂-1/X-350- | L₂-1/X-351- | L₂-1/X-352- |
| L₂-1/X-353- | L₂-1/X-354- | L₂-1/X-355- | L₂-1/X-356- | L₂-1/X-357- | L₂-1/X-358- |
| L₂-1/X-359- | L₂-1/X-360- | L₂-1/X-361- | L₂-1/X-362- | L₂-1/X-363- | L₂-1/X-364- |
| L₂-1/X-365- | L₂-1/X-366- | L₂-1/X-367- | L₂-1/X-368- | L₂-1/X-369- | L₂-1/X-370- |
| L₂-1/X-371- | L₂-1/X-372- | L₂-1/X-373- | L₂-1/X-374- | L₂-1/X-375- | L₂-1/X-376- |
| L₂-1/X-377- | L₂-1/X-378- | L₂-1/X-379- | L₂-1/X-380- | L₂-1/X-381- | L₂-1/X-382- |
| L₂-1/X-383- | L₂-1/X-384- | L₂-1/X-385- | L₂-1/X-386- | L₂-1/X-387- | L₂-1/X-388- |
| L₂-1/X-389- | L₂-1/X-390- | L₂-1/X-391- | L₂-1/X-392- | L₂-1/X-393- | L₂-1/X-394- |
| L₂-1/X-395- | L₂-1/X-396- | L₂-1/X-397- | L₂-1/X-398- | L₂-1/X-399- | L₂-1/X-400- |
| L₂-1/X-401- | L₂-1/X-402- | L₂-1/X-403- | L₂-1/X-404- | L₂-1/X-405- | L₂-1/X-406- |
| L₂-1/X-407- | L₂-1/X-408- | L₂-1/X-409- | L₂-1/X-410- | L₂-1/X-411- | L₂-1/X-412- |
| L₂-1/X-413- | L₂-1/X-414- | L₂-1/X-415- | L₂-1/X-416- | L₂-1/X-417- | L₂-1/X-418- |

and so on, substituting L₂-2.

### Utility, Testing, and Administration

### Utility

The compounds of the invention, and their pharmaceutically acceptable salts, are useful in medical treatments and exhibit biological activity, including antibacterial activity, which can be demonstrated in the tests described in the Examples. The antibacterial activity of the instant compounds may be determined by testing in standardized *in vitro* dilution tests for minimum inhibitory concentration (MICs). Such tests are well known to those skilled in the art, and are referenced and described in the fourth edition of "Antibiotics in Laboratory Medicine", by Victor Lorian, M.D., published by Williams and Wilkins. Using such standard microbiological procedures, the compounds of this invention will be found to exhibit activity against gram-positive and gram-negative bacteria such as *Staphylococcus aureus, Escherichia coli* and *Pseudomonas aeruginosa* at test levels.

The compounds of the present invention are useful in the treatment in mammals of bacterial infections, by both gram-positive and gram-negative bacteria. The compounds may be administered to the mammals in the form of a pharmaceutical composition comprising the compounds of the invention admixed with a pharmaceutically acceptable excipient.

### Pharmaceutical Formulations

When employed as pharmaceuticals, the compounds of this invention are usually administered in the form of pharmaceutical compositions. These compounds can be administered by a variety of routes including oral, rectal, transdermal, subcutaneous, intravenous, intramuscular, and intranasal. These compounds are effective as injectable intranasal and oral compositions. Such compositions are prepared in a manner well known in the pharmaceutical art and comprise at least one active compound.

This invention also includes pharmaceutical compositions which contain, as the active ingredient, one or more of the compounds described herein associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 0.001 to about 1 g, more usually about 1 to about 30 mg, of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Preferably, the compound of Formula (I) above is employed at no more than about 20 weight percent of the pharmaceutical composition, more preferably no more than about 15 weight percent, with the balance being pharmaceutically inert carrier(s).

The active compound is effective over a wide dosage range and is generally administered in a pharmaceutically effective amount. It, will be understood, however, that the amount of the compound actually administered will be determined by a physician, in the light of the relevant circumstances, including the condition to be treated, the chosen route of administration, the actual compound administered and its relative activity, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

### EXAMPLES

The following preparations and examples are given to enable those skilled in the art to more clearly understand and to practice the present invention. They should not be considered as limiting the scope of the invention, but merely as being illustrative and representative thereof.

In the examples below, the following abbreviations have the following meanings. Unless otherwise stated, all temperatures are in degrees Celsius. If an abbreviation is not defined, it has its generally accepted meaning.

| | |
|---|---|
| Å = | Angstroms |
| cm = | centimeter |
| DCC = | dicyclohexyl carbodiimide |
| DMF = | *N,N*-dimethylformamide |
| DMSO = | dimethylsulfoxide |
| g = | gram |
| HPLC = | high performance liquid chromatography |
| mg = | milligram |
| min = | minute |
| mL = | milliliter |
| mm = | millimeter |
| mmol = | millimol |
| N = | normal |
| THF = | tetrahydrofuran |
| µL = | microliters |
| µm = | microns |

### Synthetic Examples

### Example 1

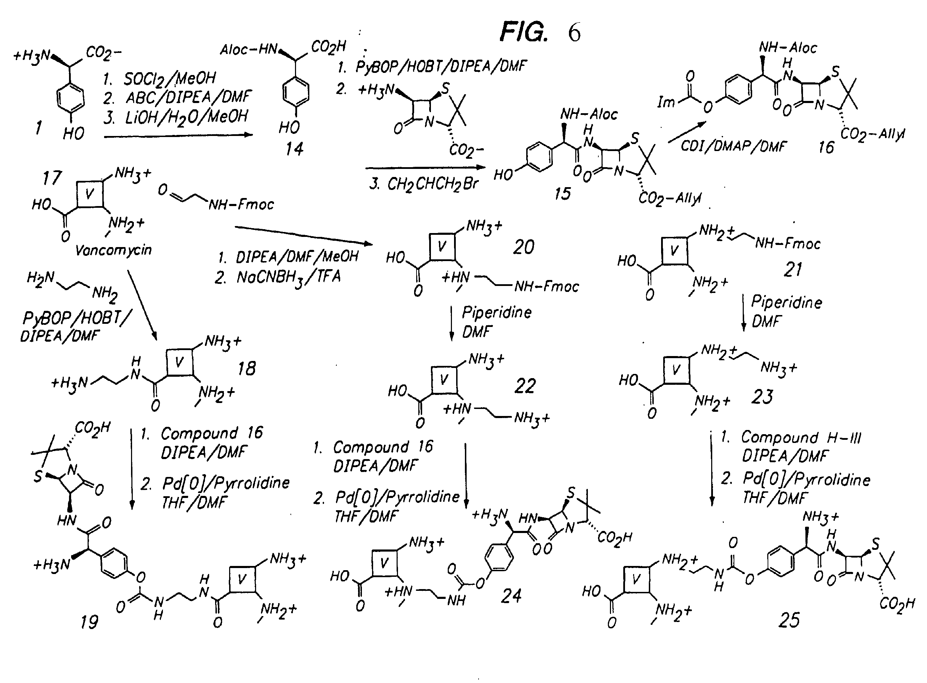

### Synthesis of Vancomycin-Amoxicillin Heterodimer (Following Figure 6)

### Method A

### Step 1

A slurry of (D)-4-hydroxyphenyl glycine **1** (10 mmol) in methanol (100 mL) is stirred with cooling in an ice bath. Thionyl chloride (11 mmol) is added dropwise over the course of 15 minutes. After addition is complete, the mixture is allowed to stir in the cooling bath for an additional 2 hours. The mixture is then concentrated to dryness to afford crude (D)-4-hydroxyphenyl glycine methyl ester hydrochloride. This material is dissolved and stirred in 100 mL dimethylformamide and treated sequentially with diisopropylethyl amine (22 mmol) followed by allyl 1-benzotriazolyl carbonate (11 mmol). After stirring 1 hour at room temperature, volatiles are removed under reduced pressure and the residue is fractionated by silica gel chromatography using ethyl acetate/hexane eluent to afford Aloc-protected (D)-4-hydroxyphenyl glycine methyl ester. The ester (7.0 mmol) is dissolved in methanol (40 mL, stirred at room temperature, and treated with a solution of lithium hydroxide (8.0 mmol) in 20 mL water. The reaction is stirred at room temperature for 2 hours and then poured into 100 mL 1 N sodium hydrogen sulfate solution and extracted with ethyl acetate. The organic extract is dried over anhydrous sodium sulfate, filtered, and then concentrated under reduced pressure. The crude is fractionated via chromatography on silica gel using methanol/methylene chloride/trifluoracetic acid eluent to afford *N*-Aloc (D)-4-hydroxyphenyl glycine 14.

### Step 2

Compound 14 (5.0 mmol) is dissolved in anhydrous dimethylformamide (20 mL), stirred at room temperature, and treated sequentially with hydroxybenzotriazole (5.0 mmol), diisopropylethyl amine (5.0 mmol) and PyBOP (5.0 mmol). After stirring for 15 minutes at room temperature, the activated acid is treated with (+)-6-aminopenicillanic acid (5.0 mmol) and the coupling reaction is stirred overnight at room temperature. The mixture is then treated with allyl bromide (5.0 mmol) and stirred for an additional 24 hours. Volatiles are removed under vacuum and the crude is fractionated by silica gel chromatography using methanol/methylene chloride eluent to afford *N*-Aloc (D)-4-hydroxyphenyl glycine allyl ester **16.**

### Step 3

Compound **16** (1.0 mmol) is dissolved in anhydrous dimethylformamide (5.0 mL), stirred in an ice/water bath, and treated sequentially with *N,N*-dimethylaminopyridine (0.1 mmol) and carbonyldiimidazole (1.0 mmol). The ice bath is removed and the reaction mixture is allowed to warm to room temperature. The imidazolide 16 thus produced is used without further manipulation in the coupling reactions described below.

### Step 4

Vancomycin-2-aminoethanamide **18** (compound **18** prepared as described in Example 5 below, 1.0 mmol) is dissolved in 5.0 mL anhydrous dimethylformamide, stirred at room temperature, and treated sequentially with diisopropylethyl amine (4.0 mmol) and the solution of the imidazolide **16** (prepared in Step 3 above). After 2 hours, volatiles are removed under vacuum and the residue is triturated with acetonitrile. The solid is then redissolved in 10 mL 1:1 anhydrous tetrahydrofuran:anhydrous dimethylformamide, stirred under nitrogen at room temperature, and treated sequentially with pyrrolidine (3.0 mmol) and tetrakis(triphenylphosphine)palladium[0] (0.25 mmol). After 2 hours, the mixture is concentrated under vacuum and the residue is dissolved in 0.1% aqueous trifluoroacetic acid and fractionated by reverse-phase HPLC using a linear gradient of acetonitrile in water (both buffered with 0.1% trifluoracetic acid) to afford the desired compound **19** upon lyopholization of the appropriate fractions.

### Method B

### Step 1

Vancomycin hydrochloride **17** (10 mmol) is slurried in 100 mL 1:1 methanol:anhydrous dimethylformamide, stirred at room temperature, and treated sequentially with diisopropylethyl amine (20 mmol) and Fmoc glycinal (prepared as described by Salvi et al. *Tetrahedron Lett.* **1994,** *35*, 1181-1184). After 2 hours the reaction mixture is cooled in an ice water bath and treated further with sodium cyanoborohydride (4.0 mmol) and trifluoroacetic acid (30 mmol). After **2** additional hours the crude product is precipitated by dropwise addition to a ten-fold volume of acetonitrile, and then fractionated by reverse-phase HPLC using a linear gradient of acetonitrile in water (both buffered with 0.1% trifluoracetic acid) to afford the adducts reductively alkylated on the *N*-methylamino terminus **20** and on the *N*'-amino group of the vancosamine residue **21.**

### Step 2

Compounds **20** and **21** (2.0 mmol each) are separately dissolved in anhydrous dimethylformamide (10 mL), stirred at room temperature and treated with excess piperidine (1.0 mL). After one hour the crude products are precipitated by dropwise addition to 50 mL acetonitrile with vigorous stirring. The crude products are fractionated by reverse-phase HPLC using a linear gradient of acetonitrile in water (both buffered with 0.1% trifluoracetic acid) to afford the *N*-aminoethyl adduct **22** and the the *N*'-aminoethyl adduct **23** upon lyopholization of the appropriate fractions.

Compounds **22** and **23** are subsequently elaborated to desired heterobivalent compounds **24** and **25**, respectively by following step **4** described for the conversion of compound **18** to compound **19**.

### Example 2

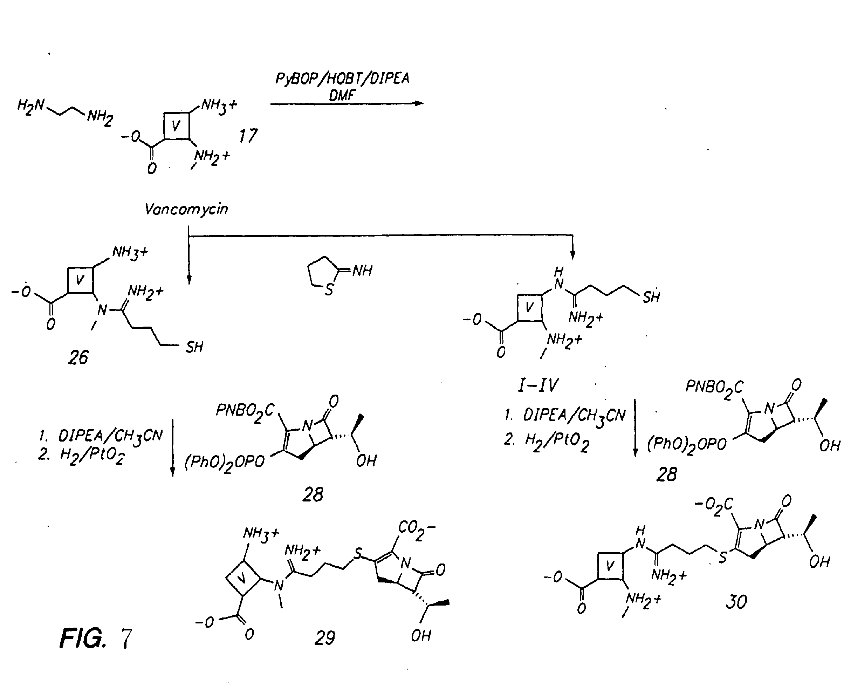

### Synthesis of Vancomycin-Imipenem Heterodimer (Following Figure 7)

### Step 1

Vancomycin hydrochloride **17** (10 mmol) is) is dissolved in water (100 mL), stirred at room temperature, and treated sequentially with triethylamine (40 mmol) and 2-iminothiolane hydrochloride (10 mmol). After two hours the reaction mixture is fractionated by reverse-phase HPLC using a linear gradient of acetonitrile in water (both buffered with 0.1% trifluoracetic acid) to afford the imine adducts modified on the *N*-methylamino terminus compound **26** and on the *N'*-amino group of the vancosamine residue compound **27.**

### Step 2

Compound **28** (2.0 mmol) is generated in acetonitrile (10 mL) as described (Salzmann et al. *J. Am. Chem. Soc.* **1980,** *102*, 6163 and Lelillo et al. *Tetrahedron Lett.* **1980,** *21*, 2783). This is then treated with a solution ofcompound **26** (2.0 mmol) and diisopropylethyl amine (11 mmol) in 10 mL anhydrous dimethylformamide and the reaction is stirred at 0 C for 1 hour. After removal of volatiles under vacuum, the crude product is dissolved in a mixture of tetrahydrofuran and water buffered to pH 7.0 with morpholinopropane sulfonic acid, treated with 10% platinum oxide (20 mg) and subjected to 40 psi H₂ for 4 hours. The mixture is filtered through a pad of celite to remove catalyst and chromatographed at 4 °C on a column of Dowex 50 X 4 (Na+ cycle, 200-400 mesh) resin eluted with deionized water. The desired compound 29 is recovered upon lyopholization of the appropriate fractions.

In a like manner, using adduct **27** in place of **26**, compound **30** is prepared.

### Example 3

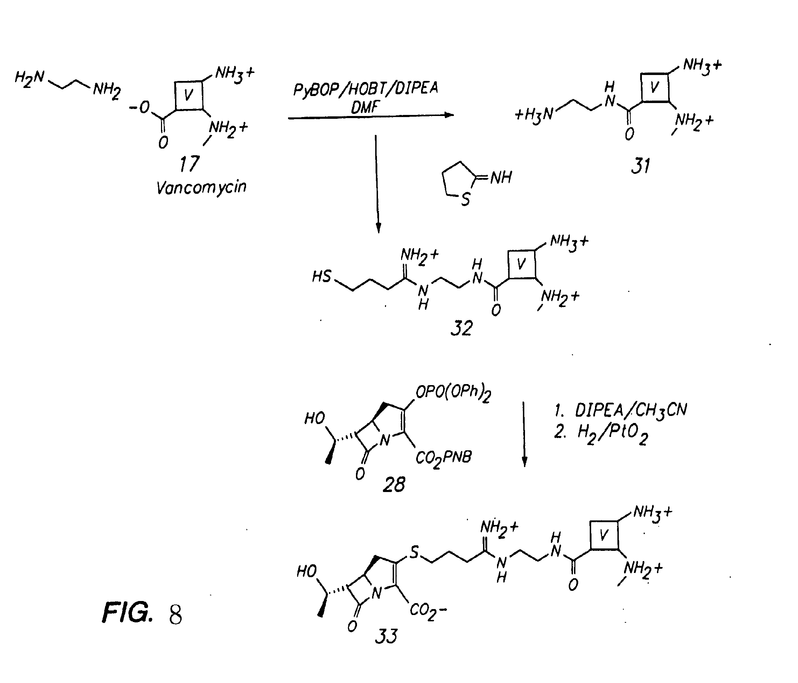

### Synthesis of Vancomycin-Imipenem Heterodimer (Following Figure 8)

### Step 1

Vancomycin hydrochloride **17** (10 mmol) is dissolved in 100 mL 1:1 anhydrous dimethylsulfoxide:dimethylformamide, stirred at room temperature, and treated sequentially with ethylenediamine (20 mmol), hydroxybenzotriazole (10 mmol), and PyBOP (10 mmol). After two hours, the crude product is precipitated by dropwise addition to 1 L vigorously stirred acetonitrile, and then fractionated by reverse-phase HPLC using a linear gradient of acetonitrile in water (both buffered with 0.1% trifluoracetic acid) to afford compound **31** after lyopholization of the appropriate fractions.

### Step 2

Compound **31** (5.0 mmol) is) is dissolved in water (50 mL), stirred at room temperature, and treated sequentially with triethylamine (20 mmol) and 2-iminothiolane hydrochloride (5.0 mmol). After two hours the reaction mixture is fractionated by reverse-phase HPLC using a linear gradient of acetonitrile in water (both buffered with 0.1% trifluoracetic acid) to afford compound **32** after lyopholization of the appropriate fractions.

### Step 3

Compound **28** (2.0 mmol) is generated in acetonitrile (10 mL) as described (Salzmann et al. *J. Am. Chem. Soc.* **1980**, *102*, 6163 and Lelillo et al. *Tetrahedron Lett*. **1980,** *21*, 2783). This is then treated with a solution of compound **32** (2.0 mmol) and diisopropylethyl amine (11 mmol) in 10 mL anhydrous dimethylformamide and the reaction is stirred at 0 °C for I hour. After removal of volatiles under vacuum, the crude product is dissolved in a mixture of tetrahydrofuran and water buffered to pH 7.0 with morpholinopropane sulfonic acid, treated with 10% platinum oxide (20 mg) and subjected to 40 psi H₂ for 4 hours. The mixture is filtered through a pad of celite to remove catalyst and chromatographed at 4 °C on a column of Dowex 50 X 4 (Na+ cycle, 200-400 mesh) resin eluted with deionized water. The desired compound **33** is recovered upon lyopholization of the appropriate fractions.

### Formulation Examples

### Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

### Example 2

A tablet Formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

### Example 3

A dry powder inhaler formulation is prepared containing the following components:

| Ingredient | Weight % |
|---|---|
| Active Ingredient | 5 |
| Lactose | 95 |

The active ingredient is mixed with the lactose and the mixture is added to a dry powder inhaling appliance.

### Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

### Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, starch, and magnesium stearate are blended, passed through a No. 20 mesh U.S. sieve, and filled into hard gelatin capsules in 150 mg quantities.

### Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Example 7

Suspensions, each containing 50 mg of medicament per 5.0 mL dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Example 8

A formulation may be prepared as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, starch, and magnesium stearate are blended, passed through a No. 20 mesh U.S. sieve, and filled into hard gelatin capsules in 425.0 mg quantities.

### Example 9

A formulation may be prepared as follows:

| Ingredient | Quantity |
|---|---|
| Active Ingredient | 5.0 mg |
| Corn Oil | 1.0 mL |

### Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. *See, e.g.,* U.S. Patent 5,023,252, issued June 11, 1991, herein incorporated by reference in its entirety. Such patches may be constructed for continuous, pulsatile, or on demand delivery of pharmaceutical agents.

Other suitable formulations for use in the present invention can be found in *Remington's Pharmaceutical Sciences,* edited by E. W. Martin (Mack Publishing Company, 18th ed., 1990).

### Biological Examples

### Example 1

### Determination of Antibacterial Activity

### In Vitro Determination of Antibacterial Activity

β-lactam resistant bacteria are obtained and phenotyped based on their sensitivity. Minimal inhibitory concentrations (MICs) are measured in a microdilution broth procedure under NCCLS guidelines. The compounds are serially diluted into Mueller-Hinton broth in 96-well microtiter plates. Overnight cultures of bacterial strains are diluted based on absorbance at 600 nm so that the final concentration in each well was 5 x 10⁵ cfu/ml. Plates are returned to a 35°C incubator. The following day (or 24 hours in the case of Enterococci strains), MICs are determined by visual inspection of the plates.

Bacterial strains which may be tested in this model include, but are not limited to, those found in Table I and Table II below. Growth conditions may be modified as necessary for each particular strain. Growing conditions and growth media for the strains listed in Table I and Table II are known in the art.

### Determination of Kill Time

Experiments to determine the time required to kill the bacteria are conducted as described in Lorian. These experiments are conducted with both staphylococcus and enterococcus strains.

Briefly, several colonies are selected from an agar plate and grown at 35°C under constant agitation until a turbidity of approximately 1.5 X 10⁸ CFU/ml is achieved. The sample is diluted to about 6 x 10⁶ CFU/ml and incubated at 35°C under constant agitation. At various times, aliquots are removed and five ten-fold serial dilutions are performed. The pour plate method is used to determine the number of colony forming units (CFUs).

### In Vivo Determination of Antibacterial Activity

### Acute tolerability studies in mice

In these studies, the compounds of Formula I are administered either intravenously or subcutaneously and observed for 5-15 minutes. If there are no adverse effects, the dose is increased in a second group of mice. This dose incrementation continues until mortality occurs, or the dose is maximized. Generally, dosing begins at 20 mg/kg and increases by 20 mg/kg each time until the maximum tolerated dose (MTD) is achieved.

### Bioavailability studies in mice

Mice are administered the compound of Formula I either intravenously or subcutaneously at a therapeutic dose (in general, approximately 50 mg/kg). Groups of animals are placed in metabolic cages so that urine and feces may be collected for analysis. Groups of animals (n=3) are sacrificed at various times (10 min, 1 hour and 4 hours). Blood is collected by cardiac puncture and the following organs are harvested: lung, liver, heart, brain, kidney, and spleen. Tissues were weighed and prepared for HPLC analysis. HPLC analysis on the tissue homogenates and fluids is used to determine the concentration of the compound of Formula I. Metabolic products resulting from changes to the compound of Formula I are also determined.

### Mouse septecemia model

In this model, an appropriately virulent strain of bacteria (most commonly S. aureus, or E. faecalis or E. faecium) is administered intraperitoneally to mice (N=5 to 10 mice per group). The bacteria was combined with hog gastric mucin to enhance virulence. The dose of bacteria (normally 10⁵-10⁷) is that which is sufficient to induce mortality in all of the mice over a three day period. One hour after the bacteria is administered, the compound of Formula I is administered in a single dose, either IV or subcutaneously. Each dose is administered to groups of 5 to 10 mice, at doses that typically range from a maximum of about 20 mg/kg to a minimum of less than 1 mg/kg. A positive control (normally β-lactam with β-lactam sensitive strains) is administered in each experiment. The dose at which approximately 50% of the animals are saved is calculated from the results.

### Neutropenic thigh model

In this model, antibacterial activity of the compound of Formula I is evaluated against an appropriately virulent strain of bacteria (most commonly S. aureus sensitive or resistant to β-lactams). Mice are initially rendered neutropenic by administration of cyclophosphamide at 200 mg/kg on day 0 and day 2. On day 4, they are infected in the left anterior thigh by an IM injection of a single dose of bacteria. The mice are administered the compound of Formula I one hour after the administration of bacteria. At various later times (normally 1, 2.5, 4 and 24 hours) the mice are sacrificed (3 per time point). The thigh is excised, homogenized and the number of CFUs (colony forming units) is determined by plating. Blood is also plated to determine the CFUs in the blood.

### Pharmacokinetic studies

The rate at which the compound of Formula I is removed from the blood can be determined in either rats or mice. In rats, the test animals are cannulated in the jugular vein. A compound of Formula I is administered via tail vein injection, and at various time points (normally 5, 15, 30, 60 minutes and 2, 4, 6 and 24 hours) blood is withdrawn from the cannula. In mice, a compound of Formula I is also administered via tail vein injection, and at various time points. Blood is normally obtained by cardiac puncture. The concentration of the remaining compound of Formula I is determined by HPLC.

## Claims

1. A compound of Formula (I):
(L)ₚ(X)_{q} (I)
or a pharmaceutically acceptable salt thereof, wherein:
*p* is 2;
*q* is 1;
one ligand, L, is a beta lactam antibiotic, and the other ligand, L, is an optionally substituted glycopeptide antibiotic, or an aglycone derivative of an optionally substituted glycopeptide antibiotic; and
X is a linker;
provided that when one of the ligands is vancomycin attached via the carboxy terminus, then the other ligand cannot be cefalexin attached to the linker via acylation of its alpha amino group.

2. The compound of Claim 1 wherein:
the ligand that is a beta lactam antibiotic is selected from penems, penams, cephems, carbapenems, oxacephems, carbacephems, and monobactam ring systems; and
the ligand that is a glycopeptide antibiotic is selected from Chloroeremomycin, Chloroorienticin, Vancomycin and aglycone derivatives thereof.

3. The compound of Claim 2 wherein the ligand that is a beta lactam antibiotic is selected from:
(i) a ligand of formula (a): 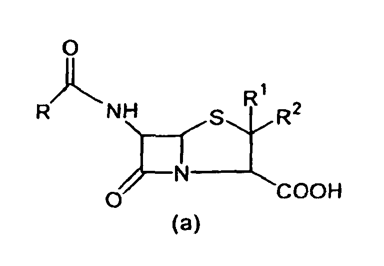 wherein:
R is substituted alkyl, aryl, aralkyl, or heteroaryl wherein each of said substituent optionally links (a) to the linker via a covalent bond or R is a covalent bond that links (a) to the linker; and
R¹ and R² are, independently of each other, alkyl or one of R¹ and R² is a covalent bond linking (a) to the linker;
(ii) a ligand of formula (b): 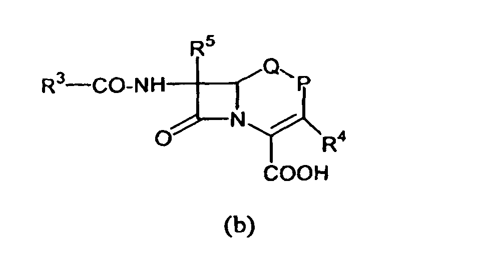 wherein:
one of P and Q is O, S, or -CH₂- and the other is -CH₂-;
R³ is substituted alkyl, heteroarylalkyl, aralkyl, heterocyclylalkyl, or -C(R⁶)=NOR⁷ (where R⁶ is aryl, heteroaryl, or substituted alkyl; and R⁷ is alkyl or substituted alkyl) wherein each of said substituents optionally links (b) to a linker or R³ is a covalent bond that links (b) to the linker; and
R⁴ is hydrogen, alkyl, alkenyl, substituted alkenyl, substituted alkyl, halo, heteroarylalkyl, heterocyclylalkyl, -SR^{a} (where R^{a} is aryl, heteroaryl, heterocyclyl, or cycloalkyl) or -CH₂SR^{a} (where R^{a} is aryl, heteroaryl, heterocyclyl, or cycloalkyl) wherein each of said substituents optionally links (b) to a linker or R⁴ is a covalent bond that links (b) to the linker;
R⁵ is hydrogen, hydroxy, or alkoxy;
(iii) a ligand of formula (c): 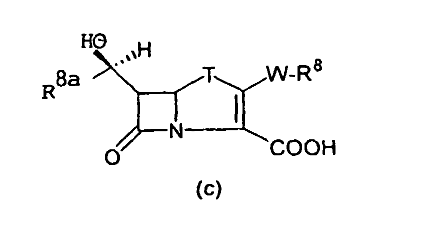 wherein:
T is S or CH₂;
R^{8a} is alkyl;
W is O, S, -OCH₂-, or CH_{2;} and R⁸ is -(alkylene)-NHC(R^{b})=NH where R^{b} is a covalent bond linking (c) to a linker; or -W-R⁸ is a covalent bond that links (c) to the linker;
(iv) a ligand of formula (d): 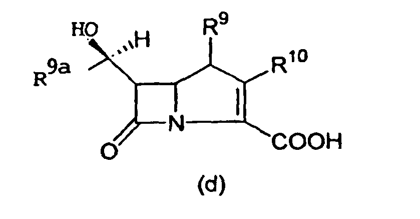 wherein:
R⁹ and R^{9a} are alkyl;
R'° is selected from the group consisting of hydrogen, alkyl, substituted alkyl, halo, aryl, heteroaryl, heterocyclyl, aralkyl, heteroaralkyl, heterocyclylalkyl or -CH₂SR^{a} (where R^{a} is aryl, heteroaryl, heterocyclyl, or cycloalkyl) wherein each of said substituents optionally links (d) to the linker or one of R⁹ and R¹⁰ is a covalent bond that links (d) to the linker; or
R⁹ and R¹⁰ together with the carbon atoms to which they are attached form an aryl, heteroaryl, cycloalkyl, substituted cycloalkyl, or heterocyclyl ring of 4 to 7 ring atoms wherein one of the ring atoms optionally links (d) to the linker; or
(v) a ligand of formula (e): 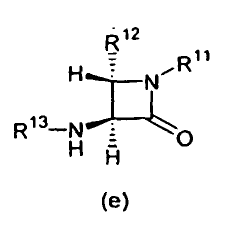 wherein:
R¹¹ is -SO₃H or -(alkylene)-COOH;
R¹² is alkyl, substituted alkyl, haloalkyl, alkoxy, aryl, aralkyl, heteroaryl, heteroaralkyl, cycloalkyl, substituted cycloalkyl, or heterocyclyl wherein each of said substituents optionally binds (e) to the linker or R¹² is a covalent bond that links (e) to the linker; and
R¹³ is alkyl, acyl, or -COC(R¹⁴)=N-OR¹⁵ wherein R¹⁴ is aryl, heteroaryl which optionally links (e) to the linker, and R¹⁵ is -(alkylene)-COOR¹⁶ wherein R¹⁶ is hydrogen or a covalent bond which optionally links (e) to the linker or R¹³ is a covalent bond that links (e) to the linker; and
the ligand that is a glycopeptide antibiotic is an optionally substituted vancomycin which is linked to the linker via any hydroxyl group, carboxyl group or amino group.

4. The compound of Claim 3 wherein the ligand that is a beta lactam antibiotic is selected from:
(i) a ligand of formula (a): 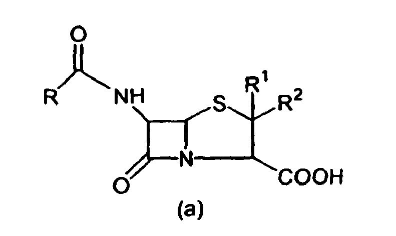 wherein:
R is: 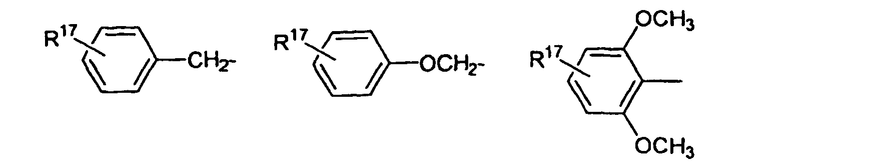 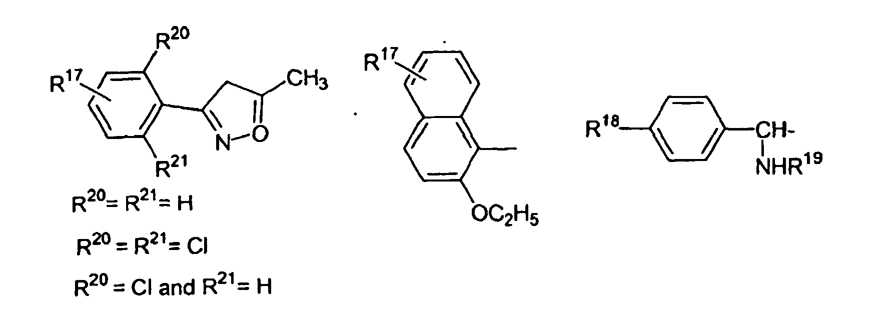 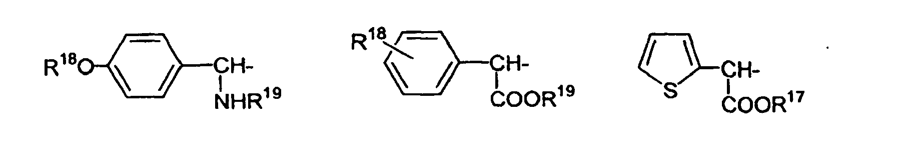 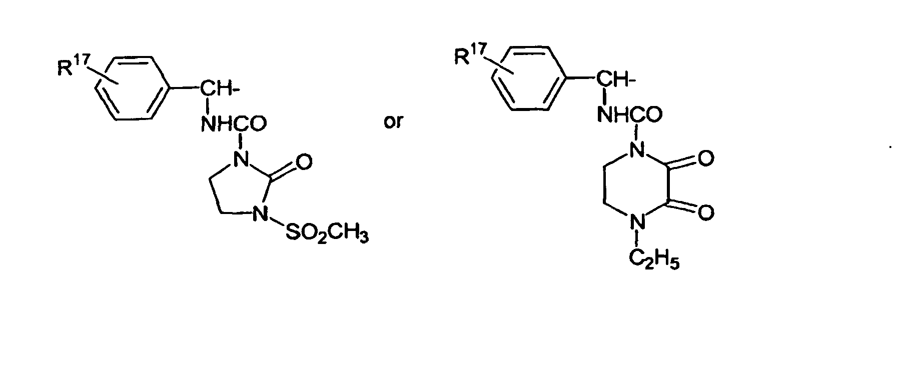
where:
R¹⁷ is a covalent bond that links the (a) group to the linker;
one of R¹⁸ and R¹⁹ is hydrogen and the other is a covalent bond that links the (a) group to the linker; and
R¹ and R² are methyl;
(ii) a ligand of formula (b): 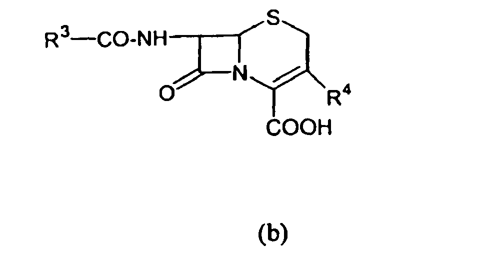 where:
R³ and R⁴ are: 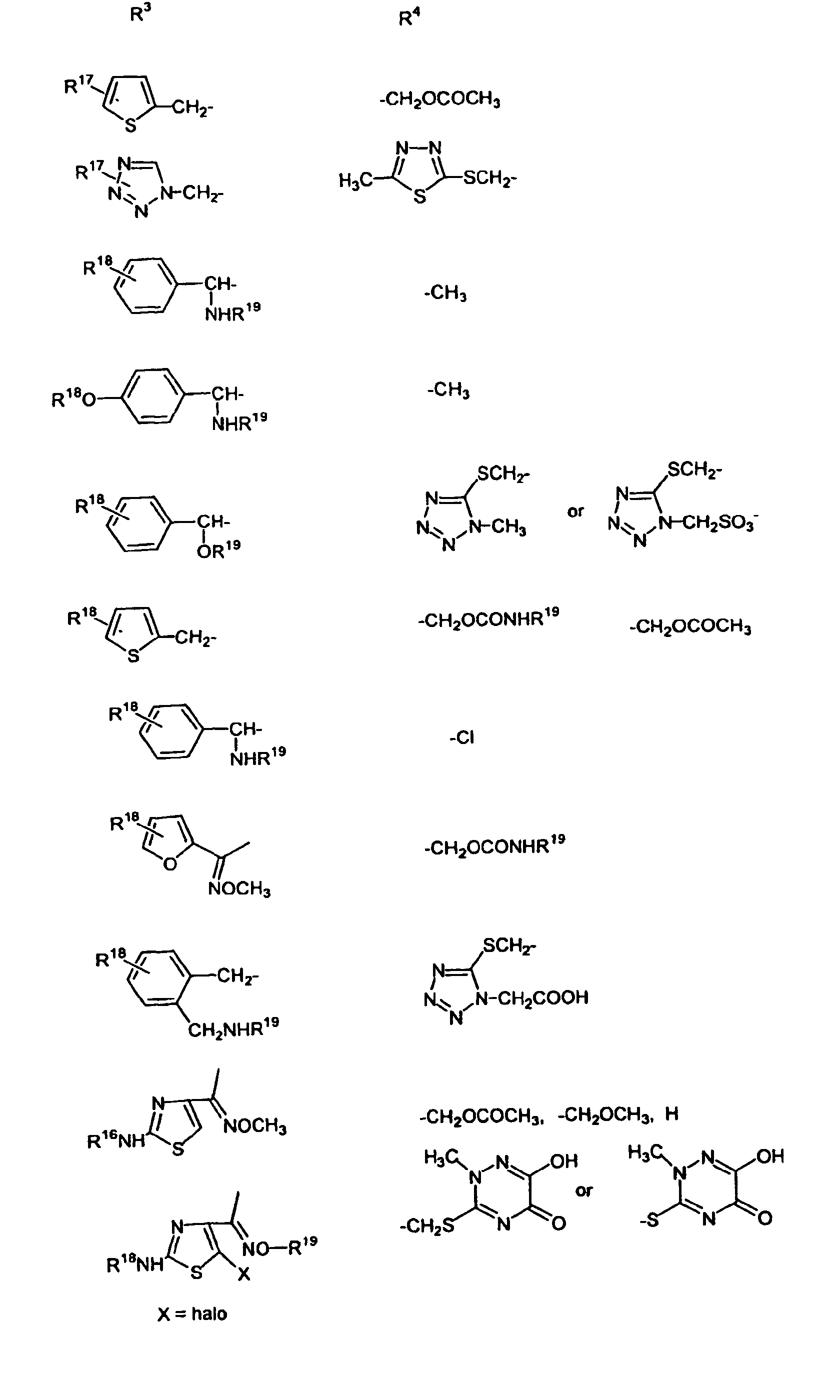 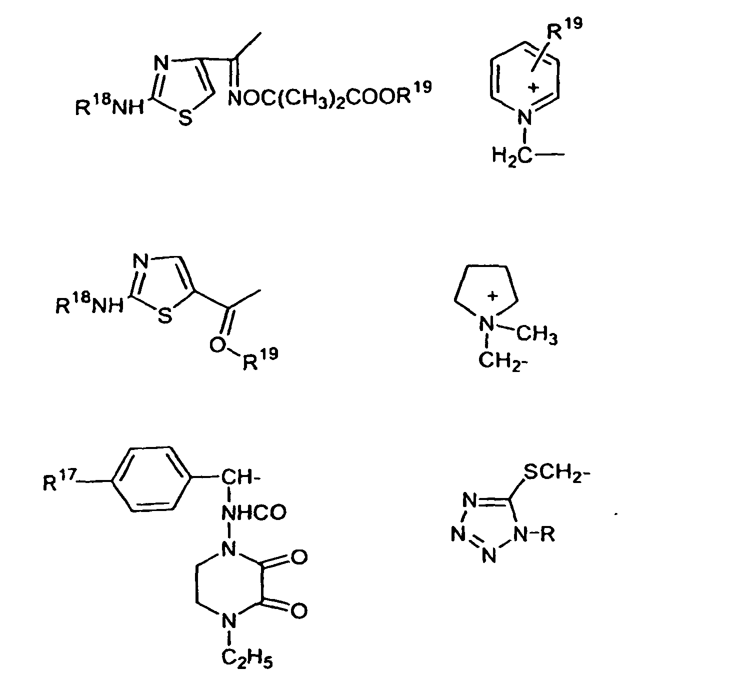
wherein:
R is alkyl;
R¹⁶ is a covalent bond that links the (b) group to the linker;
one of R¹⁸ and R¹⁹ is hydrogen or alkyl and the other is a covalent bond that links the (b) group to the linker;
(iii) a ligand of formula (c): 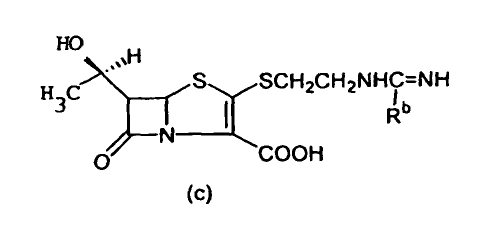 wherein R^{b} is a covalent bond linking (c) to the linker;
(iv) a ligand of formula (d): 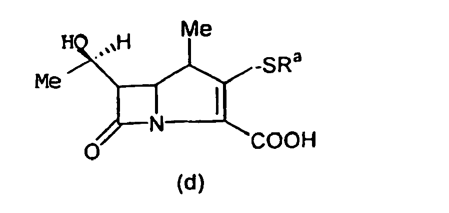 where R^{a} is: 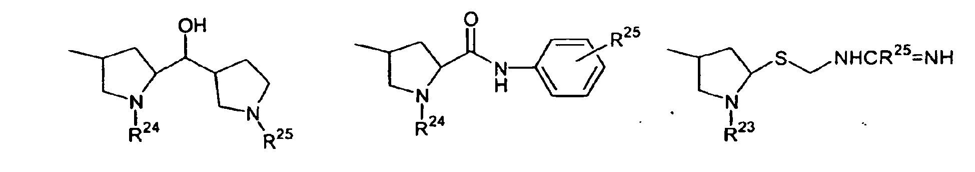 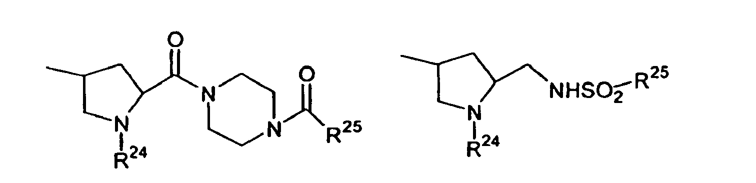 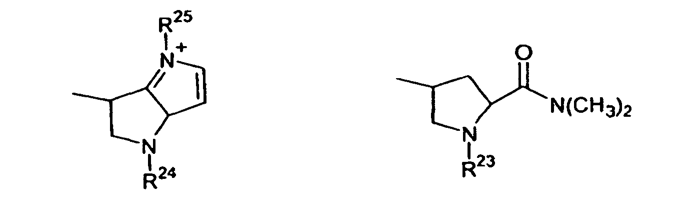 where:
R²³ is a covalent bond that links (d) to the linker;
one of R²⁴ and R²⁵ is alkyl, substituted alkyl, or aralkyl, and other is a covalent bond that links (d) to the linker; or
(v) a ligand of formula (e): 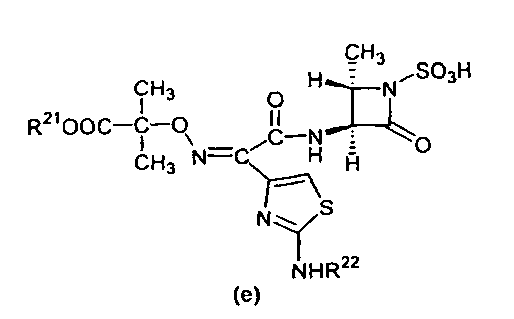
wherein one of R²¹ and R²² is hydrogen and the other links (d) to the linker.

5. The multibinding compound of Claim 4 wherein the linker is selected from a compound of formula:
-X^{a}-Z-(Y^{a}-Z)ₘ-X^{a}-
wherein
*m* is an integer of from 0 to 20;
X^{a} at each separate occurrence is selected from -O-, -S-, -NR-, -C(O)-, -C(O)O-, -OC(O)-, -C(O)NR-, -NRC(O)-, C(S), -C(S)O-, -C(S)NR-, -NRC(S)-, or a covalent bond where R is as defined below;
Z at each separate occurrence is selected from alkylene, substituted alkylene, cycloalkylene, substituted cylcoalkylene, alkenylene, substituted alkenylene, alkynylene, substituted alkynylene, cycloalkenylene, substituted cycloalkenylene, arylene, heteroarylene, heterocyclene, or a covalent bond;
each Y^{a} at each separate occurrence is selected from -O-, -C(O)-, -OC(O)-, -C(O)O-, -NR-, -S(O)n-, -C(O)NR'-, -NR'C(O)-, -NR'C(O)NR'-, -NR'C(S)NR'-, -C(=NR')-NR'-, -NR'-C(=NR')-, -OC(O)-NR'-, -NR'-C(O)-O-, -P(O)(OR')-O-, -O-P(O)(OR')-, -S(O)ₙCR'R"-, -S(O)ₙ-NR'-, -NR'-S(O)ₙ-, -S-S-, and a covalent bond; where n is 0, 1 or 2; and R, R' and R" at each separate occurrence are selected from hydrogen, alkyl, substituted alkyl, cycloalkyl, substituted cycloalkyl, alkenyl, substituted alkenyl, cycloalkenyl, substituted cycloalkenyl, alkynyl, substituted alkynyl, aryl, heteroaryl and heterocyclic.

6. A compound as claimed in claim 1, which is Vancomycin-Amoxicillin heterodimer of structure 19; Vancomycin-Imipenem heterodimer of structure 30; or Vancomycin-Imipenem heterodimer of structure 33:

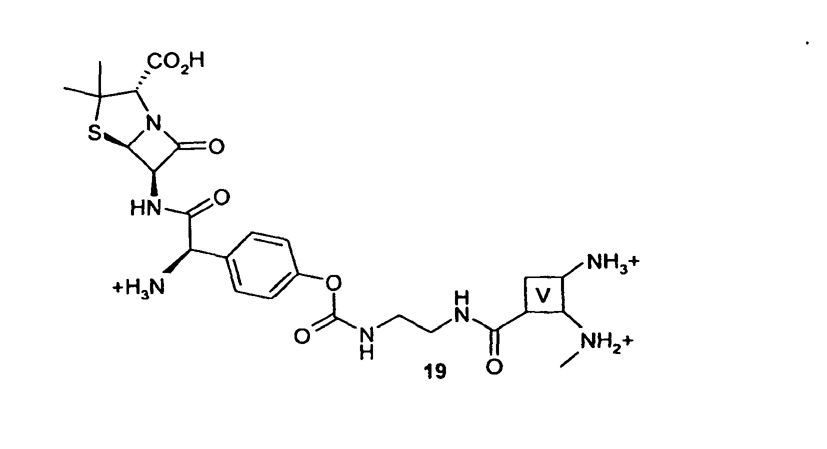

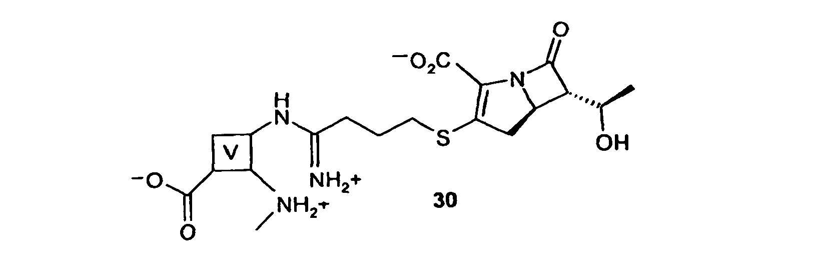

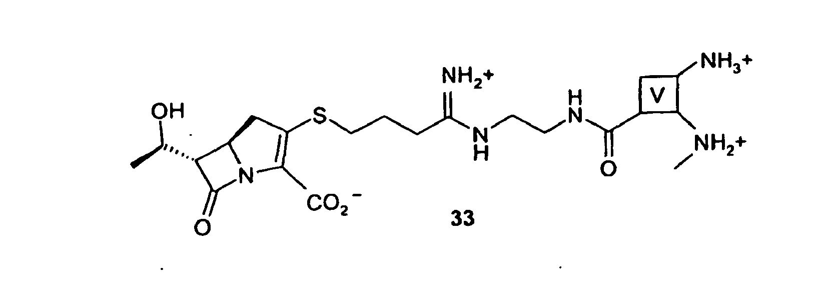

7. A pharmaceutical composition comprising a pharmaceutically acceptable carrier and an effective amount of a compound of any of claims 1 to 6.

8. A compound as claimed in any one of claims 1 to 6, or a pharmaceutical composition as claimed in claim 7 for use as a medicament.

9. A compound as claimed in claim 8 for use as a medicament for treating a bacterial disease in a mammal.

10. Use of a compound as claimed in any one of claims 1 to 6, or a pharmaceutical composition as claimed in claim 7, for the manufacture of a medicament for treating a bacterial disease in a mammal.

## Patentansprüche

1. Eine Verbindung der Formel (I):
(L)ₚ(X)_{q} (I)
oder ein pharmazeutisch annehmbares Salz davon, wobei:
p 2 ist,
q 1 ist,
ein Ligand L ein Betalactam-Antibiotikum ist und der andere Ligand L ein gegebenenfalls substituiertes Glycopeptid-Antibiotikum oder ein Aglykon-Derivat eines gegebenenfalls substituierten Glycopeptid-Antibiotikums ist und
X ein Linker ist,
mit der Maßgabe, daß, wenn einer der Liganden über das Carboxy-Ende gebundenes Vancomycin ist, der andere Ligand dann nicht durch Acylierung seiner alpha-Aminogruppe an den Linker gebundenes Cefalexin sein kann.

2. Die Verbindung nach Anspruch 1, wobei:
der Ligand, der ein Betalactam-Antibiotikum ist, ausgewählt ist aus Penemen, Penamen, Cephemen, Carbapenemen, Oxacephemen, Carbacephemen und Monobactamringsystemen und
der Ligand, der ein Glycopeptid-Antibiotikum ist, ausgewählt ist aus Chloreremomycin, Chlororienticin, Vancomycin und Aglykon-Derivaten davon.

3. Die Verbindung nach Anspruch 2, wobei der Ligand, der ein Betalactam-Antibiotikum ist, ausgewählt ist aus
(i) einem Liganden der Formel (a): 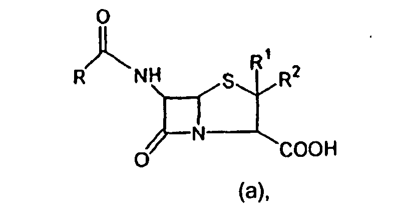 wobei:
R substituiertes Alkyl, Aryl, Aralkyl oder Heteroaryl ist, wobei jeder der Substituenten gegebenenfalls (a) mittels einer kovalenten Bindung an den Linker bindet, oder R eine kovalente Bindung ist, die (a) an den Linker bindet, und
R¹ und R² jeweils unabhängig voneinander Alkyl sind oder einer der Reste R¹ und R² eine kovalente Bindung ist, die (a) an den Linker bindet,
(ii) einem Liganden der Formel (b): 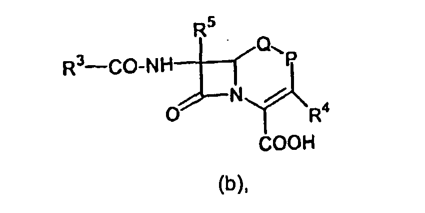 wobei:
eine der Variablen P und Q O, S oder -CH₂- ist und die andere -CH₂- ist,
R³ substituiertes Alkyl, Heteroarylalkyl, Aralkyl, Heterocyclylalkyl oder -C(R⁶)=NOR⁷ ist (wobei R⁶ Aryl, Heteroaryl oder substituiertes Alkyl ist und R⁷ Alkyl oder substituiertes Alkyl ist), wobei jeder der Substituenten gegebenenfalls (b) an einen Linker bindet, oder R³ eine kovalente Bindung ist, die (b) an den Linker bindet, und
R⁴ Wasserstoff, Alkyl, Alkenyl, substituiertes Alkenylen, substituiertes Alkyl, Halogen, Heteroarylalkyl, Heterocyclylalkyl, -SR^{a} (wobei R^{a} Aryl, Heteroaryl, Heterocyclyl oder Cycloalkyl ist) oder -CH₂SR^{a} (wobei R^{a} Aryl, Heteroaryl, Heteroeyclyl oder Cycloalkyl ist) ist, wobei jeder der Substituenten gegebenenfalls (b) an einen Linker bindet, oder R⁴ eine kovalente Bindung ist, die (b) an den Linker bindet,
R⁵ Wasserstoff, Hydroxy oder Alkoxy ist,
(iii) einem Liganden der Formel (c): 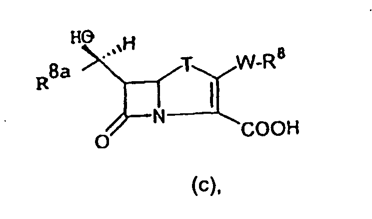 wobei:
T S oder CH₂ ist,
R^{8a} Alkyl ist,
W O, S, -OCH₂- oder CH₂ ist und R⁸ -(Alkylen)-NHC(R^{b})=NH ist, wobei R^{b} eine kovalente Bindung ist, die (c) an einen Linker bindet, oder -W-R⁸ eine kovalente Bindung ist, die (c) an den Linker bindet,
(iv) einem Liganden der Formel (d): 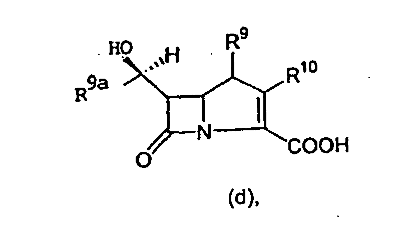 wobei:
R⁹ und R^{9a} Alkyl sind,
R¹⁰ ausgewählt ist aus der Gruppe, bestehend aus Wasserstoff, Alkyl, substituiertem Alkyl, Halogen, Aryl, Heteroaryl, Heterocyclyl, Aralkyl, Heteroaralkyl, Heterocyclylalkyl oder -CH₂SR^{a} (wobei R^{a} Aryl, Heteroaryl, Heterocyclyl oder Cycloalkyl ist), wobei jeder der Substituenten gegebenenfalls (d) an den Linker bindet, oder einer der Reste R⁹ und R¹⁰ eine kovalente Bindung ist, die (d) an den Linker bindet, oder
R⁹ und R¹⁰, zusammen mit den Kohlenstoffatomen, an die sie gebunden sind, ein Aryl, Heteroaryl, Cycloalkyl, substituiertes Cycloalkyl oder einen heterocyclischen Ring aus 4 bis 7 Ringatomen bilden, wobei eines der Ringatome gegebenenfalls (d) an den Linker bindet, oder
(v) einem Liganden der Formel (e): 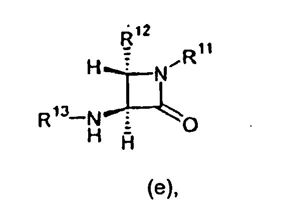 wobei:
R¹¹ -SO₃H oder -(Alkylen)-COOH ist,
R¹² Alkyl, substituiertes Alkyl, Halogenalkyl, Alkoxy, Aryl, Aralkyl, Heteroaryl, Heteroaralkyl, Cycloalkyl, substituiertes Cycloalkyl oder Heterocyclyl ist, wobei jeder der Substituenten gegebenenfalls (e) an den Linker bindet, oder R¹² eine kovalente Bindung ist, die (e) an den Linker bindet, und
R¹³ Alkyl, Acyl oder -COC(R¹⁴)=N-OR¹⁵ ist, wobei R¹⁴ Aryl, Heteroaryl ist, das gegebenenfalls (e) an den Linker bindet, und R¹⁵ -(Alkylen)-COOR¹⁶ ist, wobei R¹⁶ Wasserstoff oder eine kovalente Bindung ist, die gegebenenfalls (e) an den Linker bindet, oder R¹³ eine kovalente Bindung ist, die (e) an den Linker bindet, und
der Ligand, der ein Glycopeptid-Antibiotikum ist, ein gegebenenfalls substituiertes Vancomycin ist, das durch irgendeine Hydroxylgruppe, Carboxylgruppe oder Aminogruppe an den Linker gebunden ist.

4. Die Verbindung nach Anspruch 3, wobei der Ligand, der ein Betalactam-Antibiotikum ist, ausgewählt ist aus
(i) einem Liganden der Formel (a): 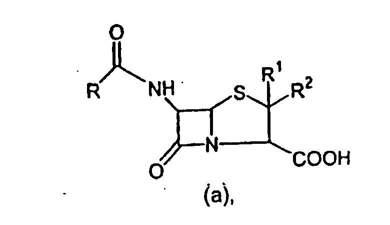 wobei:
R ist: 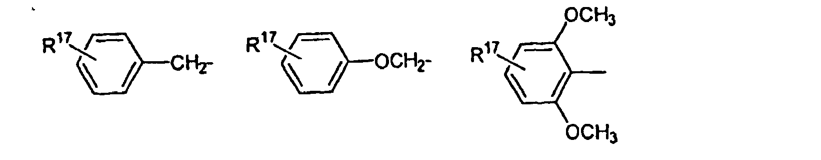 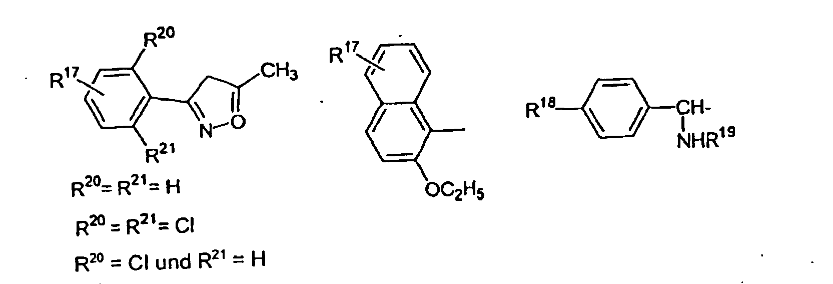 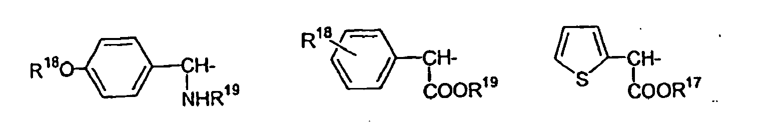 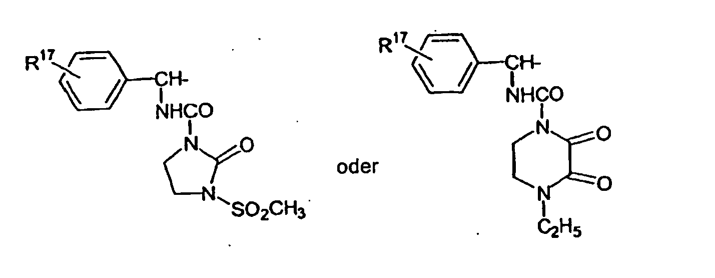
wobei:
R¹⁷ eine kovalente Bindung ist, die die Gruppe (a) an den Linker bindet,
einer der Reste R¹⁸ und R¹⁹ Wasserstoff ist und der andere eine kovalente Bindung ist, die die Gruppe (a) an den Linker bindet, und
R¹ und R² Methyl sind,
(ii) einem Liganden der Formel (b): 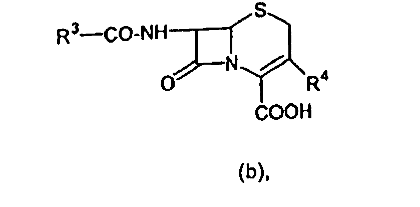 wobei:
R³ und R⁴ sind: 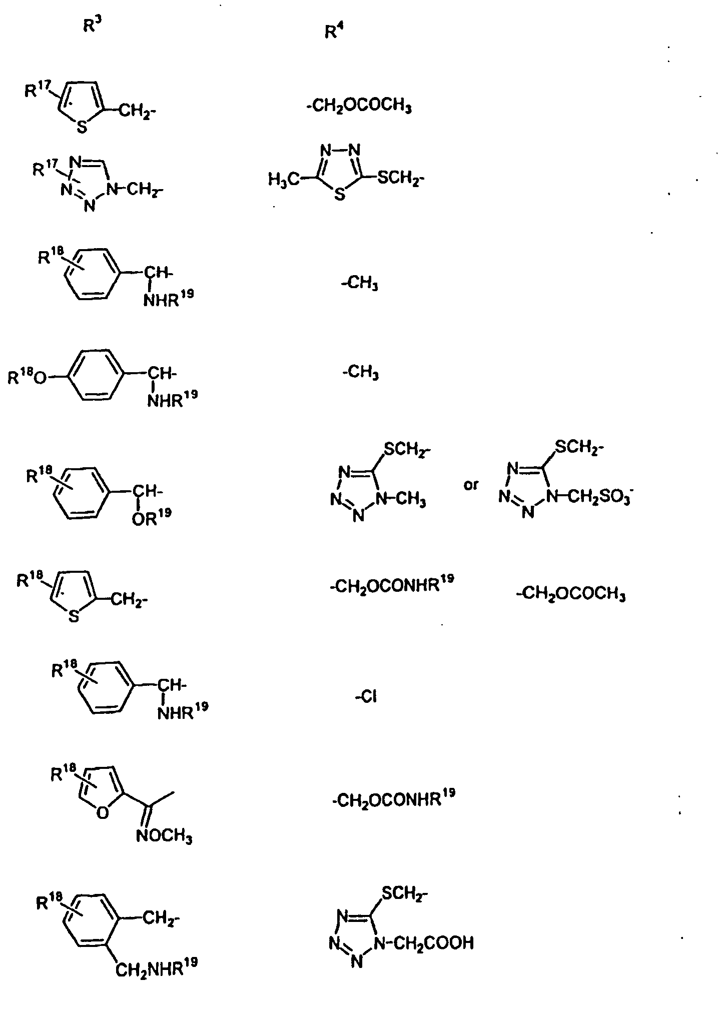 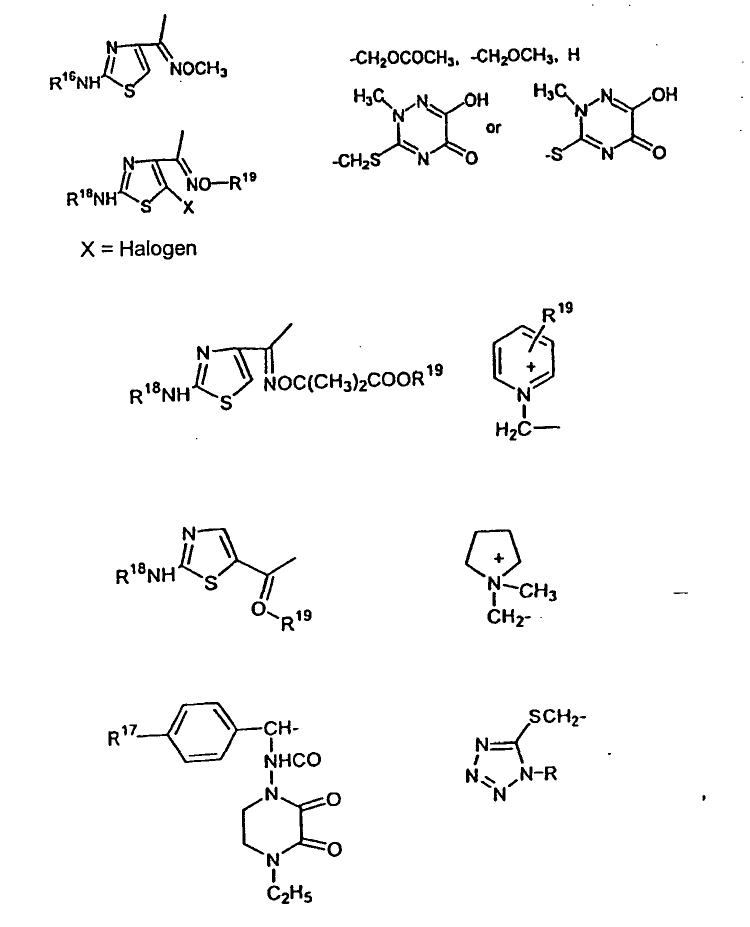
wobei:
R Alkyl ist,
R¹⁶ eine kovalente Bindung ist, die die Gruppe (b) an den Linker bindet,
einer der Reste R¹⁸ und R¹⁹ Wasserstoff oder Alkyl ist und der andere eine kovalente Bindung ist, die die Gruppe (b) an den Linker bindet,
(iii) einem Liganden der Formel (c): 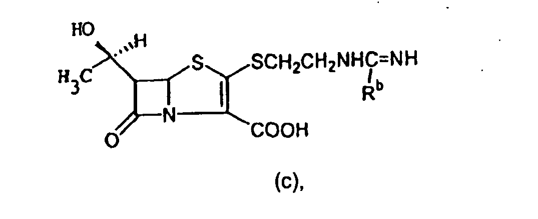 wobei R^{b} eine kovalente Bindung ist, die (c) an den Linker bindet,
(iv) einem Liganden der Formel (d): 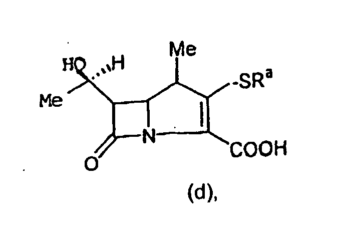 wobei R^{a} ist: 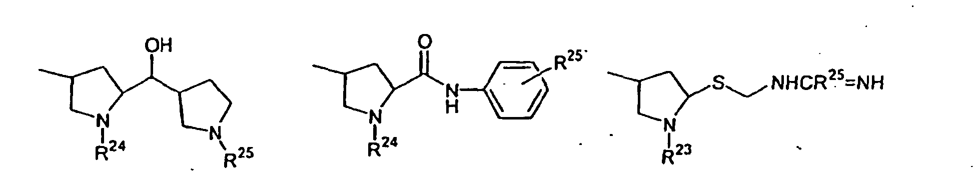 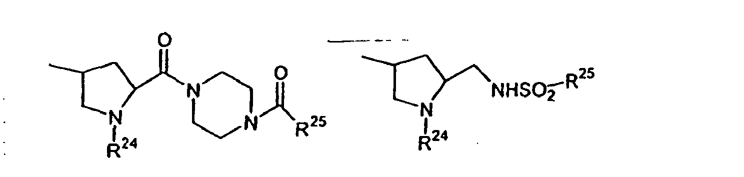 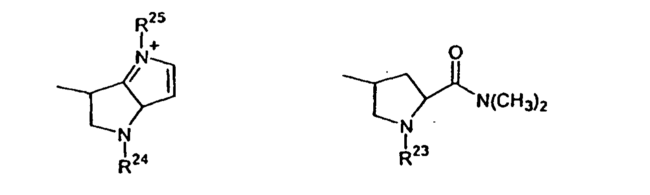 wobei:
R²³ eine kovalente Bindung ist, die (d) an den Linker bindet,
einer der Reste R²⁴ und R²⁵ Alkyl, substituiertes Alkyl oder Aralkyl ist und der andere eine kovalente Bindung ist, die (d) an den Linker bindet, oder
(v) einem Liganden der Formel (e): 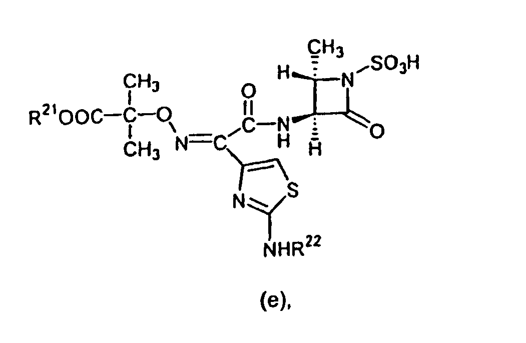
wobei einer der Reste R²¹ und R²² Wasserstoff ist und der andere (d) an den Linker bindet.

5. Die mehrbindige Verbindung nach Anspruch 4, wobei der Linker ausgewählt ist aus einer Verbindung der Formel:
-X^{a}-Z-(Y^{a}-Z)ₘ-X^{a}-,
wobei
m eine Zahl von 0 bis 20 ist,
X^{a} bei jedem einzelnen Auftreten ausgewählt ist aus -O-, -S-, -NR-, -C(O)-, -C(O)O-, -OC(O)-, -C(O)NR-, -NRC(O)-, C(S), -C(S)O-, -C(S)NR-, -NRC(S)- oder einer kovalenten Bindung, wobei R wie nachstehend definiert ist,
Z bei jedem einzelnen Auftreten ausgewählt ist aus Alkylen, substituiertem Alkylen, Cycloalkylen, substituiertem Cycloalkylen, Alkenylen, substituiertem Alkenylen, Alkinylen, substituiertem Alkinylen, Cycloalkenylen, substituiertem Cycloalkenylen, Arylen, Heteroarylen, Heterocyclen oder einer kovalenten Bindung,
jedes Y^{a} bei jedem einzelnen Auftreten ausgewählt ist -O-, -C(O)-, -OC(O)-, -C(O)O-, -NR-, -S(O)ₙ-, -C(O)NR'-, -NR'C(O)-, -NR'C(O)NR'-, -NR'C(S)NR'-, -C(=NR')-NR'-, -NR'-C(=NR')-, -OC(O)-NR'-, -NR'-C(O)-O-, -P(O)(OR')-O-, -O-P(O)(OR')-, -S(O)ₙCR'R"-, -S(O)ₙ-NR'-, -NR'-S(O)ₙ-, -S-S- und einer kovalenten Bindung, wobei n 0, 1 oder 2 ist und R, R' und R" bei jedem einzelnen Auftreten ausgewählt sind aus Wasserstoff, Alkyl, substituiertem Alkyl, Cycloalkyl, substituiertem Cycloalkyl, Alkenyl, substituiertem Alkenyl, Cycloalkenyl, substituiertem Cycloalkenyl, Alkinyl, substituiertem Alkinyl, Aryl, Heteroaryl und Heterocyclus.

6. Eine Verbindung wie in Anspruch 1 beansprucht, die Vancomycin-Amoxicillin-Heterodimer der Struktur 19, Vancomycin-Imipenem-Heterodimer der Struktur 30 oder Vancomycin-Imipenem-Heterodimer der Struktur 33 ist:

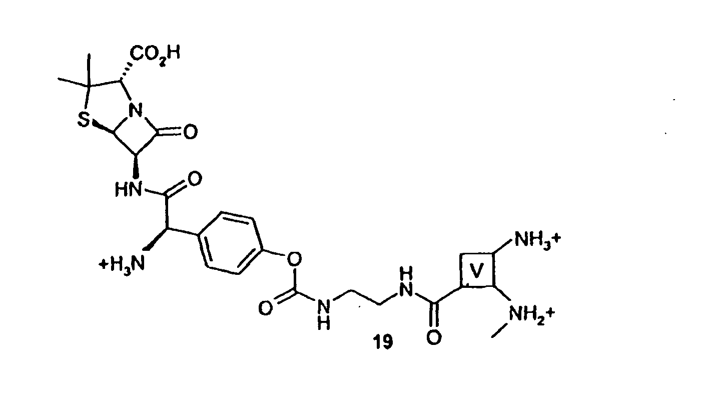

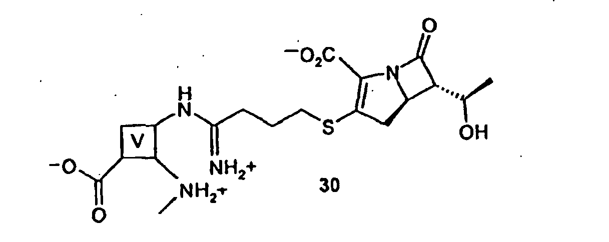

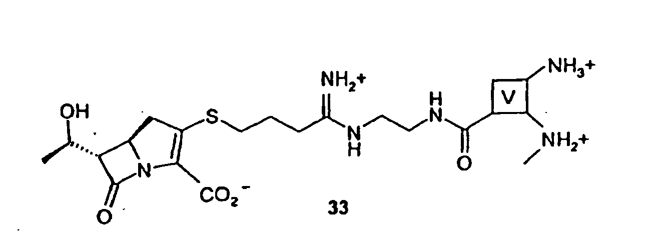

7. Eine pharmazeutische Zusammensetzung, die einen pharmazeutisch annehmbaren Träger und eine wirksame Menge einer Verbindung nach irgendeinem der Ansprüche 1 bis 6 enthält.

8. Eine wie in irgendeinem der Ansprüche 1 bis 6 beanspruchte Verbindung oder eine wie in Anspruch 7 beanspruchte pharmazeutische Zusammensetzung zur Verwendung als ein Medikament.

9. Eine wie in Anspruch 8 beanspruchte Verbindung zur Verwendung als ein Medikament zur Behandlung einer bakteriellen Erkrankung bei einem Säuger.

10. Die Verwendung einer wie in irgendeinem der Ansprüche 1 bis 6 beanspruchten Verbindung oder einer wie in Anspruch 7 beanspruchten Zusammensetzung zur Herstellung eines Medikaments zur Behandlung einer bakteriellen Erkrankung bei einem Säuger.

## Revendications

1. Le composé de formule (I):
(L)ₚ(X)_{q} (I)
dans laquelle:
p est 2;
q est 1;
un ligand L est un antibiotique de la famille des bêta-lactames, et l'autre ligand L est un antibiotique de la famille des glycopeptides le cas échéant substitué, ou un dérivé aglycone d'un glycopeptide antibiotique le cas échéant substitué, et
X est un lieur,
pourvu que lorsque l'un des ligands est la vancomycine attachée via l'extrémité carboxy, l'autre ligand ne peut être la céfalexine attachée au lieur via une acylation de son groupe alpha-amino.

2. Le composé selon la revendication 1, dans lequel:
le ligand qui est un antibiotique bêta-lactame est choisi dans le groupe comprenant: les pénèmes, les pénames, les céphèmes, les carbapénèmes, les oxacéphèmes, les carbacéphèmes et les systèmes cycliques monobactame, et
le ligand qui est un glycopeptide antibiotique est choisi dans le groupe comprenant: la chloroérémomycine, la chloroorienticine, la vancomycine et leurs dérivés aglycone.

3. Le composé selon la revendication 2, dans lequel le ligand qui est un antibiotique bêta-lactame est choisi dans le groupe comprenant:
(i) un ligand de la formule (a): 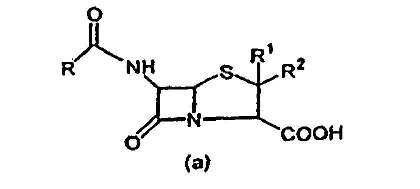 dans laquelle:
R est un alkyle, aryle, aralkyle ou hétéroaryle substitué, où chacun desdits substituants lie le cas échéant, (a) au lieur via une liaison covalente, ou R est une liaison covalente qui lie (a) au lieur, et
R¹ et R² sont indépendamment l'un de l'autre, alkyle ou un de R¹ et R² est une liaison covalente, liant (a) au lieur;
(ii) un ligand de la formule (b): 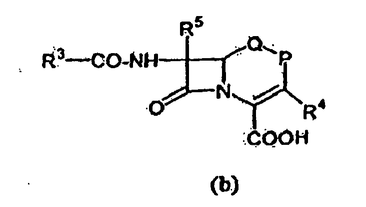 dans laquelle:
un de P et Q est O, S ou -CH₂ et l'autre est -CH₂;
R³ est un alkyle, hétéroarylalkyle, aralkyle, hétérocyclylalkyle substitué, ou C(R⁶)=NOR⁷ (où R⁶ est aryle, hétéroaryle ou alkyle substitué, et R⁷ est alkyle ou alkyle substitué), où chacun desdits substituants lie, le cas échéant, (b) au lieur ou R³ est une liaison covalente qui lie (b) au lieur, et
R⁴ est hydrogène, alkyle, alcényle, alcényle substitué, alkyle substitué, halo, hétéroarylalkyle, hétérocyclylalkyle, -SR^{a} (où R^{a} est aryle, hétéroaryle, hétérocyclique ou cycloalkyle) ou -CH₂SR^{a} (où R^{a} est aryle, hétéroaryle, hétérocyclique ou cycloalkyle), où chacun desdits substituants lie, le cas échéant, (b) au lieur ou R⁴ est une liaison covalente qui lie (b) au lieur, et
R⁵ est hydrogène, hydroxy ou alcoxy;
(iii) un ligand de la formule (c): 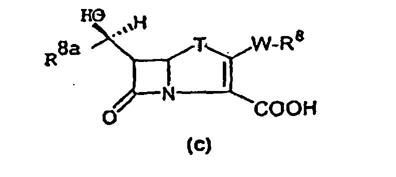 dans laquelle:
T est S ou -CH₂-;
R^{8a} est alkyle;
W est O, S, -OCH₂- ou -CH₂-, et R⁸ est -(alkylène)-NHC(R^{b})=NH, où R^{b} est une liaison covalente liant (c) au lieur, ou -W-R⁸ est une liaison covalente liant (c) au lieur,
(iv) un ligand de la formule (d): 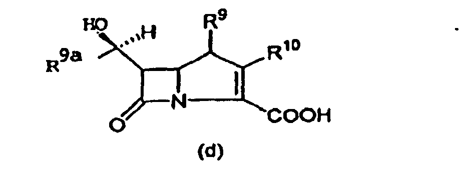 dans laquelle:
R⁹ et R^{9a} sont alkyle;
R¹⁰ est choisi dans le groupe comprenant: le groupe consistant en hydrogène, alkyle, alkyle substitué, halo, aryle, hétéroaryle, hétérocyclique, aralkyle, hétéroaralkyle, hétérocyclylalkyle ou -CH₂-SR^{a} (où R^{a} est aryle, hétéroaryle, hétérocyclique ou cycloalkyle), où chacun desdits substituants lie le cas échéant, (d) au lieur ou au moins un de R⁹ et R¹⁰ est une liaison covalente qui lie (d) au lieur, ou
R⁹ et R¹⁰ avec les atomes de carbone sur lesquels ils sont attachés, forment un cycle aryle, hétéroaryle, cycloalkyle, cycloalkyle substitué ou hétérocyclique à 4 à 7 atomes, où un des atomes cycliques lie, le cas échéant (d) au lieur, ou
(v) un ligand de formule (e): 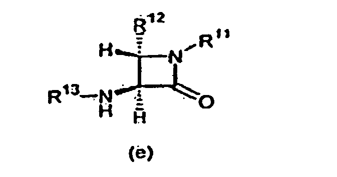 dans laquelle:
R¹¹ est -SO₃H ou -(alkylène)-COOH;
R¹² est alkyle, alkyle substitué, haloalkyle, alcoxy, aryle, aralkyle, hétéroaryle, hétéroaralkyle, cycloalkyle, cycloalkyle substitué ou hétérocyclique, où chacun desdits substituants lie le cas échéant, (e) au lieur ou R¹² est une liaison covalente qui lie (e) au lieur, et
R¹³ est alkyle, acyle ou -COC(R¹⁴)=N-OR¹⁵ où R¹⁴ est aryle, hétéroaryle, qui lie le cas échéant, (e) au lieur, et R¹⁵ est -(alkylène)-COOR¹⁶, où R¹⁶ est hydrogène ou lie le cas échéant, (e) au lieur ou R¹³ est une liaison covalente qui lie (e) au lieur,
et le ligand qui est un glycopeptide antibiotique, est une vancomycine le cas échéant substituée, qui est liée au lieur via un groupe hydroxyle, un groupe carboxyle ou un groupe amino.

4. Le composé selon la revendication 3, dans lequel le ligand qui est un antibiotique bêta-lactame est choisi dans le groupe comprenant:
(i) un ligand de la formule (a): 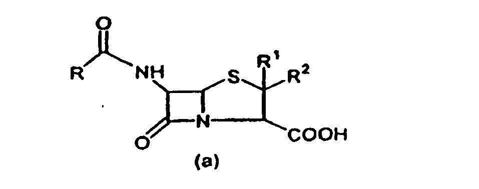 dans laquelle R est: 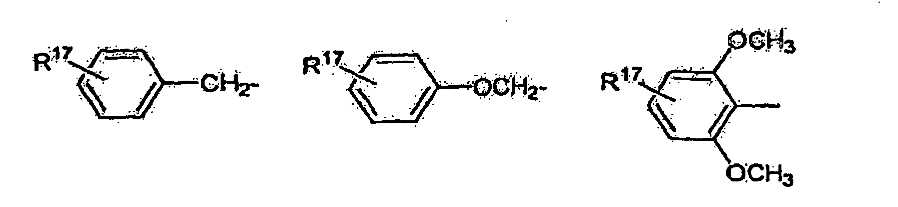 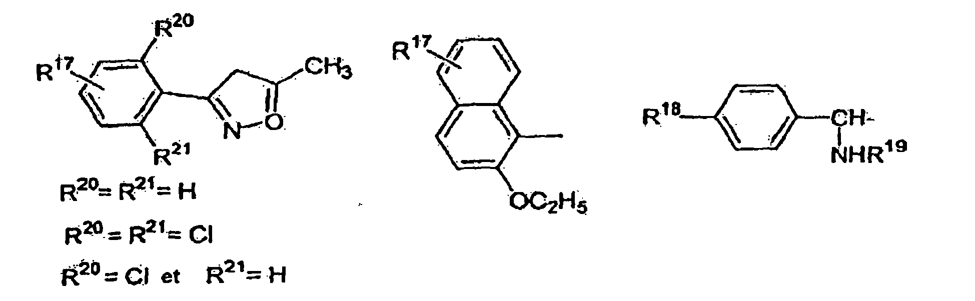 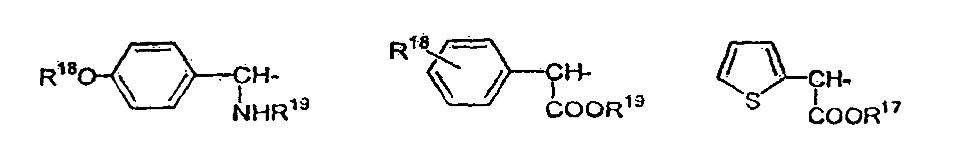 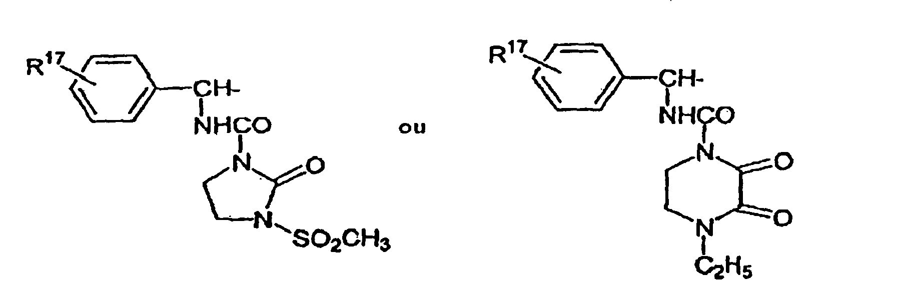 où R¹⁷ est une liaison covalente qui lie (a) au lieur,
un de R¹⁸ et R¹⁹ est hydrogène et l'autre est une liaison covalente, qui
lie le groupe (a) au lieur, et R¹ et R² sont méthyle;
(ii) un ligand de formule (b) 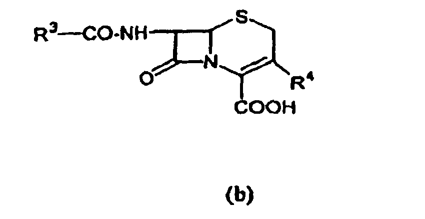 dans laquelle R³ et R⁴ sont: 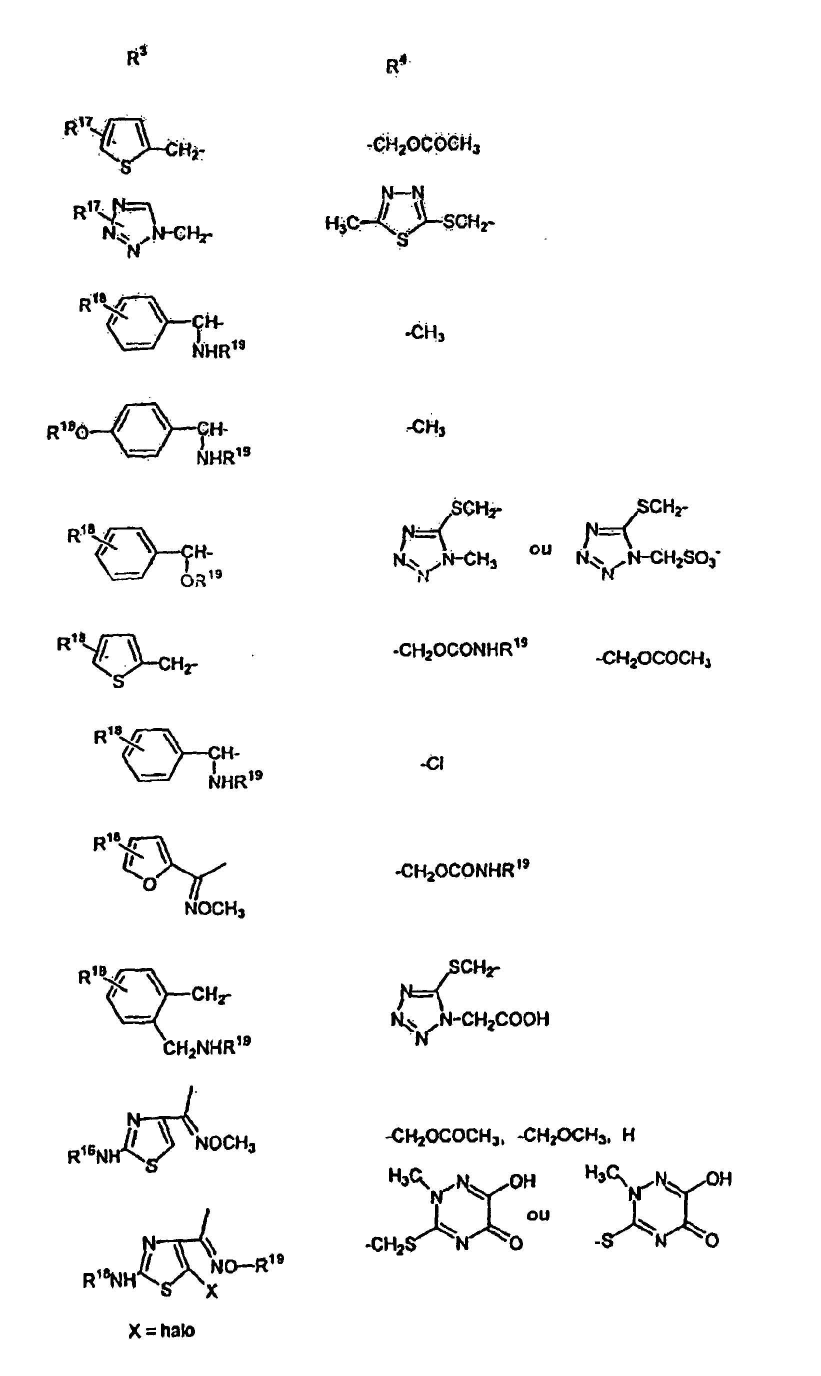 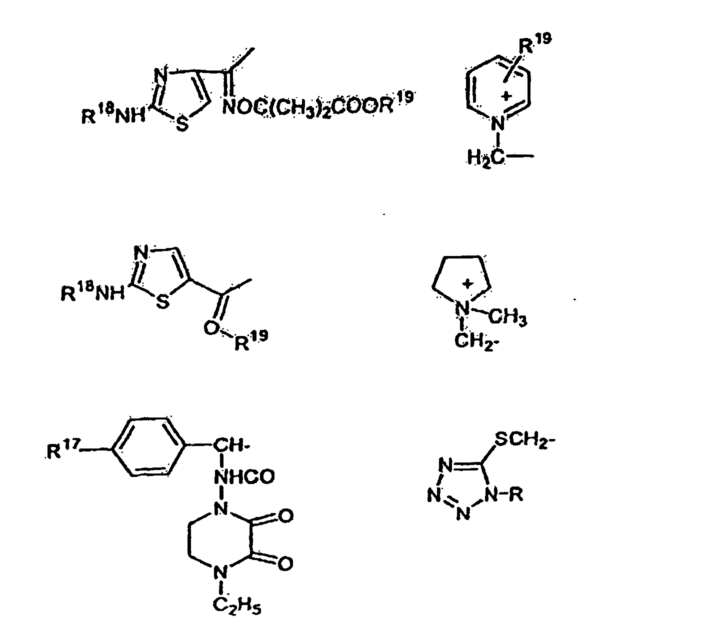 où
R est alkyle,
R¹⁶ est une liaison covalente qui lie le groupe (b) au lieur;
un de R¹⁸ et R¹⁹ est hydrogène ou alkyle et l'autre est une liaison covalente qui lie le groupe (b) au lieur;
(iii) un ligand de la formule (c): 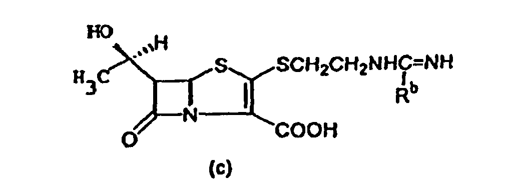 dans laquelle:
R^{b} est une liaison covalente liant (c) au lieur;
(iv) un ligand de la formule (d): 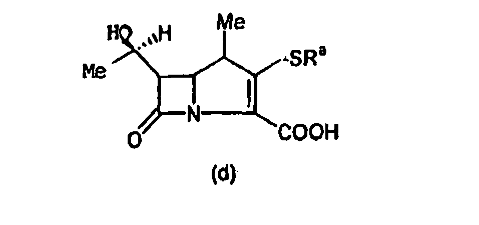 dans laquelle:
R^{a} est: 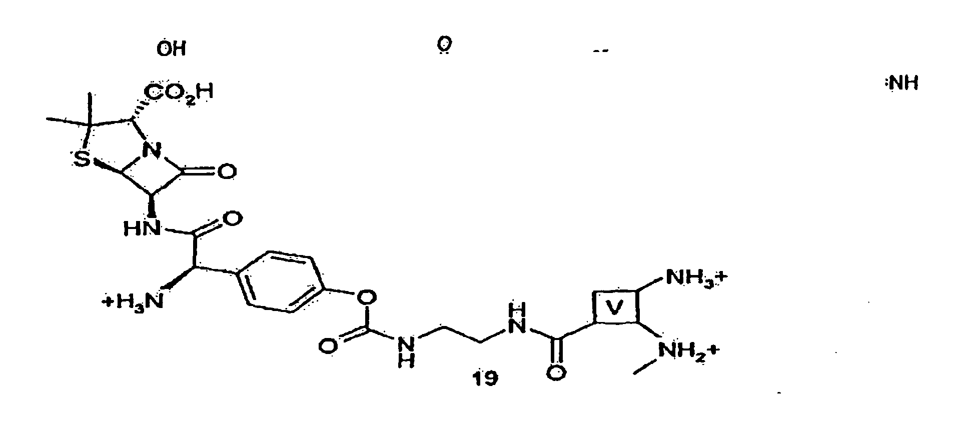 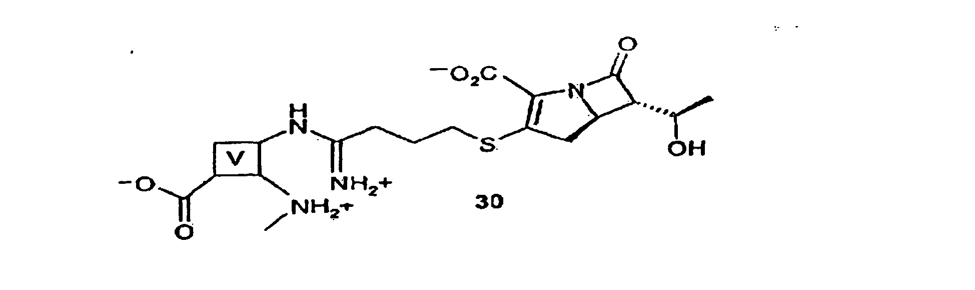 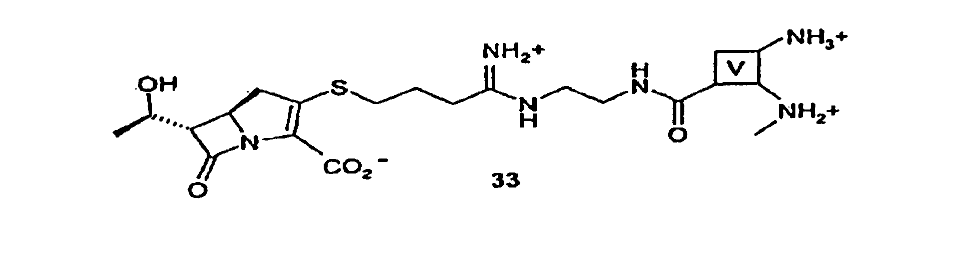
dans laquelle
R²³ est une liaison covalente qui lie (d) au lieur;
un de R²⁴ et R²⁵ est alkyle, alkyle substitué ou aralkyle, et l'autre est une liaison covalente qui lie (d) au lieur, ou
(v) un ligand de la formule (e): 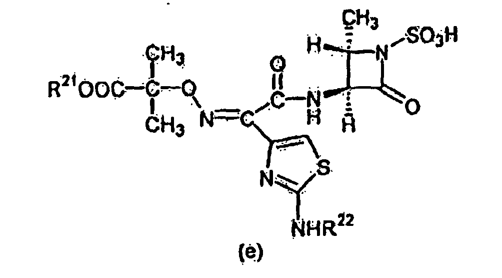
dans laquelle:
un de R²¹ et R²² est hydrogène et l'autre lie (e) au lieur.

5. Le composé multiliant de la revendication 4, où le lieur est choisi dans le groupe comprenant: un composé de formule:
-X^{a}-Z-(Y^{a}-Z)ₘ-X^{a}-où
m est un entier allant de 0 à 20;
X^{a} est choisi chaque fois séparément, parmi le groupe consistant en O, S, NR, C(O), C(O)O, OC(O), C(O)NR, NRC(O), C(S), C(S)O, C(S)NR, NRC(S), ou une liaison covalente, où R est tel que défini ci-dessous;
Z est choisi chaque fois séparément, parmi alkylène, alkylène substitué, cycloalkylène, cycloalkylène substitué, alcénylène, alcénylène substitué, alcynylène, alcynylène substitué, cycloalcénylène, cycloalcénylène substitué, arylène, hétéroarylène, hétérocyclène ou une liaison covalente;
chacun des Y^{a} est choisi chaque fois séparément, parmi O, C(O), OC(O), C(O)O, NR, S(O)ₙ, C(O)NR', NR'C(O), NR'C(O)NR', NR'C(S)NR', C(=NR')NR', NR'C(=NR'), OC(O)NR', N=C(X^{a}), NR', NR'-C(X^{a})=N, P(O)(OR')O, OP(O)(OR'), S(O)ₙCR'R", S(O)ₙNR', NR'-S(O)ₙ, S-S et une liaison covalente, où n est 0, 1 ou 2, et R, R' et R" sont choisis, chaque fois séparément, parmi hydrogène, alkyle, alkyle substitué, cycloalkyle, cycloalkyle substitué, alcényle, alcényle susbtitué, cycloalcényle, cycloalcényle substitué, alcynyle, alcynyle substitué, aryle, hétéroaryle et hétérocyclique.

6. Le composé selon la revendication 1, qui est l'hétérodimère vancomycine-amoxicilline de la structure 19, l'hétérodimère vancomycine-imipénème de la structure 30, ou l'hétérodimère vancomycine-imipénème de la structure 33:

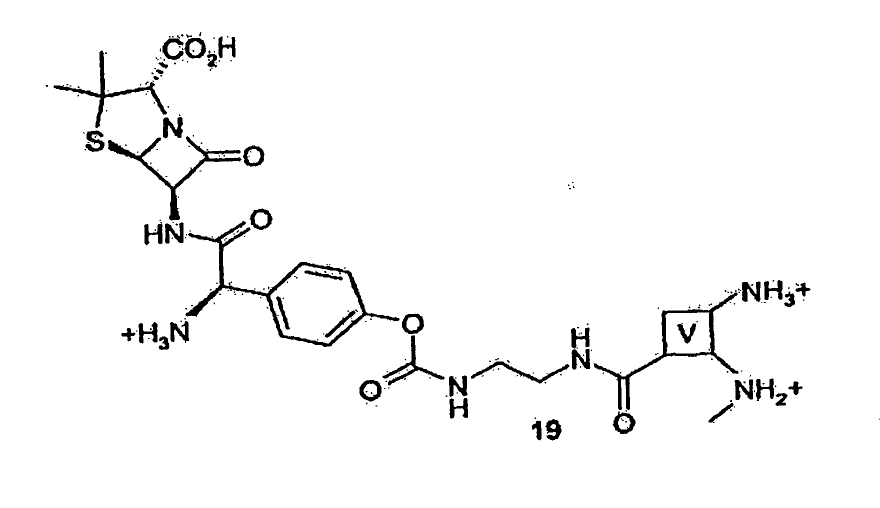

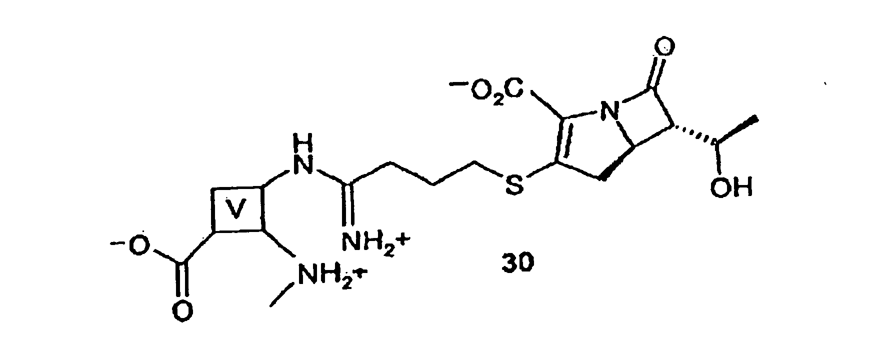

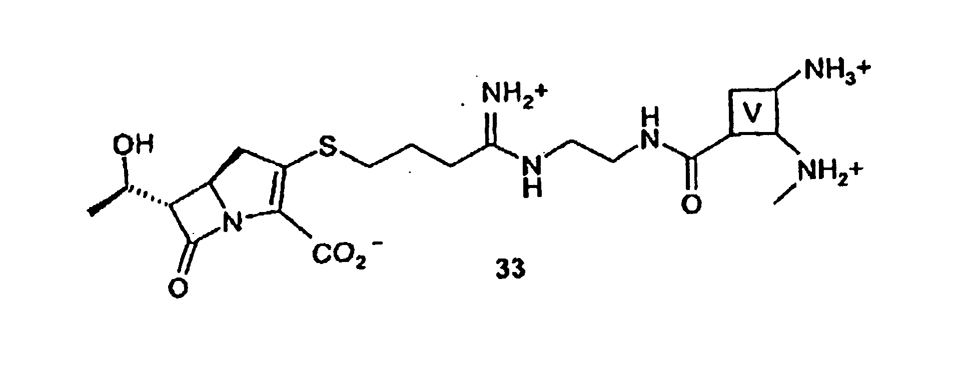

7. Une composition pharmaceutique comprenant un support pharmaceutiquement acceptable et une quantité efficace d'un composé selon l'une quelconque des revendications 1 à 6.

8. Le composé selon l'une quelconque des revendications 1 à 6, ou composition pharmaceutique selon la revendication 7, à utiliser comme médicament.

9. Le composé selon la revendication 8, à utiliser comme médicament pour traiter une maladie bactérienne chez un mammifère.

10. Utilisation d'un composé selon l'une quelconque des revendications 1 à 6, ou composition pharmaceutique selon la revendication 7, pour la préparation d'un médicament pour traiter une maladie bactérienne chez un mammifère.
